(19) Europäisches Patentamt
European Patent Office
Office européen des brevets

(11) EP 4 130 045 A1

(12) **EUROPEAN PATENT APPLICATION**
published in accordance with Art. 153(4) EPC

(43) Date of publication:
**08.02.2023 Bulletin 2023/06**

(21) Application number: **21774485.3**

(22) Date of filing: **25.03.2021**

(51) International Patent Classification (IPC):
***C07K 16/30*** *(1995.01)* ***A61K 39/395*** *(1980.01)*
***A61P 35/00*** *(2000.01)* ***A61K 31/4745*** *(2000.01)*
***A61K 31/48*** *(1974.07)*

(52) Cooperative Patent Classification (CPC):
**A61K 31/4745; A61K 31/48; A61K 39/395; A61P 35/00; C07K 16/30**

(86) International application number:
**PCT/CN2021/083014**

(87) International publication number:
**WO 2021/190602 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.03.2020 CN 202010219311**
**19.03.2021 CN 202110297397**

(71) Applicants:
- **Jiangsu Hengrui Pharmaceuticals Co., Ltd.
Lianyungang, Jiangsu 222047 (CN)**
- **Shanghai Hengrui Pharmaceutical Co., Ltd.
Shanghai 200245 (CN)**

(72) Inventors:
- **LIANG, Zhi
Shanghai 200245 (CN)**
- **LIN, Wenfeng
Shanghai 200245 (CN)**
- **SHI, Ruijun
Shanghai 200245 (CN)**
- **LIU, Xun
Shanghai 200245 (CN)**

(74) Representative: **Sonzogni, Laura Gabriella et al
Dragotti & Associati S.r.l.
Via Nino Bixio, 7
20129 Milano (IT)**

Remarks:
The complete document including Reference Table(s) and the Sequence Listing(s) can be downloaded from the EPO website

# (54) PREPARATION METHOD FOR ANTIBODY MEDICAMENT CONJUGATE

(57) Disclosed is a preparation method for an antibody medicament conjugate, comprising steps for synthesizing and purifying same.

EP 4 130 045 A1

## Description

The present application claims priority to the Chinese Patent Application (Application No. CN 202010219311.2) filed on Mar. 25, 2020 and the Chinese Patent Application (Application No. CN 202110297397.5) filed on Mar. 19, 2021.

## TECHNICAL FIELD

The present disclosure relates to a method for preparing an antibody drug conjugate, and particularly to synthesis and purification steps of a method for preparing an antibody drug conjugate.

## BACKGROUND

The statements herein merely provide background information related to the present disclosure and may not necessarily constitute the prior art.

Chemotherapy remains one of the most important anticancer means, including surgery, radiotherapy and targeted therapy. Although the variety of efficient cytotoxic drugs is large, the difference between tumor cells and normal cells is small, which limits the wide clinical application of these antitumor compounds due to their toxic side effects. As the antitumor monoclonal antibody has specificity to a tumor cell surface antigen, antibody drugs have become a front-line drug for antitumor treatment, but when the antibody is used alone as an antitumor drug, the therapeutic effect is often not satisfactory.

An antibody drug conjugate (ADC) is formed by linking a monoclonal antibody or an antibody fragment to a biologically-active cytotoxic drug via a stable chemical linker compound, fully exploiting the binding specificity of the antibody to surface antigens of normal cells and tumor cells and the high efficiency of the cytotoxic drug, and also avoiding the former's disadvantage of having a poor therapeutic effect, the latter's disadvantage of having serious toxic side effects, and the like. This means that the antibody drug conjugate can bind to tumor cells more precisely and has a reduced effect on normal cells compared to conventional chemotherapeutic drugs in the past (Mullard A, (2013) Nature Reviews Drug Discovery, 12:329-332; DiJoseph JF, Armellino DC, (2004) Blood, 103:1807-1814).

Mylotarg (gemtuzumab ozogamicin, Wyeth Pharmaceutical Co., Ltd.), the first antibody drug conjugate, was approved by U.S. FDA in 2000 for the treatment of acute myelocytic leukemia (Drugs of the Future (2000) 25(7):686; US4970198; US 5079233; US 5585089; US 5606040; US 5693762; US 5739116; US 5767285; US 5773001). Adcetris® (brentuximab vedotin, Seagen Inc.) was approved by fast track review designed by U.S. FDA in August 2011 for the treatment of Hodgkin's lymphoma and recurrent anaplastic large cell lymphoma (Nat. Biotechnol (2003) 21(7):778-784; WO2004010957; WO2005001038; US7090843; US7659241; WO2008025020). Adcetris® is a novel ADC drug that enables the drug to directly act on target CD30 on lymphoma cells and then carry out endocytosis so as to induce apoptosis of the tumor cells.

Both Mylotarg and Adcetris are targeted therapies against hematological tumors which are relatively simple in tissue structure compared to solid tumors. Kadcyla (ado-trastuzumab emtansine, T-DM1) was approved by U.S. FDA in February 2013 for the treatment of HER2-positive patients with advanced or metastatic breast cancer and having drug resistance to trastuzumab (trade name: Herceptin®) and paclitaxel (WO2005037992; US8088387). Kadcyla is the first ADC drug approved by U.S. FDA for the treatment of solid tumors.

There are several classes of small molecules with cytotoxicity for antibody drug conjugates, one of which is camptothecin derivatives having an antitumor effect by inhibiting topoisomerase I. The camptothecin derivative, irinotecan (chemical name: (1*S*, 9*S*)-1-amino-9-ethyl-5-fluoro-2,3-dihydro-9-hydroxy-4-methyl-1*H*,12*H*-benzo[*de*]pyran o[3,4:6,7] imidazo[1,2-*b*]quinoline-10,13(9*H*,15*H*)-dione), was reported for use in antibody drug conjugates (ADCs) as described in WO2014057687; Clinical Cancer Research (2016)22 (20):5097-5108; Cancer Sci (2016) 107: 1039-1046. There is still a need to further develop ADC drugs with better therapeutic effects.

## SUMMARY

The present disclosure provides a method for preparing an antibody drug conjugate, wherein the antibody drug conjugate has a structure as shown in general formula (Pc-$L_a$-Y-D):

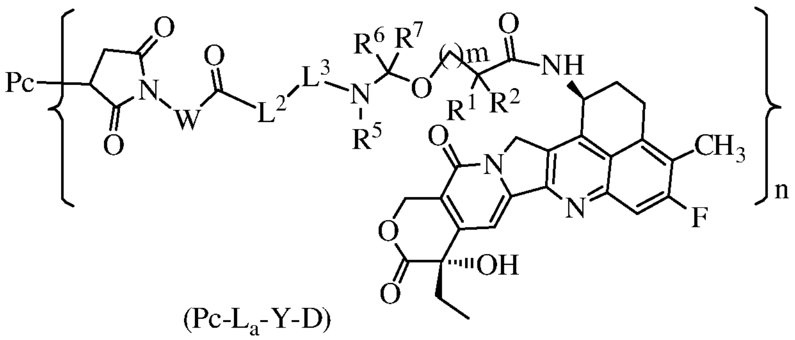

(Pc-$L_a$-Y-D)

;

wherein:

W is selected from the group consisting of $C_{1-8}$ alkyl, $C_{1-8}$ alkyl-$C_{3-7}$ cycloalkyl and linear heteroalkyl of 1 to 8 atoms, the linear heteroalkyl comprising 1 to 3 heteroatoms selected from the group consisting of N, O and S, wherein the $C_{1-8}$ alkyl, the $C_{3-7}$ cycloalkyl and the linear heteroalkyl are each independently optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-7}$ cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;

$L^3$ is a peptide residue consisting of 2 to 7 amino acid residues, wherein the amino acid residues are selected from the group consisting of amino acid residues formed from amino acids from phenylalanine (F), glycine (G), valine (V), lysine (K), citrulline, serine (S), glutamic acid (Q) and aspartic acid (D), and are optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-7}$ cycloalkyl;

$R^1$ is $C_{1-6}$ haloalkyl or $C_{3-7}$ cycloalkyl;

$R^2$ is selected from the group consisting of hydrogen, $C_{1-6}$ haloalkyl and $C_{3-7}$ cycloalkyl; or, $R^1$ and $R^2$, together with carbon atoms linked thereto, form $C_{3-7}$ cycloalkyl;

$R^5$ is selected from the group consisting of hydrogen, $C_{1-6}$ alkyl, $C_{1-6}$ haloalkyl, deuterated $C_{1-6}$ alkyl and hydroxy $C_{1-6}$ alkyl;

$R^6$ and $R^7$ are identical or different and are each independently selected from the group consisting of hydrogen, halogen, $C_{1-6}$ alkyl, $C_{1-6}$ haloalkyl, deuterated $C_{1-6}$ alkyl and hydroxy $C_{1-6}$ alkyl;

m is 0 or 1;

n is a decimal or an integer from 3 to 8;

Pc is an antibody or an antigen-binding fragment thereof;

the preparation method comprises the following steps:

step (a): reacting the antibody or the antigen-binding fragment thereof with a reducing agent at a reaction temperature of about 1 °C to about 36 °C; and

step (b): reacting the product of the step (a) with a compound of the following formula (La-Y-D);

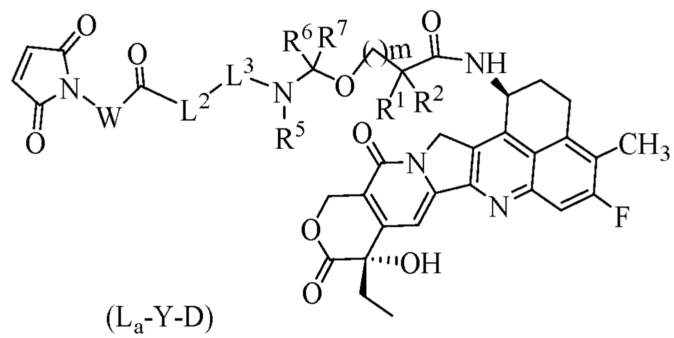

($L_a$-Y-D)

;

wherein: W, $L^2$, $L^3$, $R^1$, $R^2$, $R^5$, $R^6$, $R^7$ and m are as defined above.

In another aspect, the present disclosure provides a method for preparing an antibody drug conjugate, wherein the antibody drug conjugate has a structure as shown in the following formula:

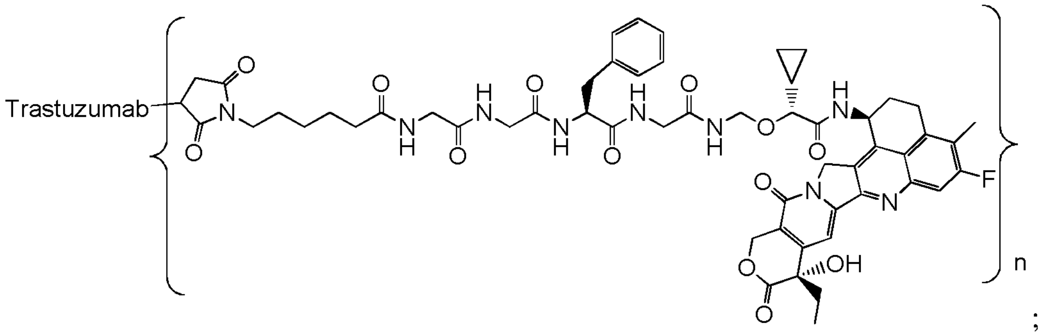

wherein n is a decimal or an integer from 4 to 8;
the preparation method comprises the following steps:

step (a): reacting the antibody or the antigen-binding fragment thereof with a reducing agent at a reaction temperature of about 1 °C to about 36 °C; and
step (b): reacting the product of the step (a) with a compound of the following formula;

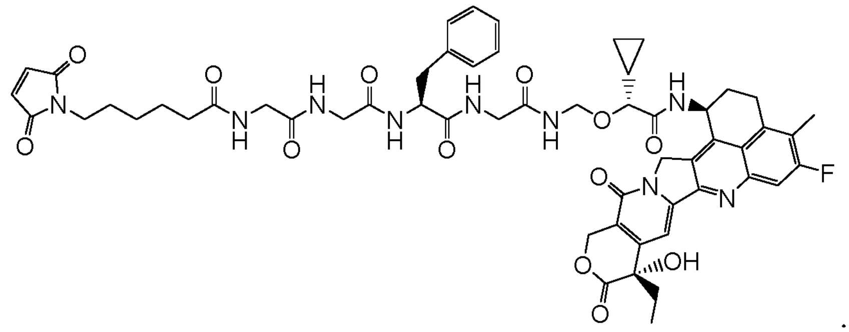

In an alternative embodiment, the reaction temperature condition in step (a) is about 4 °C to about 30 °C, preferably about 20 °C to about 30 °C, and more preferably 25 °C, non-limiting examples of which include about 20 °C, about 21 °C, about 22 °C, about 23 °C, about 24 °C, about 25 °C, about 26 °C, about 27 °C, about 28 °C, about 29 °C, and about 30 °C. In some embodiments, the reaction temperature condition is 13 °C to 28 °C or 13 °C to 25 °C.
In an alternative embodiment, the reaction in step (a) is performed at a pH of about 4.5 to about 6.5, preferably the reaction is performed at a pH of about 5.0 to about 6.0, and more preferably the reaction is performed at a pH of about 5.6. In a non-limiting example, the reaction is performed at a pH of about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, or about 6.0.
In an alternative embodiment, the reaction in step (a) is performed in a buffer; in a non-limiting example, the buffer is selected from the group consisting of histidine salt buffers, phosphate buffers and acetate buffers.
In an alternative embodiment, the reaction in step (a) is performed in a buffer; in a non-limiting example, the buffer is a histidine-hydrochloric acid buffer.
In an alternative embodiment, the buffer is selected from the group consisting of EDTA-containing histidine salt buffers and EDTA-containing histidine-hydrochloric acid buffers. Illustratively, the histidine salt buffer has a concentration of 1 mM to 100 mM, 10 mM to 50 mM, 20 mM, 30 mM, or 40 mM; EDTA has a concentration of 1 mM to 10 mM, 2 mM to 5 mM, 2.5 mM, 3 mM or 4 mM. In some embodiments, the buffer contains 10 mM to 50 mM histidine salt buffer and 1 mM to 10 mM EDTA. In some embodiments, the buffer contains 20 mM histidine-hydrochloric acid buffer and 2.5 mM EDTA. EDTA refers to ethylenediaminetetraacetic acid.
In an alternative embodiment, the reducing agent in step (a) is selected from the group consisting of tris(2-carboxyethyl)phosphine (TCEP) or a salt thereof, 1,4-dimercaptothreitol (DTT), β-mercaptoethanol (β-ME) and other suitable reducing agents, preferably TCEP or a salt thereof, and more preferably tris(2-carboxyethyl)phosphine hydrochloride.
In an alternative embodiment, the molar ratio of the reducing agent to the antibody or the antigen-binding

fragment thereof (Pc) in step (a) is 2:1 to 10:1, 2.6:1 to 7:1, 2.9:1 to 3.7:1, 3.2:1 to 3.4:1, or 3.3:1.

In an alternative embodiment, step (b) is performed in an organic solvent, preferred organic solvents including dimethyl sulfoxide (DMSO), *N,N*-dimethylformamide (DMF), N,N-dimethylacetamide (DMAc), acetonitrile, or a mixture thereof. In a non-limiting example, step (b) comprises: dissolving the compound of formula (La-Y-D) in DMSO, and mixing the product of step (a) with a solution of the compound of formula (La-Y-D) in DMSO.

In an alternative embodiment, the above preparation method further comprises a step (c) comprising purifying the product of step (b). The purification can be performed by adopting cation column chromatography or affinity column chromatography, wherein a packing material of the cation column chromatography is selected from the group consisting of Capto S Impact and Poros XS, and preferably Capto S Impact. In some embodiments, the Capto S Impact is Capto™ S Impact. In some embodiments, the Poros XS is Poros™ XS.

In an alternative embodiment, the drug loading (n) may range from 3 to 8, 4 to 8, or 5 to 7, preferably 5.3 to 6.1, and more preferably 5.7, of which cytotoxic drugs bind to per antibody or antigen-binding fragment thereof (Pc). n is a decimal or an integer. In some embodiments, n is 5.3, 5.4, 5.5, 5.6 or 5.7.

In an alternative embodiment, drug loading distribution of the antibody drug conjugate is as follows: in a population of antibody heavy chains, a proportion of antibody heavy chains binding to 4 drugs is 4% or less; preferably, a proportion of antibody heavy chains binding to 4 drugs is 4% or less, and a proportion of antibody heavy chains not binding to drugs is 6% or less. In a non-limiting example, the proportion of antibody heavy chains binding to 4 drugs is 4% or less, 3% or less, 2% or less, or 1% or less; the proportion of the antibody heavy chains not binding to drugs is 6% or less, 5% or less, 4% or less, 3% or less, 2% or less, or 1% or less. Or, in a population of antibody light chains, a proportion of antibody light chains binding to one drug is 65% or more, 66% or more, 67% or more, 68% or more, 69% or more, 70% or more, 75% or more, 80% or more, 85% or more, 90% or more, or 95% or more. In some embodiments, drug loading distribution of the antibody drug conjugate is as follows: in a population of antibody heavy chains, a proportion of antibody heavy chains binding to 4 drugs is 4% or less, and a proportion of antibody heavy chains not binding to drugs is 5% or less; In some embodiments, drug loading distribution of the antibody drug conjugate is as follows: in a population of antibody heavy chains, a proportion of antibody heavy chains binding to 4 drugs is 1% or less, and a proportion of antibody heavy chains not binding to drugs is 4% or less. In some embodiments, drug loading distribution of the antibody drug conjugate is as follows: in a population of antibody heavy chains, a proportion of antibody heavy chains binding to four drugs is 4% or less, and a proportion of antibody heavy chains not binding to drugs is 5 % or less; and in a population of antibody light chains, a proportion of antibody light chains binding to one drug is 65% or more. In some embodiments, drug loading distribution of the antibody drug conjugate is as follows: in a population of antibody heavy chains, a proportion of antibody heavy chains binding to four drugs is 1 % or less, and a proportion of antibody heavy chains not binding to drugs is 4% or less; and in a population of antibody light chains, a proportion of antibody light chains binding to one drug is 70% or more. In some embodiments, the proportion of antibody heavy chains and/or antibody light chains is determined by reversed phase chromatography.

In an alternative embodiment, the preparation method is suitable for large scale preparation. The amount of the antibody trastuzumab in the preparation method is 100 mg or more, preferably 1 g or more, more preferably 10 g or more, and most preferably 100 g or more.

In an alternative embodiment, the antibody drug conjugate has a structure as shown in general formula (Pc-$L_b$-Y-D):

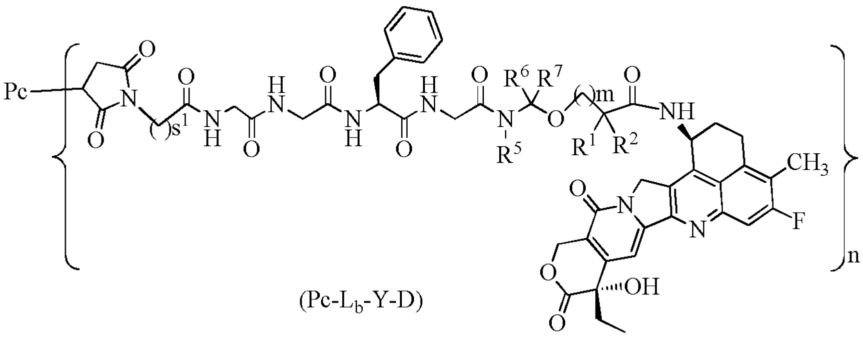

(Pc-$L_b$-Y-D)

;

wherein:

$s^1$ is an integer from 2 to 8;

Pc, $R^1$, $R^2$, $R^5$, $R^6$, $R^7$, m and n are as defined above;

the preparation method comprises the following steps:

step (a): reacting the antibody or the antigen-binding fragment thereof with a reducing agent at a reaction temperature of about 1 °C to about 36 °C; and
step (b): reacting the product of the step (a) with a compound of the following formula ($L_b$-Y-D);

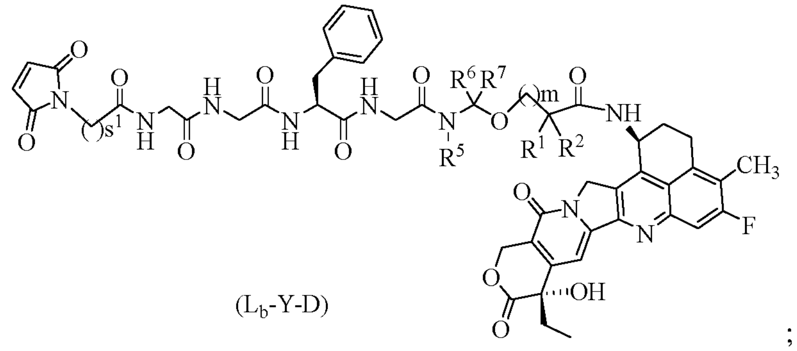

($L_b$-Y-D)

;

wherein: $s^1$, $R^1$, $R^2$, $R^5$, $R^6$, $R^7$ and m are as defined above.

In an alternative embodiment, the antibody drug conjugate described above has the following structure:

| Structure |
| --- |
| 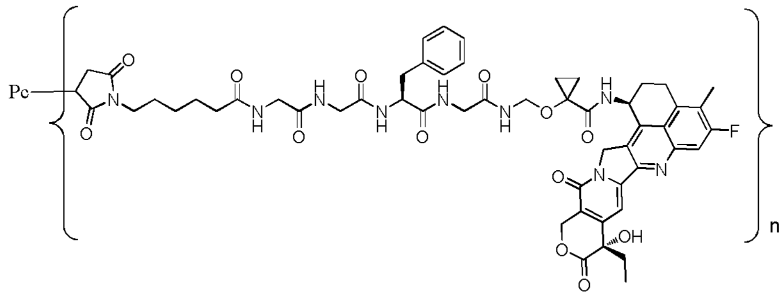 |
| 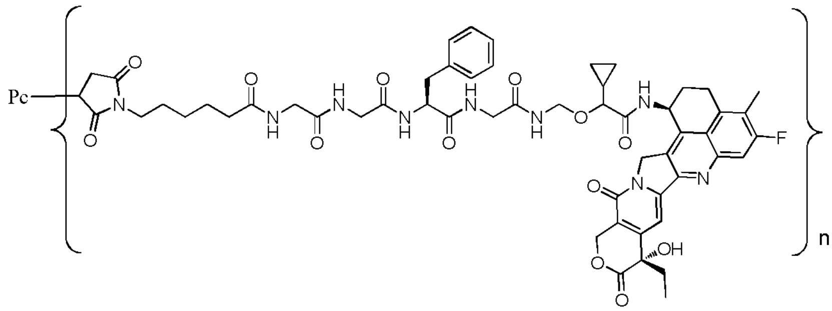 |
| 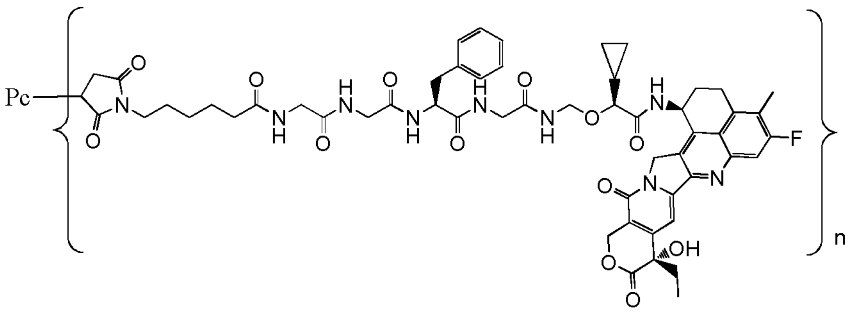 |

| |
|---|
| 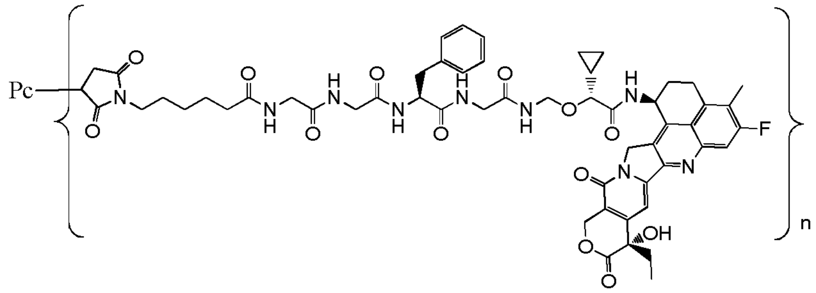 |
| 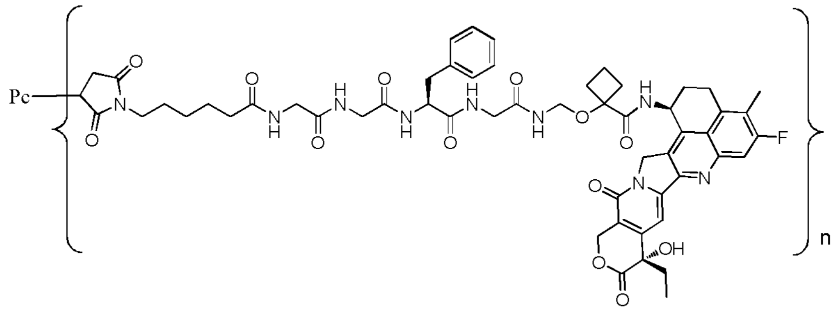 |
| 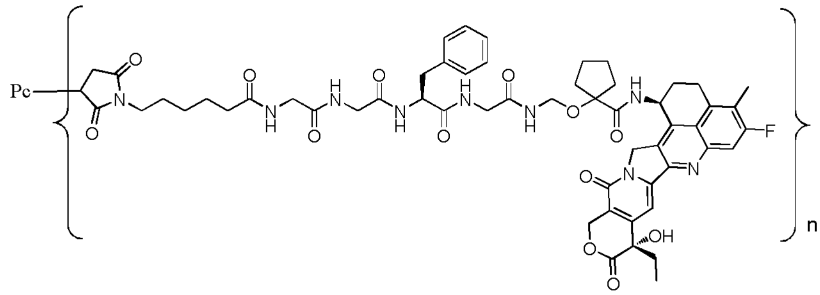 |
| 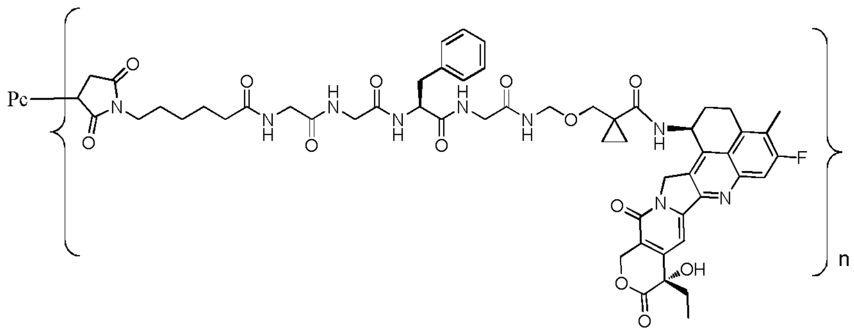 |
| 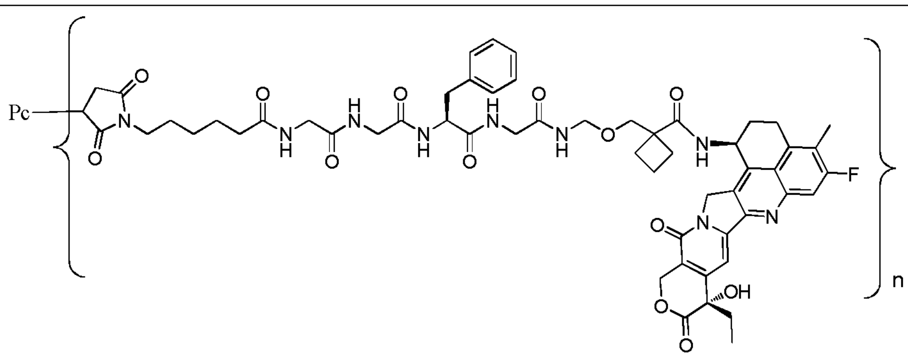 |

| |
|---|
| 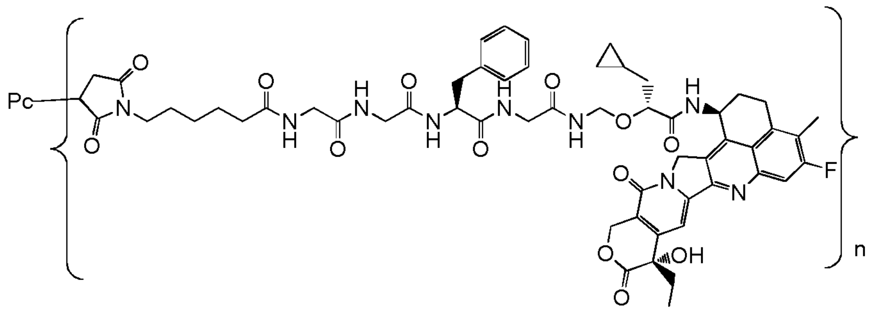 |
| 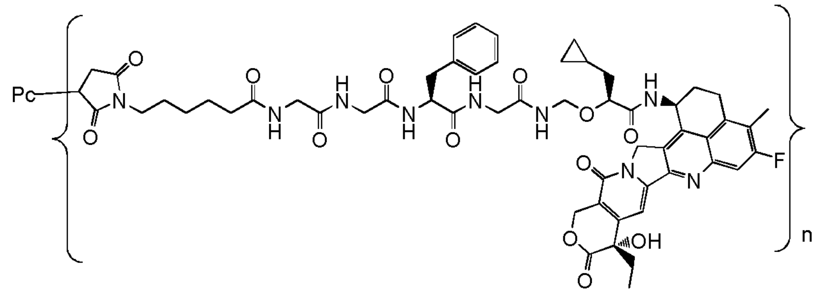 |
| 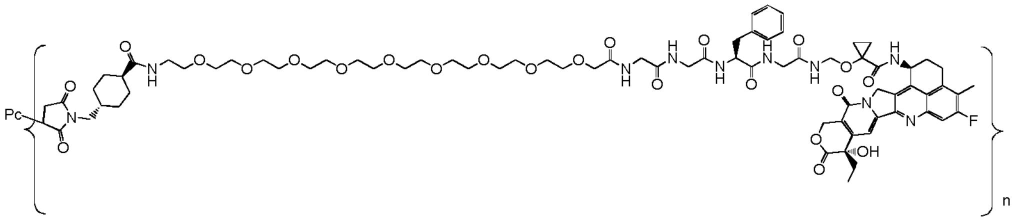 |
| 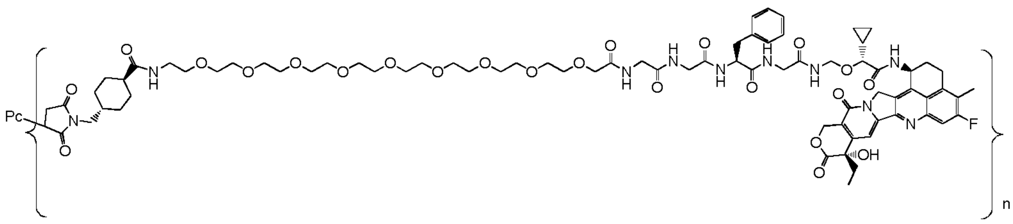 |
| 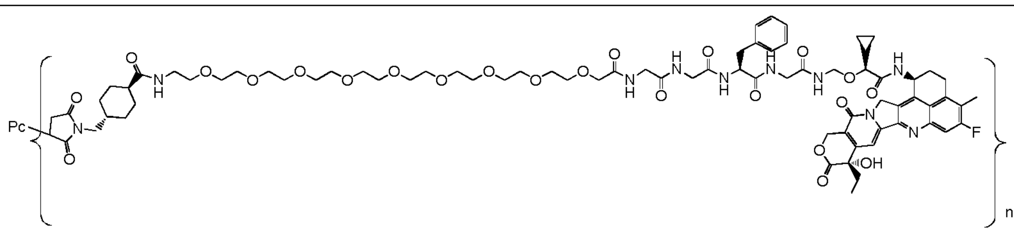 |

wherein Pc and n are as defined in general formula (Pc-$L_a$-Y-D).

In an alternative embodiment, the Pc is an antibody or an antigen-binding fragment thereof, and the antibody is selected from the group consisting of a chimeric antibody, a humanized antibody and a fully human-derived antibody. In some embodiments, the antibody is a monoclonal antibody.

In an alternative embodiment, the antibody or the antigen-binding fragment thereof is selected from the group consisting of an anti-HER2 (ErbB2) antibody, an anti-EGFR antibody, an anti-B7-H3 antibody, an anti-c-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-Integrin antibody, an anti-PSMA antibody, an anti-Tenascin-C antibody, an anti-SLC44A4 antibody, an anti-Mesothelin antibody and an antigen-binding fragment thereof; preferably, the antibody or the antigen-binding fragment thereof is selected from the group consisting of trastuzumab, pertuzumab, nimotuzumab, enobiluzumab, emibetuzumab, inotuzumab, pintuzumab, brentuximab, gemtuzumab, bivatuzumab, lorvotuzumab, cBR96, glematumamab and an antigen-binding fragment thereof.

In an alternative embodiment, the antibody conjugate has a structure as shown in the following formula:

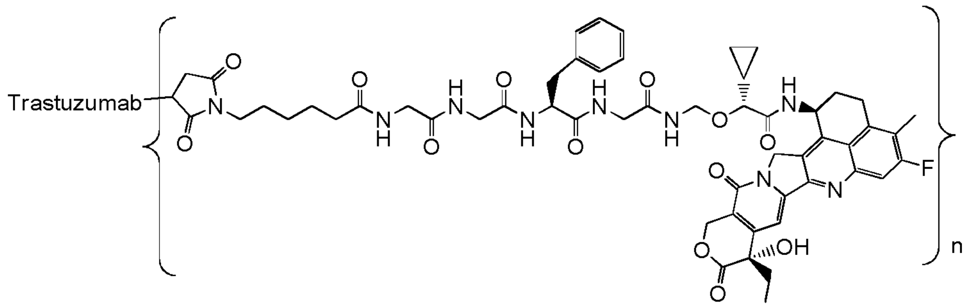

;

wherein n is a decimal or an integer from 4 to 8.

In another aspect, the present disclosure provides a method for preparing an antibody drug conjugate, wherein the antibody drug conjugate has a structure as shown in the following formula:

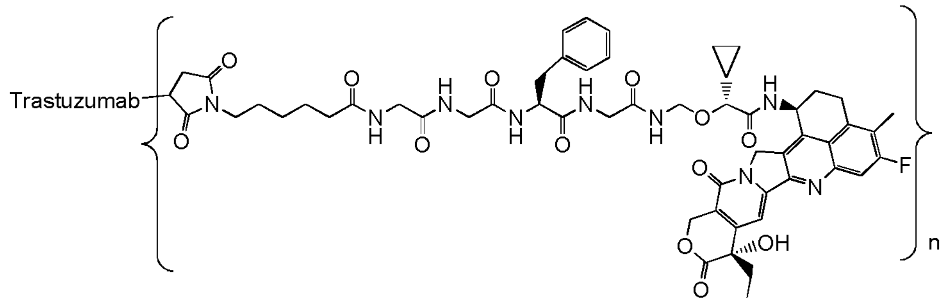

;

wherein n is a decimal or an integer from 4 to 8;

the preparation method comprises the following steps:

step (a): reacting trastuzumab with TCEP at a reaction temperature of about 4 °C to about 30 °C and a pH of about 4.5 to about 6.5; and

step (b): reacting the product of the step (a) with a compound of the following formula;

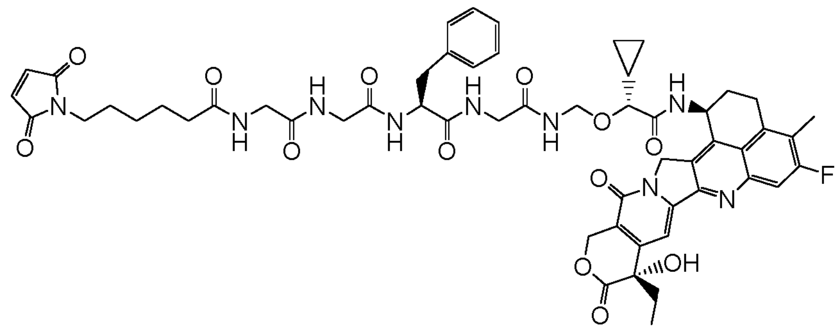

.

In an alternative embodiment, the antibody drug conjugate has a structure as shown in the following formula:

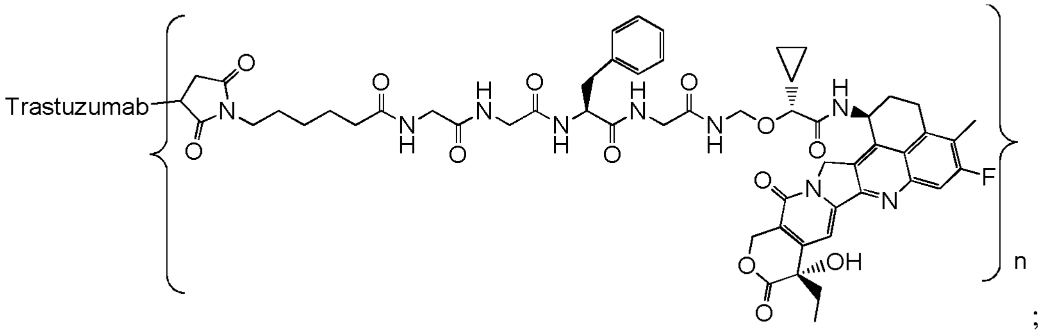

wherein n is a decimal or an integer from 4 to 8;
the preparation method comprises the following steps:

step (a): reacting trastuzumab with TCEP at a reaction temperature of about 25 °C and a pH of about 5.6, the reaction being performed in an EDTA-containing histidine-hydrochloric acid buffer;
step (b): reacting the product of the step (a) with a compound of the following formula;

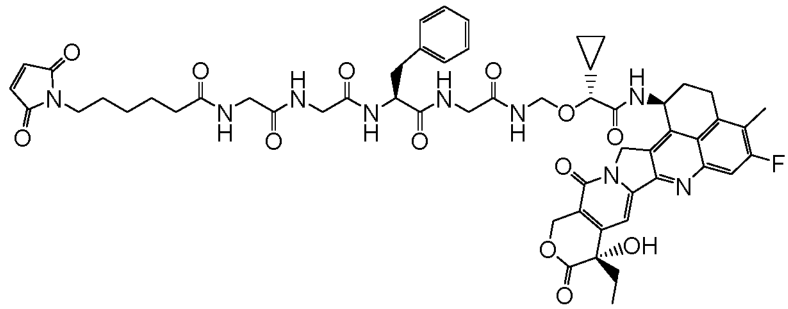

and
step (c): subjecting the product of step (b) to purification through a cation chromatography column or an affinity chromatography column. In some embodiments, the EDTA-containing histidine-hydrochloric acid buffer contains 20 mM histidine-hydrochloric acid buffer and 2.5 mM EDTA.

The present disclosure further provides an antibody drug conjugate or a pharmaceutically acceptable salt thereof, wherein the antibody drug conjugate has a structure as shown in general formula ($Pc\text{-}L_a\text{-}Y\text{-}D$):

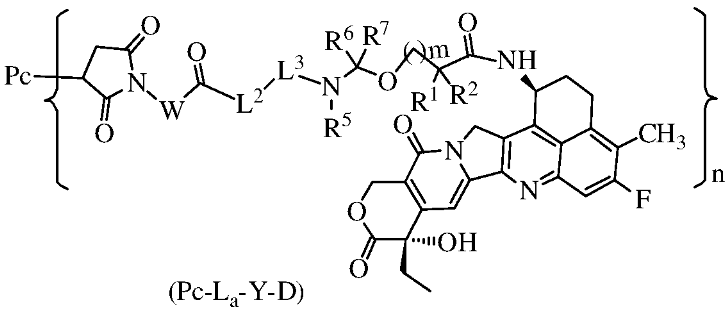

($Pc\text{-}L_a\text{-}Y\text{-}D$) ;

wherein:

W is selected from the group consisting of $C_{1-8}$ alkyl, $C_{1-8}$ alkyl-$C_{3-7}$ cycloalkyl and linear heteroalkyl of 1 to 8 atoms, the linear heteroalkyl comprising 1 to 3 heteroatoms selected from the group consisting of N, O and S, wherein the $C_{1-8}$ alkyl, the $C_{3-7}$ cycloalkyl and the linear heteroalkyl are each independently optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-7}$ cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;

$L^3$ is a peptide residue consisting of 2 to 7 amino acid residues, wherein the amino acid residues are selected from the group consisting of amino acid residues formed from amino acids from phenylalanine (F), glycine (G), valine (V), lysine (K), citrulline, serine (S), glutamic acid (Q) and aspartic acid (D), and are optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-7}$ cycloalkyl;

$R^1$ is $C_{1-6}$ haloalkyl or $C_{3-7}$ cycloalkyl;

$R^2$ is selected from the group consisting of hydrogen, $C_{1-6}$ haloalkyl and $C_{3-7}$ cycloalkyl; or, $R^1$ and $R^2$, together with carbon atoms linked thereto, form $C_{3-7}$ cycloalkyl;

$R^5$ is selected from the group consisting of hydrogen, $C_{1-6}$ alkyl, $C_{1-6}$ haloalkyl, deuterated $C_{1-6}$ alkyl and hydroxy $C_{1-6}$ alkyl;

$R^6$ and Rare identical or different and are each independently selected from the group consisting of hydrogen, halogen, $C_{1-6}$ alkyl, $C_{1-6}$ haloalkyl, deuterated $C_{1-6}$ alkyl and hydroxy $C_{1-6}$ alkyl;

m is 0 or 1;

n is a decimal or an integer from 4 to 8;

Pc is an antibody or an antigen-binding fragment thereof;

drug loading distribution of the antibody drug conjugate is as follows: in a population of antibody heavy chains, a proportion of antibody heavy chains binding to 4 drugs is 4% or less; preferably, a proportion of antibody heavy chains binding to 4 drugs is 4% or less, and a proportion of antibody heavy chains not binding to drugs is 6% or less. In some embodiments, drug loading distribution of the antibody drug conjugate is as follows: in a population of antibody heavy chains, a proportion of antibody heavy chains binding to 4 drugs is 4% or less, and a proportion of antibody heavy chains not binding to drugs is 5% or less; In some embodiments, drug loading distribution of the antibody drug conjugate is as follows: in a population of antibody heavy chains, a proportion of antibody heavy chains binding to 4 drugs is 1% or less, and a proportion of antibody heavy chains not binding to drugs is 4% or less. In some embodiments, drug loading distribution of the antibody drug conjugate is as follows: in a population of antibody heavy chains, a proportion of antibody heavy chains binding to four drugs is 4% or less, and a proportion of antibody heavy chains not binding to drugs is 5% or less; and in a population of antibody light chains, a proportion of antibody light chains binding to one drug is 65% or more. In some embodiments, drug loading distribution of the antibody drug conjugate is as follows: in a population of antibody heavy chains, a proportion of antibody heavy chains binding to four drugs is 1 % or less, and a proportion of antibody heavy chains not binding to drugs is 4% or less; and in a population of antibody light chains, a proportion of antibody light chains binding to one drug is 70% or more.

The present disclosure further provides an antibody drug conjugate or a pharmaceutically acceptable salt thereof, wherein the antibody drug conjugate is prepared by the method for preparing an antibody drug conjugate described above; and drug loading distribution of the antibody drug conjugate is as follows: in a population of antibody heavy chains, a proportion of antibody heavy chains binding to 4 drugs is 4% or less; preferably, a proportion of antibody heavy chains binding to 4 drugs is 4% or less, and a proportion of antibody heavy chains not binding to drugs is 6% or less. In some embodiments, drug loading distribution of the antibody drug conjugate is as follows: in a population of antibody heavy chains, a proportion of antibody heavy chains binding to 4 drugs is 4% or less, and a proportion of antibody heavy chains not binding to drugs is 5% or less; In some embodiments, drug loading distribution of the antibody drug conjugate is as follows: in a population of antibody heavy chains, a proportion of antibody heavy chains binding to 4 drugs is 1% or less, and a proportion of antibody heavy chains not binding to drugs is 4% or less. In some embodiments, drug loading distribution of the antibody drug conjugate is as follows: in a population of antibody heavy chains, a proportion of antibody heavy chains binding to four drugs is 4% or less, and a proportion of antibody heavy chains not binding to drugs is 5% or less; and in a population of antibody light chains, a proportion of antibody light chains binding to one drug is 65% or more. In some embodiments, drug loading distribution of the antibody drug conjugate is as follows: in a population of antibody heavy chains, a proportion of antibody heavy chains binding to four drugs is 1 % or less, and a proportion of antibody heavy chains not binding to drugs is 4% or less; and in a population of antibody light chains, a proportion of antibody light chains binding to one drug is 70% or more.

The present disclosure further provides an antibody drug conjugate or a pharmaceutically acceptable salt thereof, wherein the antibody drug conjugate has a structure as shown in the following formula:

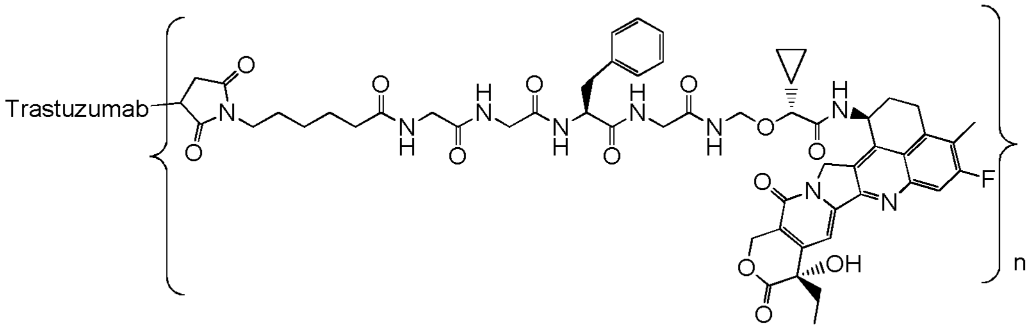
;

wherein n is a decimal or an integer from 4 to 8;
the antibody drug conjugate is prepared by the method for preparing an antibody drug conjugate described above; and drug loading distribution of the antibody drug conjugate is as follows: in a population of antibody heavy chains, a proportion of antibody heavy chains binding to 4 drugs is 4% or less; preferably, a proportion of antibody heavy chains binding to 4 drugs is 4% or less, and a proportion of antibody heavy chains not binding to drugs is 6% or less. In some embodiments, drug loading distribution of the antibody drug conjugate is as follows: in a population of antibody heavy chains, a proportion of antibody heavy chains binding to 4 drugs is 4% or less, and a proportion of antibody heavy chains not binding to drugs is 5% or less; In some embodiments, drug loading distribution of the antibody drug conjugate is as follows: in a population of antibody heavy chains, a proportion of antibody heavy chains binding to 4 drugs is 1% or less, and a proportion of antibody heavy chains not binding to drugs is 4% or less. In some embodiments, drug loading distribution of the antibody drug conjugate is as follows: in a population of antibody heavy chains, a proportion of antibody heavy chains binding to four drugs is 4% or less, and a proportion of antibody heavy chains not binding to drugs is 5% or less; and in a population of antibody light chains, a proportion of antibody light chains binding to one drug is 65% or more. In some embodiments, drug loading distribution of the antibody drug conjugate is as follows: in a population of antibody heavy chains, a proportion of antibody heavy chains binding to four drugs is 1 % or less, and a proportion of antibody heavy chains not binding to drugs is 4% or less; and in a population of antibody light chains, a proportion of antibody light chains binding to one drug is 70% or more.

Detailed Description of the Invention

The present disclosure provides a preparation method that is more conducive to large-scale production. Specifically, the product obtained by the preparation method has the advantages of narrower drug loading distribution, lower content of free toxin and higher yield.

Terms

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs.

The application PCT/CN2019/107873 is incorporated herein by reference in its entirety. The antibody drug conjugate (ADC) is formed by linking an antibody or an antibody fragment to a biologically-active cytotoxin or a small molecule drug with cell killing activity via a stable chemical linker compound, fully exploiting the specificity of the antibody to tumor cells or the binding specificity of high-expression antigen cells and the high efficiency of the cytotoxin, and avoiding toxic side effects on normal cells. The antibody drug conjugate can bind to tumor cells precisely and has a reduced effect on normal cells compared to conventional chemotherapeutic drugs in the past.

"Buffer" refers to a buffer that resists changes in pH by the action of its acid-base conjugate components. Examples of buffers that control the pH in an appropriate range include acetate, succinate, gluconate, histidine salt, oxalate, lactate, phosphate, citrate, tartrate, fumarate, glycylglycine and other organic acid buffers.

"Histidine salt buffer" is a buffer comprising histidine ions. Examples of histidine salt buffers include a histidine-hydrochloric acid buffer, a histidine-acetic acid buffer, a histidine-phosphoric acid buffer, a histidine-sulfuric acid buffer, and the like, and preferably a histidine-hydrochloric acid buffer, the histidine-acetic acid buffer being formulated with histidine and acetic acid, and the histidine-hydrochloric acid buffer being formulated with histidine and hydrochloric acid or histidine and histidine hydrochloride.

"Phosphate buffer" is a buffer comprising phosphate ions. Examples of phosphate buffers include a disodium hydrogen phosphate-sodium dihydrogen phosphate buffer, a disodium hydrogen phosphate-potassium dihydrogen phosphate buffer, a disodium hydrogen phosphate-citric acid buffer, and the like. The preferred phosphate buffer is the disodium hydrogen phosphate-sodium dihydrogen phosphate buffer.

"Acetate buffer" is a buffer comprising acetate ions. Examples of acetate buffers include an acetic acid-sodium acetate buffer, an acetic acid histidine salt buffer, an acetic acid-potassium acetate buffer, an acetic acid-calcium acetate buffer, an acetic acid-magnesium acetate buffer, and the like. The preferred acetate buffer is the acetic acid-sodium acetate buffer.

The terms "about" and "approximately" as used herein mean that a numerical value is within an acceptable error range for the particular value determined by one of ordinary skill in the art, and the numerical value depends in part on how the value is measured or determined (i.e., the limits of the measurement system). For example, "about" can mean within 1 or more than 1 standard deviation per the practice in the art. Or, "about" or "substantially comprising" may mean a range of up to 20%. Furthermore, particularly for biological systems or processes, the term can mean up to an order of magnitude or up to 5-fold of a numerical value. When a particular value is provided in the present application and claims, unless otherwise stated, the meaning of "about" or "substantially comprising" should be assumed to be within an acceptable error range for that particular value.

The three-letter and single-letter codes for amino acids used in the present disclosure are described as in J. Biol. Chem, 243, p3558 (1968).

The "antibody" described herein refers to an immunoglobulin, and is of a tetrapeptide chain structure formed by linking two identical heavy chains and two identical light chains of an intact antibody by interchain disulfide bonds. The heavy chain constant regions of an immunoglobulin differ in their amino acid composition and arrangement, and thus in their antigenicity. Accordingly, immunoglobulins can be divided into five classes, otherwise called isotypes of immunoglobulins, namely IgM, IgD, IgG, IgA and IgE, with their corresponding heavy chains being $\mu$ chain, $\delta$ chain, $\gamma$ chain, $\alpha$ chain and $\varepsilon$ chain, respectively. Ig of the same class can be divided into different subclasses according to differences in the amino acid composition of the hinge regions and the number and positions of disulfide bonds of the heavy chains; for example, IgG may be divided into IgG1, IgG2, IgG3 and IgG4. Light chains are classified into $\kappa$ or $\lambda$ chains by the differences in the constant regions. Each of the five classes of Ig may have a $\kappa$ chain or $\lambda$ chain. The antibody described in the present disclosure is preferably specific antibodies against cell surface antigens on target cells, non-limiting examples of which are one or more of the following: an anti-HER2 (ErbB2) antibody, an anti-EGFR antibody, an anti-B7-H3 antibody, an anti-c-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-Integrin antibody, an anti-PSMA antibody, an anti-Tenascin-C antibody, an anti-SLC44A4 antibody and an anti-Mesothelin antibody; the antibody is preferably trastuzumab (trade name: Herceptin), pertuzumab (also known as 2C4, trade name: Perjeta), nimotuzumab (trade name: Taixinsheng®), enoblituzumab, emibetuzumab, inotuzumab, pinatuzumab, brentuximab, gemtuzumab, bivatuzumab, lorvotuzumab, cBR96 or glematumamab.

In the heavy and light chains of the antibody, the sequences of about 110 amino acids near the N-terminus vary considerably and thus are referred to as variable regions (Fv regions); the remaining amino acid sequences near the C-terminus are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs arranged from the amino-terminus to the carboxy-terminus in the following order: FR1, CDR1, FR2, CDR2, FR3, CDR3 and FR4. The 3 CDRs of the light chain refer to LCDR1, LCDR2 and LCDR3, and the 3 CDRs of the heavy chain refer to HCDR1, HCDR2 and HCDR3. CDR amino acid residues of the LCVR and HCVR regions of the antibodies or antigen-binding fragments described in the present disclosure correspond with known Kabat numbering scheme (LCDRs 1-3, HCDRs 1-3) in terms of number and positions.

In the present disclosure, the antibody light chain of the present disclosure may further comprise a light chain constant region comprising a human or murine $\kappa$ and $\lambda$ chains or variants thereof.

In the present disclosure, the antibody heavy chain of the present disclosure may further comprise a heavy chain constant region comprising human or murine IgG1, IgG2, IgG3 and IgG4 or variants thereof.

The antibody of the present disclosure includes a murine antibody, a chimeric antibody and a humanized antibody, and preferably a humanized antibody.

The term "murine antibody" used herein refers to an antibody prepared from mice according to the knowledge and skill in the art. During the preparation, a test subject is injected with a specific antigen, and then hybridomas expressing antibodies with desired sequence or functional properties are isolated.

The term "chimeric antibody" refers to an antibody obtained by fusing a variable region of a murine antibody and a constant region of a human antibody, which can reduce an immune response induced by the murine antibody.

The chimeric antibody is established by firstly establishing hybridoma secreting murine specific monoclonal antibody, then cloning a variable region gene from the mouse hybridoma cells, cloning a constant region gene of human antibody as required, linking the mouse variable region gene and the human constant region gene into a chimeric gene, inserting the chimeric gene into an expression vector, and finally expressing chimeric antibody molecules in a eukaryotic system or prokaryotic system.

The term "humanized antibody", also known as a CDR-grafted antibody, refers to an antibody produced by grafting murine CDR sequences into a human antibody variable region framework, i.e., a different type of human species antibody framework sequence. Such antibody can overcome the strong heterogeneous reaction induced by the chimeric antibody because of carrying a large amount of mouse protein components. Such framework sequences can be obtained from public DNA databases or published references that include germline antibody gene sequences. For example, germline DNA sequences of genes of the human heavy and light chain variable regions can be found in the "VBase" human species sequence database, as well as in Kabat, E. A. et al., 1991 Sequences of Proteins of Immunological Interest, 5th edition. To avoid the decrease in activity caused by the decrease in immunogenicity, the FR sequence in human antibody variable region can be subjected to minimum reverse mutation or back mutation to maintain activity. The humanized antibody of the present disclosure also include humanized antibodies which were further subjected to CDR affinity maturation by phage display.

The term "naked antibody" refers to an antibody that is not conjugated to a heterologous moiety (e.g., a cytotoxic moiety) or a radioactive label.

The "antigen-binding fragment of an antibody" described herein may refer to Fab fragments, Fab' fragments and $F(ab')_2$ fragments having antigen-binding activity, and Fv fragments and scFv fragments binding to an antigen. The Fv fragment comprises the heavy chain variable region and the light chain variable region of the antibody, but no the constant region, and has the smallest antibody fragment of the entire antigen-binding sites. Generally, the Fv antibody also comprises a polypeptide linker between the VH and VL domains, and is capable of forming the structure required for antigen binding. Two antibody variable regions can also be linked into a single polypeptide chain using different linkers, known as single chain antibody or single chain fv (sFv).

The term "antigen-binding site" disclosed herein refers to a continuous or discontinuous three-dimensional spatial site on an antigen that is recognized by an antibody or an antigen-binding fragment of the present disclosure.

The "ADCC", i.e., antibody-dependent cell-mediated cytotoxicity described herein, refers to the direct killing of antibody-coated target cells by Fc receptor-expressing cells through recognition of the Fc segment of the antibody. The ADCC effector function of the antibody may be reduced or eliminated by modification of the Fc segment of the IgG. The modification refers to a mutation in the heavy chain constant region of the antibody, such as a mutation selected from the group consisting of N297A, L234A and L235A of IgG1; IgG2/4 chimera, and F234A/L235A of IgG4.

The "mutations" in the mutant sequences described herein include, but are not limited to, "back mutation", "conservative modification" or "conservative replacement or substitution". The "conservative modification" or "conservative replacement or substitution" described herein refers to replacement of amino acids in a protein with other amino acids having similar characteristics (e.g., charge, side-chain size, hydrophobicity/hydrophilicity, or backbone conformation and rigidity), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)). In addition, the replacement of amino acids with similar structure or function is unlikely to disrupt the biological activity.

The term "mutant sequence" described herein refers to a nucleotide sequence and/or amino acid sequence having a different degree of percent sequence identity to the nucleotide sequence and/or amino acid sequence of the present disclosure, when mutational modifications such as replacements, insertions or deletions are appropriately made to the nucleotide sequence and/or amino acid sequence of the present disclosure. The sequence identity described herein may be at least 85%, 90% or 95%, and preferably at least 95%. Non-limiting examples of the sequence identity include 85%, 86%, 87%, 88%, 89%, 90%, 91%, 92%, 93%, 94%, 95%, 96%, 97%, 98%, 99% and 100%. Sequence comparison and percent identity determination between two sequences can be performed by the default settings for the BLASTN/BLASTP algorithm available on the website National Center For Biotechnology Institute.

The term "linker unit" or "linker fragment" refers to a chemical structure fragment or bond, which is linked to an antibody or antigen-binding fragment thereof at one end and to a drug at the other end, and also may be linked to an antibody or drug after being linked to another linker.

The linker includes stretcher units, spacer units and amino acid units, and may be synthesized using methods known in the art, such as those described in US2005-0238649A1. The linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research 52: 127-131(1992); U.S. Patent No. 5,208,020).

The engineered antibody or antigen-binding fragment of the present disclosure can be prepared and purified

using conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems may result in glycosylation of antibodies, particularly at the highly conserved N-terminal site of the Fc region. Positive clones are expanded in a serum-free medium of a bioreactor to produce antibodies. The culture with the secreted antibody can be purified using conventional techniques, for example, purification is carried out on an A or G Sepharose FF column containing an adjusted buffer. Non-specifically bound fractions are washed away. The bound antibody is eluted using pH gradient method, and the antibody fragments are detected by SDS-PAGE and collected. The antibody can be filtered and concentrated using conventional methods. Soluble mixtures and polymers can also be removed using conventional methods, such as molecular sieves and ion exchange. The resulting product needs to be immediately frozen, e.g., at -70 °C, or lyophilized.

The term "alkyl" refers to a saturated aliphatic hydrocarbon group that is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms. Non-limiting examples of alkyl include methyl, ethyl, n-propyl, isopropyl, n-butyl, isobutyl, *tert-butyl, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. More preferred is a lower alkyl having 1 to 6 carbon atoms, and non-limiting examples of lower alkyl include methyl, ethyl, *n*-propyl, isopropyl, n-butyl, isobutyl, *tert-butyl, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. Alkyl may be substituted or unsubstituted. When substituted, the substituent may be substituted at any available connection site, wherein the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "heteroalkyl" refers to alkyl containing one or more heteroatoms selected from the group consisting of N, O and S, wherein the alkyl is as defined above.

The term "alkylene" refers to a saturated linear or branched aliphatic hydrocarbon group having 2 residues derived from the parent alkane by removal of two hydrogen atoms from the same carbon atom or two different carbon atoms. It is a linear or branched group containing 1 to 20 carbon atoms, preferably alkylene containing 1 to 12 carbon atoms, and more preferably alkylene containing 1 to 6 carbon atoms. Non-limiting examples of alkylene include, but are not limited to, methylene($-CH_2-$), 1,1-ethylidene($-CH(CH_3)-$), 1,2-ethylidene($-CH_2CH_2-$), 1,1-propylidene($-CH(CH_2CH_3)-$), 1,2-propylidene($-CH_2CH(CH_3)-$), 1,3-propylidene($-CH_2CH_2CH_2-$), 1,4-butylidene($-CH_2CH_2CH_2CH_2-$), 1,5-butylidene($-CH_2CH_2CH_2CH_2CH_2-$), etc. The alkylene may be substituted or unsubstituted. When substituted, the substituent may be substituted at any available connection site with one or more substituents preferably independently optionally selected from the group consisting of alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropyloxy, cyclobutoxy, cyclopentyloxy and cyclohexyloxy. The alkoxy may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, more preferably 3 to 10 carbon atoms, and most preferably 3 to 7 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "heterocyclyl" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen and $S(O)_m$ (where m is an integer from 0 to 2), excluding a cyclic portion of -O-O-, -O-S- or -S-S-, and the remaining ring atoms are carbon atoms. Heterocycloalkyl preferably contains 3 to 12 ring atoms,

of which 1 to 4 are heteroatoms; more preferably, a cycloalkyl ring contains 3 to 10 ring atoms. Non-limiting examples of monocyclic heterocyclyl include pyrrolidinyl, piperidinyl, piperazinyl, morpholinyl, thiomorpholinyl, homopiperazinyl, etc. Non-limiting examples of polycyclic heterocyclyl include spiro heterocyclyl, fused heterocyclyl, and bridged heterocyclyl. The term "spiro heterocyclyl" refers to a 5- to 20-membered polycyclic heterocyclyl group in which monocyclic rings share one atom (referred to as the spiro atom), wherein one or more ring atoms are heteroatoms selected from the group consisting of nitrogen, oxygen and $S(O)_m$ (where m is an integer from 0 to 2), and the remaining ring atoms are carbon atoms. These rings may contain one or more double bonds, but none of them has a fully conjugated $\pi$-electron system. Preferably, the bridged heterocyclyl is 6- to 14-membered, and more preferably 7- to 10-membered. According to the number of spiro atoms shared among the rings, the spiro heterocyclyl may be monospiro heterocyclyl, bispiro heterocyclyl or polyspiro heterocyclyl, preferably monospiro heterocyclyl and bispiro heterocyclyl, and more preferably 4-membered/4-membered, 4-membered/5-membered, 4-membered/6-membered, 5-membered/5-membered or 5-membered/6-membered monospiro heterocyclyl. Non-limiting examples of spiro heterocyclyl include:

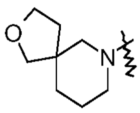 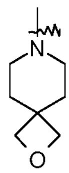 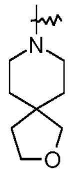 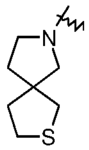 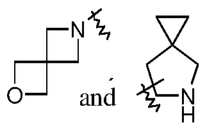 , and .

The term "fused heterocyclyl" refers to a 5- to 20-membered polycyclic heterocyclyl group in which each ring shares a pair of adjacent atoms with the other rings in the system, wherein one or more of the rings may contain one or more double bonds, but none of them has a fully conjugated $\pi$-electron system, wherein one or more of the ring atoms are heteroatoms selected from the group consisting of nitrogen, oxygen and $S(O)_m$ (where m is an integer from 0 to 2), and the remaining ring atoms are carbon atoms. Preferably, the bridged heterocyclyl is 6- to 14-membered, and more preferably 7- to 10-membered. According to the number of the formed rings, the fused heterocyclyl may be bicyclic, tricyclic, tetracyclic or polycyclic fused heterocyclyl, preferably bicyclic or tricyclic fused heterocyclyl, and more preferably 5-membered/5-membered or 5-membered/6-membered bicyclic fused heterocyclyl. Non-limiting examples of fused heterocyclyl include:

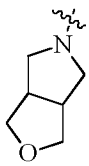 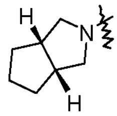 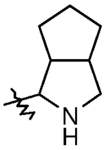 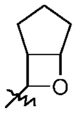 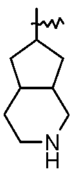 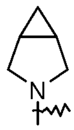 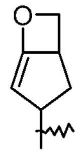 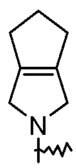 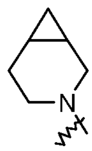

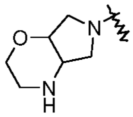 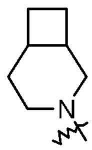 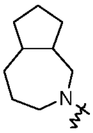 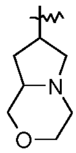 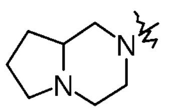 and 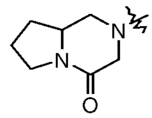 .

The term "bridged heterocyclyl" refers to a 5- to 14-membered polycyclic heterocyclyl group in which any two rings share two atoms that are not directly connected to each other, wherein these rings may contain one or more double bonds, but none of them has a fully conjugated $\pi$-electron system, wherein one or more of the ring atoms are heteroatoms selected from the group consisting of nitrogen, oxygen and $S(O)_m$ (where m is an integer from 0 to 2), and the remaining ring atoms are carbon atoms. Preferably, the bridged heterocyclyl is 6- to 14-membered, and more preferably 7- to 10-membered. According to the number of the formed rings, the bridged heterocyclyl may be bicyclic, tricyclic, tetracyclic or polycyclic bridged heterocyclyl, preferably bicyclic, tricyclic or tetracyclic bridged heterocyclyl, and more preferably bicyclic or tricyclic bridged heterocyclyl. Non-limiting examples of bridged heterocyclyl include:

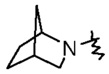 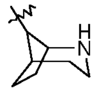 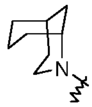 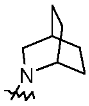

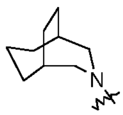 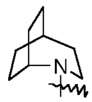 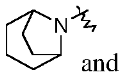 and 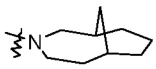 .

The heterocyclyl ring may be fused to an aryl, heteroaryl or cycloalkyl ring, wherein the ring connected to the parent structure is heterocyclyl. Non-limiting examples of heterocyclyl ring include, but are not limited to:

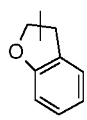 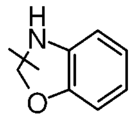 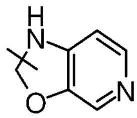 , 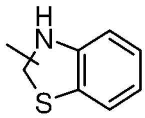 , etc.

Heterocyclyl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "aryl" refers to a 6- to 14-membered, preferably 6- to 10-membered, carbon monocyclic or fused polycyclic (i.e., rings sharing a pair of adjacent carbon atoms) group having a conjugated π-electron system, such as phenyl and naphthyl, preferably phenyl. The aryl ring may be fused to a heteroaryl, heterocyclyl or cycloalkyl ring, wherein the ring connected to the parent structure is the aryl ring. Non-limiting examples of aryl ring include, but are not limited to:

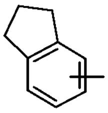 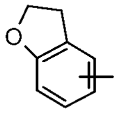 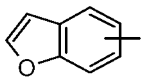 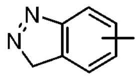 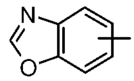 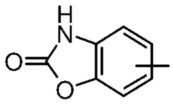 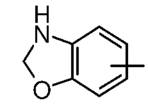

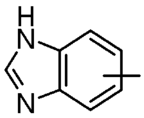 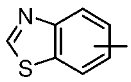 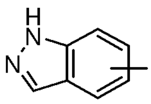 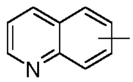 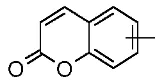 and 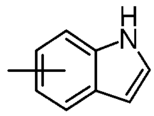 .

Aryl may be substituted or unsubstituted, and when it is substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "heteroaryl" refers to a heteroaromatic system containing 1 to 4 heteroatoms and 5 to 14 ring atoms, wherein the heteroatoms are selected from the group consisting of oxygen, sulfur and nitrogen. The heteroaryl is preferably 5- to 10-membered, more preferably 5- or 6-membered, such as furanyl, thienyl, pyridinyl, pyrrolyl, N-alkylpyrrolyl, pyrimidinyl, pyrazinyl, imidazolyl and tetrazolyl. The heteroaryl ring may be fused to an aryl, heterocyclyl or cycloalkyl ring, wherein the ring connected to the parent structure is a heteroaryl ring. Non-limiting examples of the heteroaryl ring include:

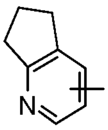 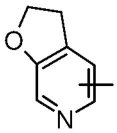 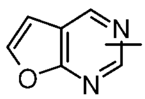 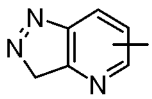 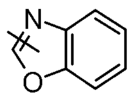 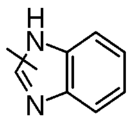 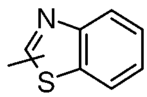

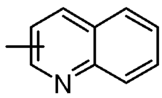 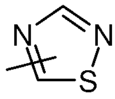 and 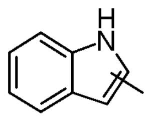 .

Heteroaryl may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "amino protecting group" refers to a group that can be easily removed and is intended to protect an amino group from being changed when a reaction is conducted elsewhere in the molecule. Non-limiting examples of the amino protecting group include 9-fluorenylmethoxycarbonyl, *tert*-butoxycarbonyl, acetyl, benzyl, allyl, p-methoxybenzyl, etc. These groups may be optionally substituted with 1 to 3 substituents selected from the group consisting of halogen, alkoxy and nitro. The amino protecting group is preferably 9-fluorenylmethoxycarbonyl.

The term "cycloalkylalkyl" refers to alkyl substituted with one or more cycloalkyl groups, preferably one cycloalkyl group, wherein the alkyl is as defined above, and the cycloalkyl is as defined above.

The term "haloalkyl" refers to alkyl substituted with one or more halogens, wherein the alkyl is as defined above.

The term "deuterated alkyl" refers to alkyl substituted with one or more deuterium atoms, wherein the alkyl is as defined above.

The term "hydroxy" refers to -OH group.

The term "halogen" refers to fluorine, chlorine, bromine or iodine.

The term "amino" refers to $-NH_2$.

The term "nitro" refers to $-NO_2$.

The term "cyano" refers to -CN.

The term "optional" or "optionally" means that the event or circumstance subsequently described may, but not necessarily, occur, and that the description includes instances where the event or circumstance occurs or does not occur. For example, "optionally comprising 1-3 antibody heavy chain variable regions" means that the antibody heavy chain variable region of a particular sequence may, but not necessarily, be present.

The term "substituted" means that one or more, preferably up to 5, more preferably 1 to 3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue efforts. For example, it may be unstable when amino or hydroxy having a free hydrogen is bound to a carbon atom having an unsaturated (e.g., olefinic) bond.

The term "drug loading" (DAR) refers to the average amount of cytotoxic drug loaded on each antibody or antigen-binding fragment thereof in an ADC, and may also be represented as a drug-to-antibody ratio, which is an integer or a decimal. In embodiments of the present disclosure, the drug loading is represented as n, and exemplary values may be a mean of 1, 2, 3, 4, 5, 6, 7, 8, 9 and 10. The mean number of drugs per ADC molecule after coupling reactions can be characterized by conventional methods such as UV/visible spectroscopy, mass spectrometry, ELISA assays and HPLC.

The term "drug loading distribution" refers to the distribution of antibodies linked to different numbers of drugs in a population of antibody drug conjugates, e.g., the distribution of antibodies linked to 0, 2, 4, 6 and 8 drugs in a population. Notably, DAR of 1, 3, 5 and 7 drugs may also be included in the mixture due to the potential generation of degradation products. In the present disclosure, drug loading distribution of antibodies can be characterized using antibody heavy chains binding to different numbers of drugs, for example: $H_0$ represents a heavy chain not binding to drugs, $H_1$ represents a heavy chain binding to one drug, $H_2$ represents a heavy chain binding to two drugs, $H_3$ represents a heavy chain binding to three drugs, and $H_4$ represents a heavy chain binding to four drugs. Illustratively, a proportion of $H_3$ of 4% means that in a population of heavy chains of antibody drug conjugates, a proportion of the heavy chains binding to three drugs is 4%. Accordingly, the drug loading distribution of antibodies in the present disclosure can also be characterized using antibody light chains binding to different numbers of drugs, $L_0$ representing an antibody light chain not binding to drugs and $L_1$ representing an antibody light chain binding to one drug.

"Giving", "administering" and "treating", when applied to animals, humans, experimental subjects, cells, tissues, organs or biological fluid, refer to contact of an exogenous drug, a therapeutic agent, a diagnostic agent or a composition with the animals, humans, subjects, cells, tissues, organs or biological fluid. "Giving", "administering," and "treating" can refer to, for example, therapeutic, pharmacokinetic, diagnostic, research, and experimental methods. The treatment of the cells comprises contacting the reagent with the cells and contacting the reagent with fluid, wherein the fluid is in contact with the cells. "Giving", "administering" and "treating" also refer to treating, e.g., a cell, by a reagent, diagnosis, a binding composition, or by another cell *in vitro* and *ex vivo.* "Treating", when applied to human, veterinary or research subjects, refers to therapeutic treatment, preventive or prophylactic measures, and research and diagnostic applications.

"Treating" or "treatment" refers to administering a therapeutic agent, such as a composition comprising any one of the conjugation compounds of the present disclosure, either internally or externally to a patient with one or more symptoms of a disease on which the therapeutic agent is known to have a therapeutic effect. Typically, the therapeutic agent is administered in an amount effective to alleviate one or more symptoms of the disease in the patient or population being treated to induce regression of such symptoms or to inhibit the development of such symptoms to any clinically

measurable degree. The amount of therapeutic agent effective to alleviate the symptoms of any particular disease (also referred to as a "therapeutically effective amount") may vary depending on a variety of factors, such as the disease state, age, and weight of the patient, and the ability of the drug to produce a desired therapeutic effect in the patient.

Whether a symptom of a disease has been alleviated can be evaluated by any clinical testing methods commonly used by doctors or other health care professionals to evaluate the severity or progression of the symptom. Although the embodiments of the present disclosure (for example, treatment methods or products) may not be effective in alleviating the symptoms of each disease of interest, they shall reduce the symptoms of a disease of interest in a statistically significant number of patients, as determined according to any statistical testing methods known in the art, such as Student t-test, chi-square test, Mann and Whitney's U test, Kruskal-Wallis test (H test), Jonckheere-Terpstra test and Wilcoxon test.

"Effective amount" comprises an amount sufficient to ameliorate or prevent a symptom or condition of a medical disease. An effective amount also refers to an amount sufficient to allow or facilitate diagnosis. The effective amount for a particular patient or veterinary subject may vary depending on the factors such as the condition to be treated, the general health of the patient, the method and route and dosage of administration, and the severity of side effects. An effective amount can be the maximum dose or administration regimen to avoid significant side effects or toxic effects.

"Exchange" refers to the exchange of a solvent system that solubilizes an antibody protein. For example, a high-salt or hypertonic solvent system comprising the antibody protein is exchanged, by physical operations, with a buffer system of a stable formulation, such that the antibody protein is present in the stable formulation. The physical operations include, but are not limited to, ultrafiltration, dialysis or reconstitution following centrifugation.

## BRIEF DESCRIPTION OF THE DRAWINGS

FIG. 1A shows the results of plasma stability test for ADC-19 of the present disclosure.
FIG. 1B shows the results of plasma stability test for ADC-18 of the present disclosure.
FIG. 1C shows the results of plasma stability test for ADC-20 of the present disclosure.
FIG. 2 shows the evaluation of the efficacy of ADC-21 and ADC-24 on JIMT-1 tumor-bearing mice.
FIG. 3 shows the evaluation of the therapeutic effect of ADCs on human breast cancer cell SK-BR-3 xenograft tumor nude mice.
FIG. 4 shows the results of plasma stability test for ADC-25 of the present disclosure.
FIG. 5 shows the therapeutic effect of ADCs on xenograft tumors of human brain astrocytoma U87MG nude mice.
FIG. 6 shows the therapeutic effect of ADCs on xenograft tumors of human pharyngeal cancer hydrothorax metastatic cells Detroit 562 nude mice.
FIG. 7 shows the therapeutic effect of ADCs on xenograft tumors of human glioblastoma U87MG nude mice.

## DETAILED DESCRIPTION

The present disclosure is further described below with reference to examples, but these examples are not intended to limit the scope of the present invention.

Experimental procedures without specific conditions indicated in the examples of the present disclosure, are generally conducted according to conventional conditions, or according to conditions recommended by the manufacturer of the starting materials or commercial products. Reagents without specific origins indicated are commercially available conventional reagents.

I. Preparation of antibody drug conjugate and property and biological test thereof

The structure of the compounds were determined by nuclear magnetic resonance (NMR) or mass spectrometry (MS). NMR spectra were measured using a Bruker AVANCE-400 nuclear magnetic resonance instrument, with deuterated dimethyl sulfoxide (*DMSO-d6*), deuterated chloroform ($CDCl_3$) and deuterated methanol ($CD_3OD$) as determination solvents and tetramethylsilane (TMS) as internal standard. Chemical shifts were given in unit of $10^{-6}$ (ppm).

MS spectra were measured using a FINNIGAN LCQAd (ESI) mass spectrometer (manufacturer: Thermo, model: Finnigan LCQ advantage MAX).

UPLC analysis was performed using a Waters Acquity UPLC SQD liquid chromatography-mass spectrometry system.

HPLC analysis was performed using an Agilent 1200DAD high pressure liquid chromatograph (Sunfire C18 150 × 4.6 mm chromatography column) and a Waters 2695-2996 high pressure liquid chromatograph (Gimini C18 150 × 4.6 mm chromatography column).

UV-HPLC analysis was performed using a Thermo nanodrop2000 ultraviolet spectrophotometer.

Proliferation inhibition rates and $IC_{50}$ values were measured using a PHERA starFS microplate reader (BMG, Germany).

Yantai Huanghai HSGF254 or Qingdao GF254 silica gel plates of specifications 0.15 mm to 0.2 mm were adopted for thin layer chromatography (TLC) analysis and 0.4 mm to 0.5 mm for TLC separation and purification.

Yantai Huanghai silica gel of 200-300 mesh is generally used as a carrier in column chromatography.

Known starting materials of the present disclosure may be synthesized using or according to methods known in the art, or may be purchased from ABCR GmbH & Co.KG, Acros Organnics, Aldrich Chemical Company, Accela ChemBio Inc, Chembee Chemicals, etc.

In the Examples, the reactions were performed in an argon atmosphere or a nitrogen atmosphere unless otherwise stated.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Parr 3916EKX hydrogenator, Qinglan QL-500 hydrogenator or HC2-SS hydrogenator was used in the pressurized hydrogenation reactions.

The hydrogenation reactions usually involve 3 cycles of vacuumization and hydrogen purge.

A CEM Discover-S 908860 microwave reactor was used in the microwave reactions.

In the Examples, the solution in the reaction refers to an aqueous solution unless otherwise stated.

In the Examples, the reaction temperature is room temperature unless otherwise stated. The room temperature is the optimum reaction temperature, which ranges from 20 °C to 30 °C.

Preparation of PBS buffer at pH 6.5 in Examples: 8.5 g of $KH_2PO_4$, 8.56 g of $K_2HPO_4.3H_2O$, 5.85 g of NaCl and 1.5 g of EDTA were added to a flask, and the volume was brought to 2 L. The additions were all ultrasonically dissolved, and the solution was well mixed by shaking to give the desired buffer.

The eluent system for column chromatography and the developing solvent system for thin layer chromatography used for compound purification include: A: dichloromethane and isopropanol system, B: dichloromethane and methanol system, and C: petroleum ether and ethyl acetate system. The volume ratio of solvents was adjusted according to the polarity of the compound, or by adding a small amount of triethylamine and acidic or basic reagent.

Some of the compounds of the present disclosure are characterized by Q-TOF LC/MS. Q-TOF LC/MS analysis used an Agilent 6530 accurate-mass quadrupole time-of-flight mass spectrometer and an Agilent 1290-Infinity ultra-high performance liquid chromatograph (Agilent Poroshell 300SB-C8 5 μm, 2.1 × 75 mm chromatography column).

Example 1-1

*N*-((1*S*,9*S*)-9-ethyl-5-fluoro-9-hydroxy-4-methyl-10,13-dioxo-2,3,9,10,13,15-hexahydro -1*H*,12*H*-benzo[*de*]pyrano[3',4':6,7]indolizino[1,2-*b*]quinolin-1-yl)-1-hydroxycycloprop ane-1-carboxamide 1

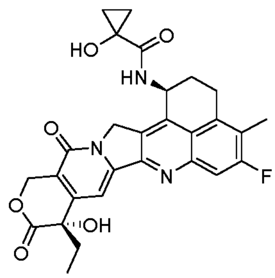

**1**

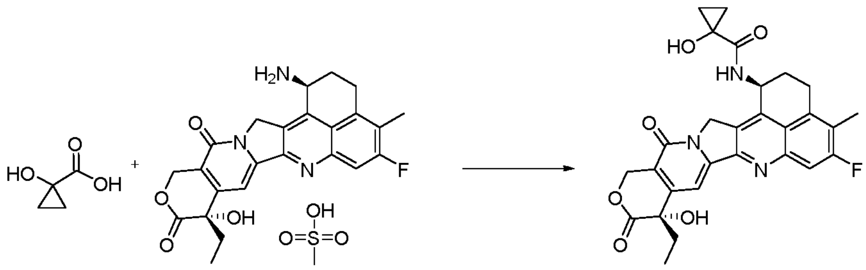

**1a** **1b** **1**

To exatecan mesylate 1b (2.0 mg, 3.76 μmol, prepared using the method disclosed in Patent Application "EP0737686A1") was added 1 mL of N,N-dimethylformamide. The mixture was cooled to 0-5 °C in an ice-water bath, followed by addition of a drop of triethylamine. The reaction solution was stirred until it became clear. To the reaction solution were successively added 1-hydroxycyclopropylcarboxylic acid 1a (1.4 mg, 3.7 μmol, prepared using known method "Tetrahedron Letters, 25(12), 1269-72; 1984") and 4-(4,6-dimethoxy-1,3,5-triazin-2-yl)-4-methylmorpholinium chloride (3.8 mg, 13.7 μmol). After the addition was completed, the reaction solution was stirred at 0-5 °C for 2 h, quenched with 5 mL of water, and extracted with ethyl acetate (8 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product 1 (1.6 mg, 82.1% yield).

MS m/z (ESI): 520.2 [M+1].

$^1$H NMR (400 MHz, $CDCl_3$): δ 7.90-7.84 (m, 1H), 7.80-7.68(m, 1H), 5.80-5.70 (m, 1H), 5.62-5.54(m, 2H), 5.44-5.32 (m, 2H), 5.28-5.10(m, 2H), 3.40-3.15 (m, 3H), 2.44 (s, 3H), 2.23(t, 1H), 2.06-1.75 (m, 2H), 1.68-1.56 (m, 1H), 1.22-1.18 (m, 2H), 1.04-0.98 (m, 2H), 0.89 (t, 3H).

Example 1-2

(*S*)-2-cyclopropyl-*N*-((1*S*,9*S*)-9-ethyl-5-fluoro-9-hydroxy-4-methyl-10,13-dioxo-2,3,9,1 0,13,15-hexahydro-1*H*,12*H*-benzo[*de*]pyrano[3',4':6,7]indolizino[1,2-*b*]quinolin-1-yl)-2 -hydroxyacetamide **2-A**

(*R*)-2-cyclopropyl-*N*-((1*S*,9*S*)-9-ethyl-5-fluoro-9-hydroxy-4-methyl-10,13-dioxo-2,3,9,1 0,13,15-hexahydro-1*H*,12*H*-benzo[*de*]pyrano[3',4':6,7]indolizino[1,2-*b*]quinolin-1-yl)-2 -hydroxyacetamide **2-B**

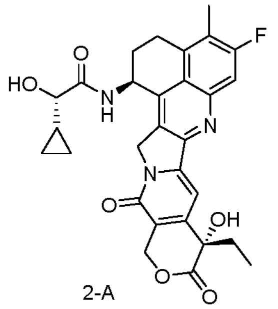
2-A

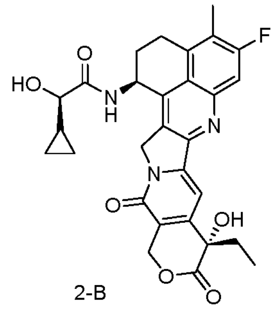
2-B

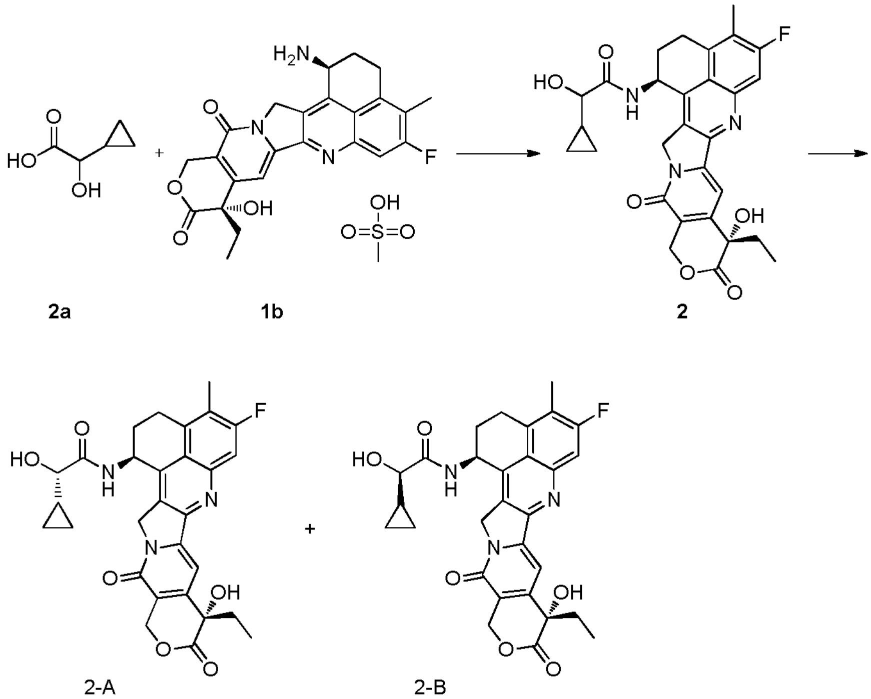
2a 1b 2

2-A 2-B

To **1b** (4 mg, 7.53 μmol) were added 2 mL of ethanol and 0.4 mL of N,N-dimethylformamide. The mixture was purged with argon three times, and cooled to 0-5 °C in an ice-water bath, followed by dropwise addition of 0.3 mL of N-methylmorpholine. The reaction solution was stirred until it became clear. To the reaction solution were successively added 2-cyclopropyl-2-hydroxyacetic acid 2a (2.3 mg, 19.8 μmol, prepared using the method disclosed in Patent Application "WO2013106717"), 1-hydroxybenzotriazole (3 mg, 22.4 μmol) and 1-(3-dimethylaminopropyl)-3-ethylcarbodiimide hydrochloride (4.3 mg, 22.4 μmol). After the addition was completed, the reaction solution was stirred at 0-5 °C for 1 h. The ice-water bath was removed, and the reaction solution was heated to 30 °C, stirred for 2 h, and concentrated under reduced pressure. The resulting crude compound **2** was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min), and the corresponding fractions were collected and concentrated under reduced pressure to give the title product (2-A: 1.5 mg, 2-B: 1.5 mg).

MS m/z (ESI): 534.0.[M+1].

Single-configuration compound 2-B (shorter retention time)

UPLC analysis: retention time: 1.06 min; purity: 88% (chromatography column: ACQUITY UPLC BEHC18 1.7 μm 2.1 × 50 mm; mobile phase: A-water (5 mmol of $NH_4OAc$), B-acetonitrile).

$^1H$ NMR (400 MHz, DMSO-$d_6$): δ 8.37 (d, 1H), 7.76 (d, 1H), 7.30 (s, 1H), 6.51 (s, 1H), 5.58-5.56 (m, 1H), 5.48 (d, 1H), 5.41 (s, 2H), 5.32-5.29 (m, 2H), 3.60 (t, 1H), 3.19-3.13 (m, 1H), 2.38 (s, 3H), 2.20-2.14 (m, 1H), 1.98 (q, 2H), 1.87-1.83 (m, 1H), 1.50-1.40 (m, 1H), 1.34-1.28 (m, 1H), 0.86 (t, 3H), 0.50-0.39 (m, 4H).

Single-configuration compound 2-A (longer retention time)

UPLC analysis: retention time: 1.10 min; purity: 86% (chromatography column: ACQUITY UPLC BEHC18 1.7

μm 2.1 × 50 mm; mobile phase: A-water (5 mmol of $NH_4OAc$), B-acetonitrile).

$^1$H NMR (400 MHz, DMSO-$d_6$): δ 8.35 (d, 1H), 7.78 (d, 1H), 7.31 (s, 1H), 6.52 (s, 1H), 5.58-5.53 (m, 1H), 5.42 (s, 2H), 5.37 (d, 1H), 5.32 (t, 1H), 3.62 (t, 1H), 3.20-3.15 (m, 2H), 2.40 (s, 3H), 2.25-2.16 (m, 1H), 1.98 (q, 2H), 1.87-1.82 (m, 1H), 1.50-1.40 (m, 1H), 1.21-1.14 (m, 1H), 0.87 (t, 3H), 0.47-0.35 (m, 4H).

Example 1-3

(*S*)-*N*-((1*S*,9*S*)-9-ethyl-5-fluoro-9-hydroxy-4-methyl-10,13-dioxo-2,3,9,10,13,15-hexah ydro-1*H*,12*H*-benzo[*de*]pyrano[3',4':6,7]indolizino[1,2-*b*]quinolin-1-yl)-3,3,3-trifluoro-2-hydroxypropionamide **3-A**

(*R*)-*N*-((1*S*,9*S*)-9-ethyl-5-fluoro-9-hydroxy-4-methyl-10,13-dioxo-2,3,9,10,13,15-hexah ydro-1*H*,12*H*-benzo[*de*]pyrano[3',4':6,7]indolizino[1,2-*b*]quinolin-1-yl)-3,3,3-trifluoro-2-hydroxypropionamide **3-B**

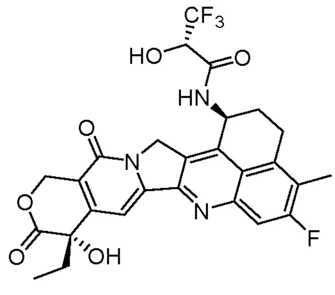

3-A

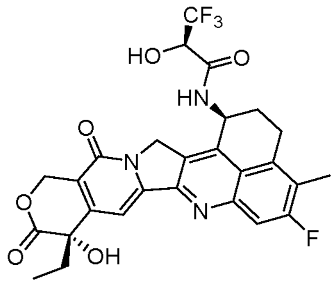

3-B

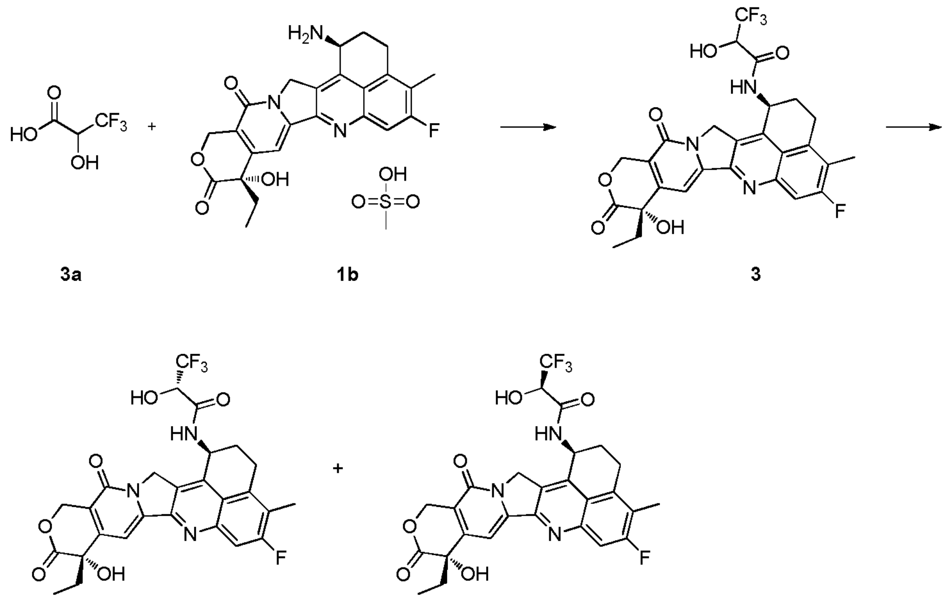

**3a** **1b** **3**

3-A 3-B

To **1b** (5.0 mg, 9.41 μmol) were added 2 mL of ethanol and 0.4 mL of N,N-dimethylformamide. The mixture was cooled to 0-5 °C in an ice-water bath, followed by dropwise addition of 0.3 mL of N-methylmorpholine. The reaction solution was stirred until it became clear. To the reaction solution were successively added 3,3,3-trifluoro-2-hydroxypropionic acid **3a** (4.1 mg, 28.4 μmol, supplier Alfa), 1-hydroxybenzotriazole (3.8 mg, 28.1 μmol) and 1-(3-dimethylaminopropyl)-3-ethylcarbodiimide hydrochloride (5.4 mg, 28.2 μmol). After the addition was completed, the reaction solution was stirred at 0-5 °C for 10 min. The ice-water bath was removed, and the reaction solution was heated to 30 °C, stirred for 8 h, and concentrated under reduced pressure. The resulting crude compound **3** was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min), and the corresponding

fractions were collected and concentrated under reduced pressure to give the title product (1.5 mg, 1.5 mg).

MS m/z (ESI): 561.9.[M+1].

Single-configuration compound (shorter retention time)

UPLC analysis: retention time: 1.11 min; purity: 88% (chromatography column: ACQUITY UPLC BEHC18 1.7 μm 2.1 × 50 mm; mobile phase: A-water (5 mmol of $NH_4OAc$), B-acetonitrile).

$^1H$ NMR (400 MHz, DMSO-$d_6$): δ 8.94 (d, 1H), 7.80 (d, 1H), 7.32 (s, 1H), 7.20 (d, 1H), 6.53 (s, 1H), 5.61-5.55 (m, 1H), 5.45-5.23 (m, 3H), 5.15-5.06 (m, 1H), 4.66-4.57 (m, 1H), 3.18-3.12 (m, 1H), 2.40 (s, 3H), 2.26-2.20 (m, 1H), 2.16-2.08 (m, 1H), 2.02-1.94 (m, 1H), 1.89-1.82 (m, 1H), 1.50-1.40 (m, 1H), 0.87 (t, 3H).

Single-configuration compound (longer retention time)

UPLC analysis: retention time: 1.19 min; purity: 90% (chromatography column: ACQUITY UPLC BEHC18 1.7 μm 2.1 × 50 mm; mobile phase: A-water (5 mmol of $NH_4OAc$), B-acetonitrile).

$^1H$ NMR (400 MHz, DMSO-$d_6$): δ 8.97 (d, 1H), 7.80 (d, 1H), 7.31 (s, 1H), 7.16 (d, 1H), 6.53 (s, 1H), 5.63-5.55 (m, 1H), 5.45-5.20 (m, 3H), 5.16-5.07 (m, 1H), 4.66-4.57 (m, 1H), 3.18-3.12 (m, 1H), 2.40 (s, 3H), 2.22-2.14 (m, 1H), 2.04-1.95 (m, 2H), 1.89-1.82 (m, 1H), 1.50-1.40 (m, 1H), 0.87 (t, 3H).

Example 1-4

*N*-((1*S*,9*S*)-9-ethyl-5-fluoro-9-hydroxy-4-methyl-10,13-dioxo-2,3,9,10,13,15-hexahydro -1*H*,12*H*-benzo[*de*]pyrano[3',4':6,7]indolizino[1,2-*b*]quinolin-1-yl)-1-hydroxycyclopent ane-1-carboxamide **4**

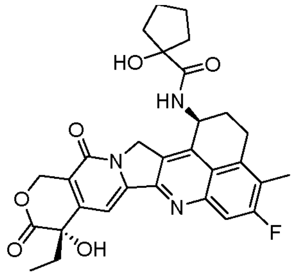

**4**

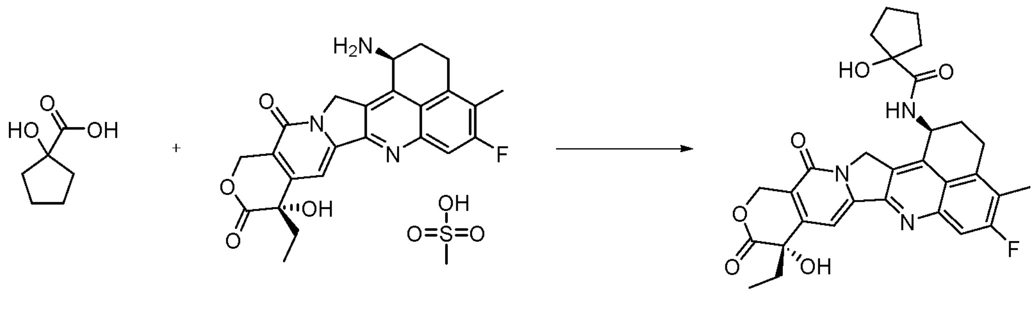

**4a** **1b** **4**

To **1b** (3.0 mg, 5.64 μmol) was added 1 mL of *N,N*-dimethylformamide. The mixture was cooled to 0-5 °C in an ice-water bath, followed by addition of a drop of triethylamine. The reaction solution was stirred until it became clear. To the reaction solution were successively added 1-hydroxy-cyclopentanecarboxylic acid **4a** (2.2 mg, 16.9 μmol, prepared using the method disclosed in Patent Application "WO2013106717") and 4-(4,6-dimethoxy-1,3,5-triazin-2-yl)-4-methylmorpholinium chloride (4.7 mg, 16.9 μmol). After the addition was completed, the reaction solution was stirred at 0-5 °C for 1 h, quenched with 5 mL of water, and extracted with ethyl acetate (10 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **4** (2.5 mg, 80.9% yield).

MS m/z (ESI): 548.0.[M+1].

$^1$H NMR (400 MHz, $CDCl_3$): δ 7.73-7.62 (m, 2H), 5.75-5.62 (m, 1H), 5.46-5.32 (m, 2H), 5.26-5.10 (m, 1H), 3.30-3.10 (m, 1H), 2.43 (s, 3H), 2.28-2.20 (m, 2H), 2.08-1.84 (m, 8H), 1.69-1.58 (m, 2H), 1.04-1.00 (m, 2H), 0.89 (t, 3H).

Example 1-5

*N*-((1*S*,9*S*)-9-ethyl-5-fluoro-9-hydroxy-4-methyl-10,13-dioxo-2,3,9,10,13,15-hexa hydro-1*H*,12*H*-benzo[*de*]pyrano[3',4':6,7]indolizino[1,2-*b*]quinolin-1-yl)-1-(hydroxyme thyl)cyclopropane-1-carboxamide **5**

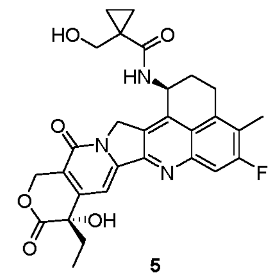

**5**

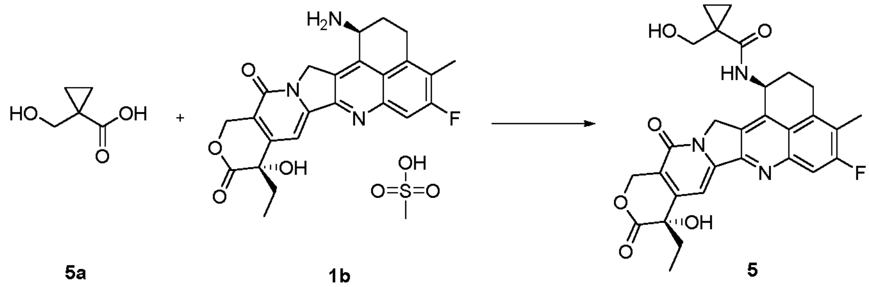

**5a** **1b** **5**

To **1b** (2.0 mg, 3.76 μmol) was added 1 mL of N,N-dimethylformamide. The mixture was cooled to 0-5 °C in an ice-water bath, followed by addition of a drop of triethylamine. The reaction solution was stirred until it became clear. To the reaction solution were successively added 1-(hydroxymethyl)-cyclopentanecarboxylic acid **5a** (0.87 mg, 7.5 μmol, prepared using the method disclosed in Patent Application "WO201396771") and 4-(4,6-dimethoxy-1,3,5-triazin-2-yl)-4-methylmorpholinium chloride (2 mg, 7.24 μmol). After the addition was completed, the reaction solution was stirred at 0-5 °C for 2 h, quenched with 5 mL of water, and extracted with ethyl acetate (8 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **5** (1.0 mg, 50% yield).

MS m/z (ESI): 533.9.[M+1].

$^1$H NMR (400 MHz, $CDCl_3$): δ 8.07 (s, 1H), 7.23-7.18 (m, 2H), 6.71-6.64 (m, 1H), 6.55-6.51 (m, 1H), 5.36-5.27 (m, 2H), 4.67-4.61 (m, 2H), 3.53-3.48 (m, 1H), 3.30-3.22 (m, 2H), 3.18-3.13 (m, 1H), 2.71-2.61 (m, 2H), 2.35-2.28 (m, 1H), 2.04-1.91 (m, 4H), 1.53-1.40 (m, 3H), 0.91-0.75 (m, 4H).

Example 1-6

*N*-((1*S*,9*S*)-9-ethyl-5-fluoro-9-hydroxy-4-methyl-10,13-dioxo-2,3,9,10,13,15-hexahydro -1*H*,12*H*-benzo[*de*]pyrano[3',4':6,7]indolizino[1,2-*b*]quinolin-1-yl)-1-(hydroxymethyl)c yclobutane-1-carboxamide **6**

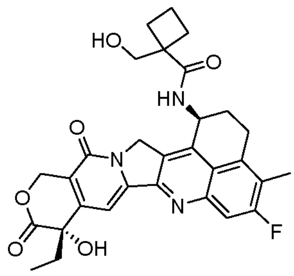

6

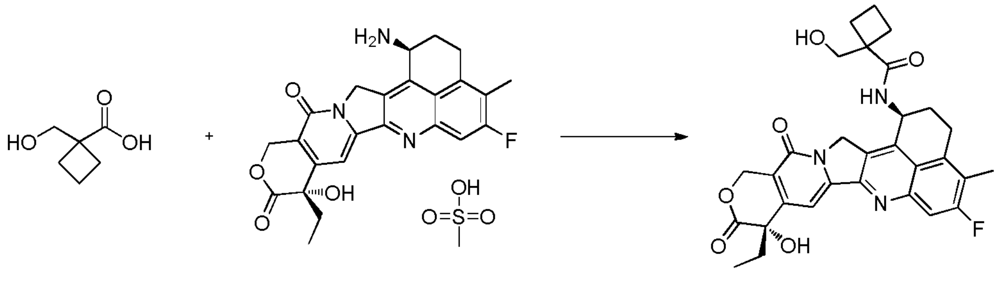

6a 1b 6

To **1b** (3.0 mg, 5.64 μmol) was added 1 mL of *N,N*-dimethylformamide. The mixture was cooled to 0-5 °C in an ice-water bath, followed by addition of a drop of triethylamine. The reaction solution was stirred until it became clear. To the reaction solution were successively added 1-(hydroxymethyl)cyclobutane-1-carboxylic acid **6a** (2.2 mg, 16.9 μmol; prepared using the method disclosed in the document "Journal of the American Chemical Society, 2014, vol.136, #22, p.8138-8142") and 4-(4,6-dimethoxy-1,3,5-triazin-2-yl)-4-methylmorpholinium chloride (4.7 mg, 16.9 μmol). After the addition was completed, the reaction solution was stirred at 0-5 °C for 1 h, quenched with 5 mL of water, and extracted with ethyl acetate (10 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **6** (2.1 mg, 67.9% yield).

MS m/z (ESI): 548.0.[M+1].

$^1$H NMR (400 MHz, DMSO-$d_6$): δ 7.85-7.62 (m, 1H), 6.88 (br, 1H), 5.87-5.48 (m, 2H), 5.47-5.33 (m, 1H), 5.31-5.06 (m, 1H), 4.25-3.91 (m, 2H), 3.25 (br, 1H), 2.60-2.32 (m, 3H), 2.23 (t, 1H), 2.15-1.95 (m, 3H), 1.70-1.56 (m, 2H), 1.41-1.17 (m, 9H), 1.03 (s, 1H), 0.95-0.80 (m, 2H).

Example 1-7

*N*-((1*S*,9*S*)-9-ethyl-5-fluoro-9-hydroxy-4-methyl-10,13-dioxo-2,3,9,10,13,15-hexahydro -1*H*,12*H*-benzo[*de*]pyrano[3',4':6,7]indolizino[1,2-*b*]quinolin-1-yl)-1-hydroxycyclobuta ne-1-carboxamide **7**

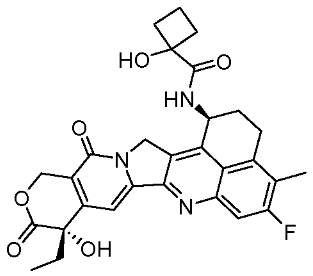

7

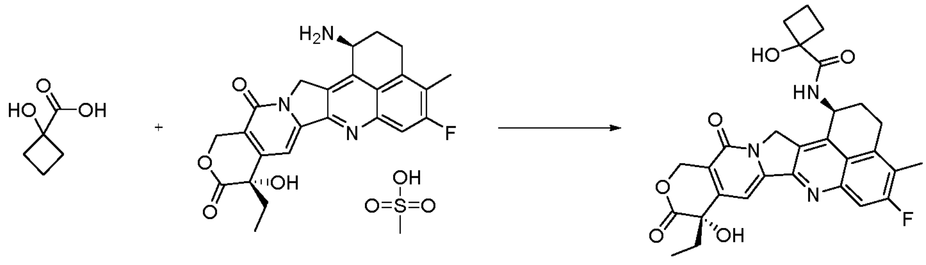

7a 1b 7

To **1b** (3.0 mg, 5.64 μmol) were added 2 mL of ethanol and 0.4 mL of *N,N*-dimethylformamide. The mixture was cooled to 0-5 °C in an ice-water bath, followed by dropwise addition of 0.3 mL of N-methylmorpholine. The reaction solution was stirred until it became clear. To the reaction solution were successively added 1-hydroxycyclobutanecarboxylic acid **7a** (2.0 mg, 17.22 μmol, supplier PharmaBlock), 1-hydroxybenzotriazole (2.3 mg, 17.0 μmol) and 1-(3-dimethylaminopropyl)-3-ethylcarbodiimide hydrochloride (3.2 mg, 16.7 μmol). After the addition was completed, the reaction solution was stirred at 0-5 °C for 10 min. The ice-water bath was removed, and the reaction solution was stirred at room temperature for 2 h, and concentrated under reduced pressure. The resulting residue was purified by thin layer chromatography with developing solvent system B to give the title product 7 (2.5 mg, 83.1% yield).

MS m/z (ESI): 534.0.[M+1].

$^1$H NMR (400 MHz, DMSO-*$d_6$*): δ 8.28 (d, 1H), 7.75 (d, 1H), 7.29 (s, 1H), 6.51 (s, 1H), 6.12 (s, 1H), 5.59-5.51 (m, 1H), 5.41 (s, 2H), 5.20-5.01 (m, 2H), 3.27-3.17 (m, 1H), 3.15-3.05 (m, 1H), 2.71-2.63 (m, 1H), 2.37 (s, 3H), 2.12-2.05 (m, 1H), 2.03-1.94 (m, 2H), 1.92-1.78 (m, 4H), 1.50-1.42 (m, 1H), 0.90-0.83 (m, 4H).

Example 1-8

1-(((*S*)-7-benzyl-20-(2,5-dioxo-2,5-dihydro-1*H*-pyrrol-1-yl)-3,6,9,12,15-pentaoxo-2,5,8, 11,14-pentaazaeicosyl)oxy)-*N*-((1*S*,9*S*)-9-ethyl-5-fluoro-9-hydroxy-4-methyl-10,13-dio xo-2,3,9,10,13,15-hexahydro-1*H*,12*H*-benzo[*de*]pyrano[3',4':6,7]indolizino[1,2-*b*]quinol in-1-yl)cyclopropane-1-carboxamide **8**

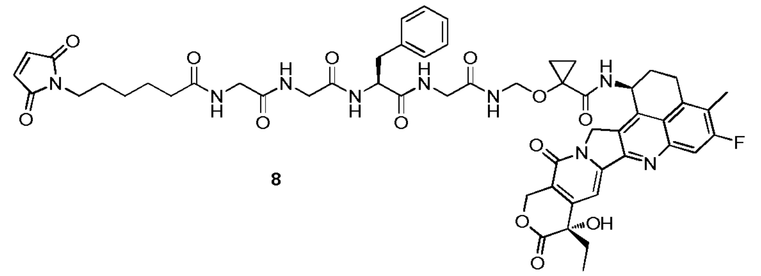

**8**

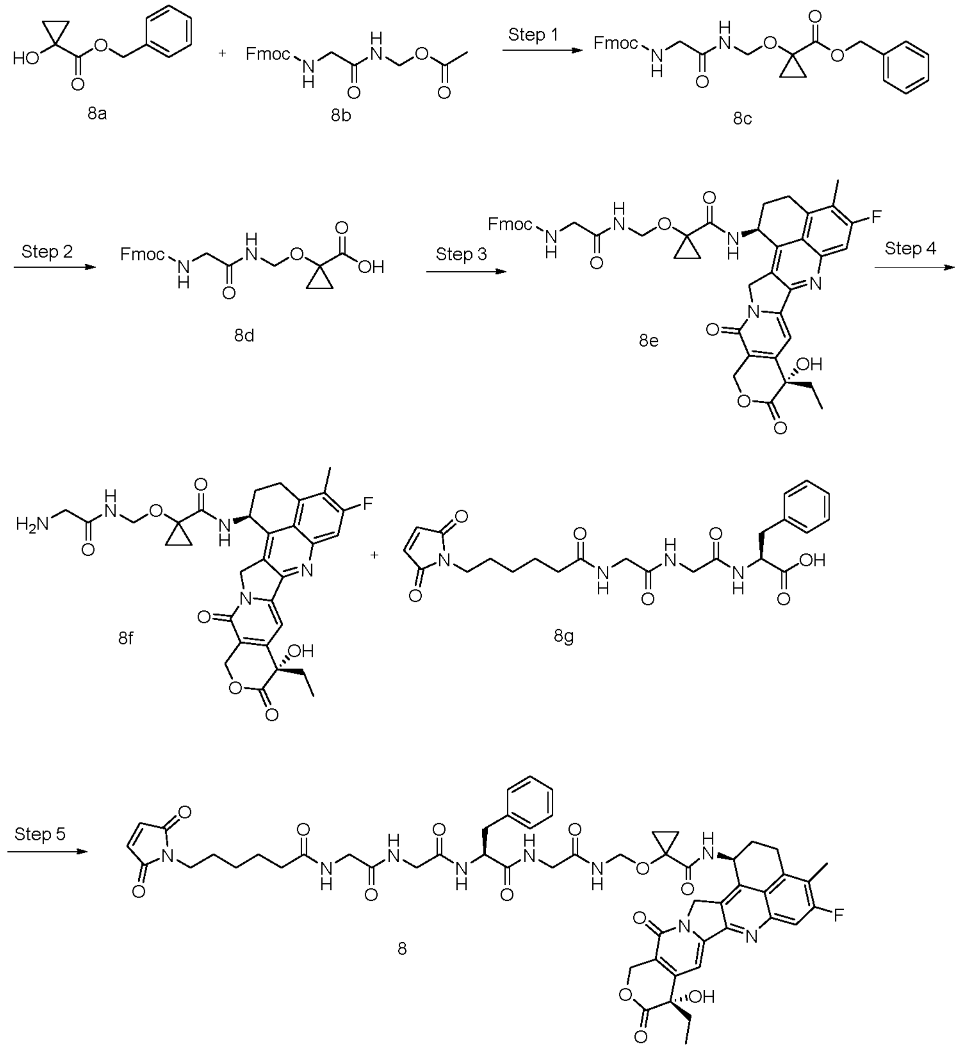


8a 8b 8c 8d 8e 8f 8g 8

Step 1

Benzyl 1-((2-((((9H-fluoren-9-yl)methoxy)carbonyl)amino)acetamido)methoxy)cyclopropane-1 -carboxylate **8e**

To a reaction flask were added benzyl 1-hydroxycyclopropane-1-carboxylate **8a** (104 mg, 0.54 mmol; prepared using the method disclosed in Patent Application "US2005/20645 ") and 2-((((9*H*-fluoren-9-yl)methoxy)carbonyl)amino)acetamido)methyl acetate **8b** (100 mg, 0.27 mmol; prepared using the method disclosed in Patent Application "CN105829346A"), followed by addition of 5 mL of tetrahydrofuran. The reaction solution was purged with argon three times, cooled to 0-5 °C in an ice-water bath, and added with potassium tert-butoxide (61 mg, 0.54 mmol). The ice bath was removed, and the reaction solution was heated to room temperature and stirred for 10 min, added with 20 mL of ice water, and extracted with ethyl acetate (5 mL × 2) and chloroform (5 mL × 5). The organic phases were combined and concentrated. The resulting residue was dissolved in 3 mL of 1,4-dioxane, and 0.6 mL of water, sodium bicarbonate (27 mg, 0.32 mmol) and 9-fluorenylmethyl chloroformate (70 mg, 0.27 mmol) were added. The reaction solution was stirred at room temperature for 1 h, added with 20 mL of water, and extracted with ethyl acetate (8 mL × 3). The organic

phase was washed with saturated sodium chloride solution (20 mL), dried over anhydrous sodium sulfate, and filtered. The filtrate was concentrated under reduced pressure, and the resulting residue was purified by silica gel column chromatography with developing solvent system B to give the title product 8c (100 mg, 73.6% yield).

MS m/z (ESI): 501.0.[M+1].

Step 2

1-((2-((((9*H*-fluoren-9-yl)methoxy)carbonyl)amino)acetamido)methoxy)cyclopropane-1 -carboxylic acid **8d**

**8c** (50 mg, 0.10 mmol) was dissolved in 3 mL of a solvent mixture of tetrahydrofuran and ethyl acetate (V:V = 2:1), and palladium on carbon (25 mg, 10% loading) was added. The reaction solution was purged with hydrogen three times and stirred at room temperature for 1 h. The reaction solution was filtered through celite, and the filter cake was rinsed with tetrahydrofuran. The filtrate was concentrated to give the title product **8d** (41 mg, 100% yield).

MS m/z (ESI): 411.0.[M+1].

Step 3

(9*H*-fluoren-9-yl)methyl(2-(((1-(((1*S*,9*S*)-9-ethyl-5-fluoro-9-hydroxy-4-methyl-10,13-di oxo-2,3,9,10,13,15-hexahydro-1*H*,12*H*-benzo[*de*]pyrano[3',4':6,7]indolizino[1,2-*b*]quin olin-1-yl)aminocarbonyl)cyclopropoxy)methyl)amino)-2-oxoethyl)carbamate **8e**

To a reaction flask was added **1b** (7 mg, 0.013 mmol), followed by addition of 1 mL of N,N-dimethylformamide. The reaction solution was purged with argon three times, and cooled to 0-5 °C in an ice-water bath, followed by addition of a drop of triethylamine, a solution of **8d** (7 mg, 0.017 mmol) in 0.5 mL of *N*,*N*-dimethylformamide, and 4-(4,6-dimethoxy-1,3,5-triazin-2-yl)-4-methylmorpholinium chloride (7 mg, 0.026 mmol). The reaction solution was stirred in an ice bath for 35 min, added with 10 mL of water, and extracted with ethyl acetate (5 mL × 3). The organic phase was washed with saturated sodium chloride solution (10 mL), dried over anhydrous sodium sulfate, and filtered. The filtrate was concentrated under reduced pressure, and the resulting residue was purified by thin layer chromatography with developing solvent system B to give the title product **8e** (8.5 mg, 78.0% yield).

MS m/z (ESI): 828.0.[M+1].

Step 4

1-((2-Aminoacetylamino)methoxy)-*N*-((1*S*,9*S*)-9-ethyl-5-fluoro-9-hydroxy-4-methyl-10 ,13-dioxo-2,3,9,10,13,15-hexahydro-1*H*,12*H*-benzo[*de*]pyrano [3',4':6,7]indolizino[1,2-*b* ]quinolin-1-yl)cyclopropane-1-carboxamide **8f**

**8e** (4 mg, 4.84 μmol) was dissolved in 0.2 mL of dichloromethane, and 0.1 mL of diethylamine was added. The reaction solution was stirred at room temperature for 2 h, and concentrated under reduced pressure. 2 mL of toluene was added, followed by concentration under reduced pressure; the procedures were repeated twice. The residue was slurried with 3 mL of *n*-hexane, and the upper n-hexane layer was removed; the procedures were repeated three times. The slurry was concentrated under reduced pressure to give the crude title product **8f** (2.9 mg), which was directly used in the next step without purification.

MS m/z (ESI): 606.0.[M+1].

Step 5

1-(((*S*)-7-benzyl-20-(2,5-dioxo-2,5-dihydro-1*H*-pyrrol-1-yl)-3,6,9,12,15-pentaoxo-2,5,8, 11,14-pentaazaeicosyl)oxy)-*N*-((1*S*,9*S*)-9-ethyl-5-fluoro-9-hydroxy-4-methyl-10,13-dio xo-2,3,9,10,13,15-hexahydro-1*H*,12*H*-benzo[*de*]pyrano[3',4':6,7]indolizino[1,2-*b*]quinol in-1-yl)cyclopropane-1-carboxamide **8**

Crude **8f** (2.9 mg, 4.84 μmol) was dissolved in 0.5 mL of N,N-dimethylformamide. The reaction solution was purged with argon three times, cooled to 0-5 °C in an ice-water bath, and added with a solution of (*S*)-2-2-(-2-(6-(2,5-dioxo-1*H*-pyrrol-1-yl)hexanamido)acetylamino)-3-phenylpropionic acid **8g** (2.7 mg, 5.80 μmol, prepared using the method disclosed in Patent Application "EP2907824") in 0.3 mL of *N*,*N*-dimethylformamide, followed by addition of 4-(4,6-dimethoxy-1,3,5-triazin-2-yl)-4-methylmorpholinium chloride (2.7 mg, 9.67 μmol). The reaction solution was stirred in an ice bath for 30 min, and the ice bath was removed. The reaction solution was heated to room temperature and stirred for 15 min, and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient

elution, flow rate: 18 mL/min). The corresponding fractions were collected and concentrated under reduced pressure to give the title product **8** (2 mg, 39.0% yield).

MS m/z (ESI): 1060.0.[M+1].

$^1$H NMR (400 MHz, DMSO-$d_6$): δ 9.01 (d, 1H), 8.77 (t, 1H), 8.21 (t, 1H), 8.08-7.92 (m, 2H), 7.73 (d, 1H), 7.28 (s, 1H), 7.24-7.07 (m, 4H), 6.98 (s, 1H), 6.50 (s, 1H), 5.61 (q, 1H), 5.40 (s, 2H), 5.32 (t, 1H), 5.12 (q, 2H), 4.62 (t, 1H), 4.52 (t, 1H), 4.40-4.32 (m, 1H), 3.73-3.47 (m, 8H), 3.16-3.04 (m, 2H), 2.89 (dd, 1H), 2.69-2.55 (m, 2H), 2.37-2.23 (m, 4H), 2.12-1.93 (m, 4H), 1.90-1.74 (m, 2H), 1.52-1.38 (m, 4H), 1.33-1.11 (m, 5H), 0.91-0.81 (m, 4H).

Example 1-9

*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadec-16-yl) -6-(2,5-dioxo-2,5-dihydro-1*H*-pyrrol-1-yl)hexanamide **9-A**

*N*-((2*S*, 10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadec-16-yl) -6-(2,5-dioxo-2,5-dihydro-1*H*-pyrrol-1-yl)hexanamide **9-B**

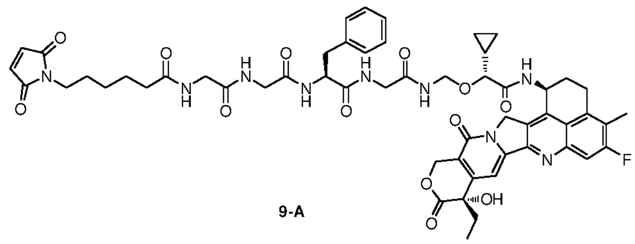

**9-A**

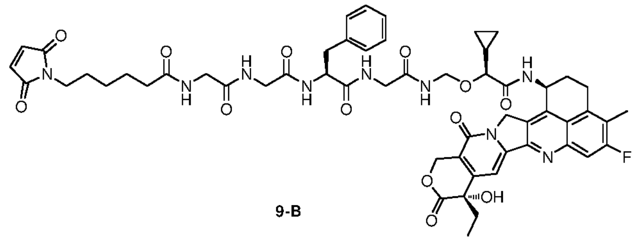

**9-B**

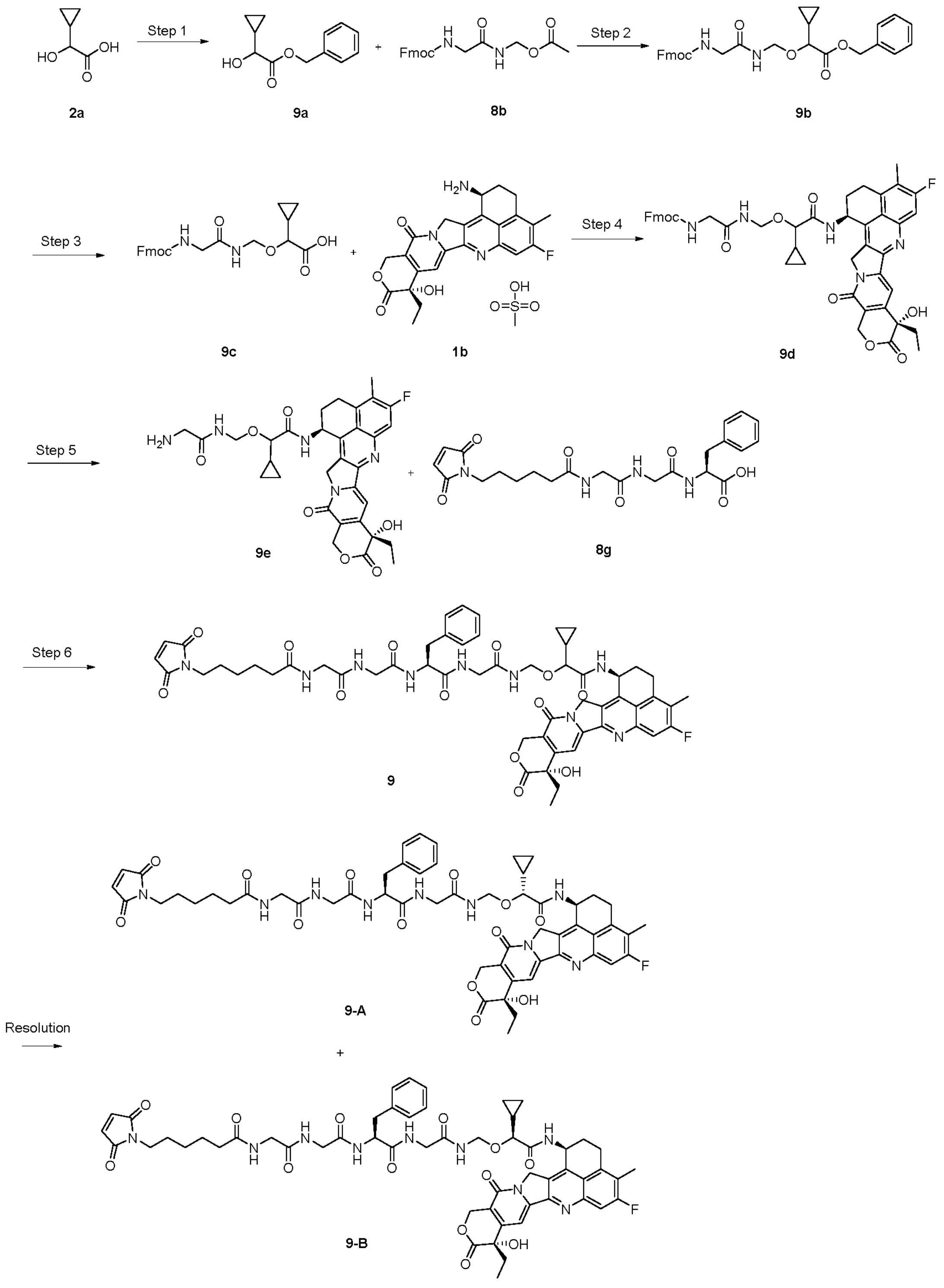


2a, 9a, 8b, 9b, 9c, 1b, 9d, 9e, 8g, 9, 9-A, 9-B

Step 1

Benzyl 2-cyclopropyl-2-hydroxyacetate **9a**

**2a** (1.3 g, 11.2 mmol; prepared using the method disclosed in Patent Application "WO2013/106717") was dissolved in 50 mL of acetonitrile, and potassium carbonate (6.18 g, 44.8 mmol), benzyl bromide (1.33 mL, 11.2 mmol) and tetrabutylammonium iodide (413 mg, 1.1 mmol) were successively added. The reaction solution was stirred at room temperature for 48 h and filtered through celite, and the filter cake was rinsed with ethyl acetate (10 mL). The filtrates were combined and concentrated under reduced pressure, and the resulting residue was purified by silica gel column

chromatography with developing solvent system C to give the title product **9a** (2 g, 86.9% yield).

Step 2

Benzyl

10-cyclopropyl-1-(9*H*-fluoren-9-yl)-3,6-dioxo-2,9-dioxa-4,7-diazaundecan-11-oate **9b** To a reaction flask were added **9a** (120.9 mg, 0.586 mmol) and **8b** (180 mg, 0.489 mmol), followed by addition of 4 mL of tetrahydrofuran. The reaction solution was purged with argon three times, cooled to 0-5 °C in an ice-water bath, and added with potassium tert-butoxide (109 mg, 0.98 mmol). The ice bath was removed, the reaction solution was heated to room temperature and stirred for 40 min, added with 10 mL of ice water, and extracted with ethyl acetate (20 mL × 2) and chloroform (10 mL × 5). The organic phases were combined and concentrated. The resulting residue was dissolved in 4 mL of dioxane, and 2 mL of water, sodium bicarbonate (49.2 mg, 0.586 mmol) and 9-fluorenylmethyl chloroformate (126 mg, 0.49 mmol) were added. The reaction solution was stirred at room temperature for 2 h, added with 20 mL of water, and extracted with ethyl acetate (10 mL × 3). The organic phase was washed with saturated sodium chloride solution (20 mL), dried over anhydrous sodium sulfate, and filtered. The filtrate was concentrated under reduced pressure. The resulting residue was purified by silica gel column chromatography with developing solvent system C to give the title product **9b** (48 mg, 19% yield).

MS m/z (ESI): 515.0.[M+1].

Step 3

10-cyclopropyl-1-(9*H*-fluoren-9-yl)-3,6-dioxo-2,9-dioxa-4,7-diazaundecan-11-oic acid **9c**

**9b** (20 mg, 0.038 mmol) was dissolved in 4.5 mL of a solvent mixture of tetrahydrofuran and ethyl acetate (V:V = 2:1), and palladium on carbon (12 mg, 10% loading, dry) was added. The reaction solution was purged with hydrogen three times and stirred at room temperature for 1 h. The reaction solution was filtered through celite, and the filter cake was rinsed with ethyl acetate. The filtrate was concentrated to give the crude title product **9c** (13 mg), which was directly used in the next step without purification.

MS m/z (ESI): 424.9 [M+1].

Step 4

(9*H*-fluoren-9-yl)methyl(2-(((1-cyclopropyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indoli zino[1,2-*b*]quinolin-1-yl)amino)-2-oxoethoxy)methyl)amino)-2-oxoethyl)carbamate **9d** To a reaction flask was added **1b** (10 mg, 18.8 μmol), followed by addition of 1 mL of *N,N*-dimethylformamide. The reaction solution was purged with argon three times, and cooled to 0-5 °C in an ice-water bath, followed by addition of a drop of triethylamine, crude product **9c** (13 mg, 30.6 μmol), and 4-(4,6-dimethoxy-1,3,5-triazin-2-yl)-4-methylmorpholinium chloride (16.9 mg, 61.2 μmol). The reaction solution was stirred in an ice bath for 40 min, added with 10 mL of water, and extracted with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **9d** (19 mg, 73.6% yield).

MS m/z (ESI): 842.1.[M+1].

Step 5

2-((2-Aminoacetamido)methoxy)-2-cyclopropyl-*N*-((1*S*,9*S*)-9-ethyl-5-fluoro-9-hydroxy -4-methyl-10,13-dioxo-2,3,9,10,13,15-hexahydro-1*H*,12*H*-benzo[*de*]pyrano[3',4':6,7]in dolizino[1,2-*b*]quinolin-1-yl)acetamide **9e**

**9d** (19 mg, 22.6 μmol) was dissolved in 2 mL of dichloromethane, and 1 mL of diethylamine was added. The reaction solution was stirred at room temperature for 2 h, and concentrated under reduced pressure. 1 mL of toluene was added, followed by concentration under reduced pressure; the procedures were repeated twice. The residue was slurried with 3 mL of n-hexane and let stand. Then, the supernatant was removed, and the solid was kept. The solid residue was concentrated under reduced pressure and dried using an oil pump to give the crude title product **9e** (17 mg), which was directly used in the next step without purification.

MS m/z (ESI): 638.0.[M+18].

Step 6

*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadec-16-yl) -6-(2,5-dioxo-2,5-dihydro-1*H*-pyrrol-1-yl)hexanamide **9-A**

*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadec-16-yl) -6-(2,5-dioxo-2,5-dihydro-1*H*-pyrrol-1-yl)hexanamide **9-B**

Crude **9e** (13.9 mg, 22.4 μmol) was dissolved in 0.6 mL of *N,N*-dimethylformamide. The reaction solution was purged with argon three times, cooled to 0-5 °C in an ice-water bath, and added with a solution of **8g** (21.2 mg, 44.8 μmol) in 0.3 mL of N,N-dimethylformamide, followed by addition of 4-(4,6-dimethoxy-1,3,5-triazin-2-yl)-4-methylmorpholinium chloride (18.5 mg, 67.3 μmol). The reaction solution was stirred in an ice bath for 10 min, and the ice bath was removed. The reaction solution was heated to room temperature and stirred for 1 h to give compound **9.** The reaction solution was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product (9-A: 2.4 mg, 9-B: 1.7 mg).

MS m/z (ESI): 1074.4.[M+1].

Single-configuration compound 9-A (shorter retention time)

UPLC analysis: retention time: 1.14 min; purity: 85% (chromatography column: ACQUITY UPLC BEHC18 1.7 μm 2.1 × 50 mm; mobile phase: A-water (5 mmol of $NH_4OAc$), B-acetonitrile).

$^1H$ NMR (400 MHz, DMSO-$d_6$): δ 8.60 (t, 1H), 8.51-8.49 (d, 1H), 8.32-8.24 (m, 1H), 8.13-8.02 (m, 2H), 8.02-7.96 (m, 1H), 7.82-7.75 (m, 1H), 7.31 (s, 1H), 7.26-7.15 (m, 4H), 6.99 (s, 1H), 6.55-6.48 (m, 1H), 5.65-5.54 (m, 1H), 5.41 (s, 2H), 5.35-5.15 (m, 3H), 4.74-4.62 (m, 1H), 4.54-4.40 (m, 2H), 3.76-3.64 (m, 4H), 3.62-3.48 (m, 2H), 3.20-3.07 (m, 2H), 3.04-2.94 (m, 1H), 2.80-2.62 (m, 1H), 2.45-2.30 (m, 3H), 2.25-2.15 (m, 2H), 2.15-2.04 (m, 2H), 1.93-1.78 (m, 2H), 1.52-1.39 (m, 3H), 1.34-1.12 (m, 5H), 0.87 (t, 3H), 0.64-0.38 (m, 4H).

Single-configuration compound 9-B (longer retention time)

UPLC analysis: retention time: 1.16 min; purity: 89% (chromatography column: ACQUITY UPLC BEHC18 1.7 μm 2.1 × 50 mm; mobile phase: A-water (5 mmol of $NH_4OAc$), B-acetonitrile).

$^1H$ NMR (400 MHz, DMSO-$d_6$): δ 8.68-8.60 (m, 1H), 8.58-8.50 (m, 1H), 8.32-8.24 (m, 1H), 8.13-8.02 (m, 2H), 8.02-7.94 (m, 1H), 7.82-7.75 (m, 1H), 7.31 (s, 1H), 7.26-7.13 (m, 3H), 6.99 (s, 1H), 6.55-6.48 (m, 1H), 5.60-5.50 (m, 1H), 5.41 (s, 2H), 5.35-5.15 (m, 2H), 4.78-4.68 (m, 1H), 4.60-4.40 (m, 2H), 3.76-3.58 (m, 4H), 3.58-3.48 (m, 1H), 3.20-3.10 (m, 2H), 3.08-2.97 (m, 2H), 2.80-2.72 (m, 2H), 2.45-2.30 (m, 3H), 2.25-2.13 (m, 2H), 2.13-2.04 (m, 2H), 2.03-1.94 (m, 2H), 1.91-1.78 (m, 2H), 1.52-1.39 (m, 3H), 1.34-1.12 (m, 4H), 0.91-0.79 (m, 3H), 0.53-0.34 (m, 4H).

Example 1-10

*N*-((2S,10*S*)-10-benzyl-2-(((1*S*,9*S*)-9-ethyl-5-fluoro-9-hydroxy-4-methyl-10,13-dioxo-2, 3,9,10,13,15-hexahydro-1*H*,12*H*-benzo[*de*]pyrano[3',4':6,7]indolizino[1,2-*b*]quinolin-1-yl)aminocarbonyl)-1,1,1-trifluoro-6,9,12,15-tetraoxo-3-oxa-5,8,11,14-tetraazahexadec-1 6-yl)-6-(2,5-dioxo-2,5-dihydro-1*H*-pyrrol-1-yl)hexanamide **10-A**

*N*-((2*R*,10*S*)-10-benzyl-2-(((1*S*,9*S*)-9-ethyl-5-fluoro-9-hydroxy-4-methyl-10,13-dioxo-2, 3,9,10,13,15-hexahydro-1*H*,12*H*-benzo[*de*]pyrano[3',4':6,7]indolizino[1,2-b]quinolin-1-yl)aminocarbonyl)-1,1,1-trifluoro-6,9,12,15-tetraoxo-3-oxa-5,8,11,14-tetraazahexadec-1 6-yl)-6-(2,5-dioxo-2,5-dihydro-1*H*-pyrrol-1-yl)hexanamide **10-B**

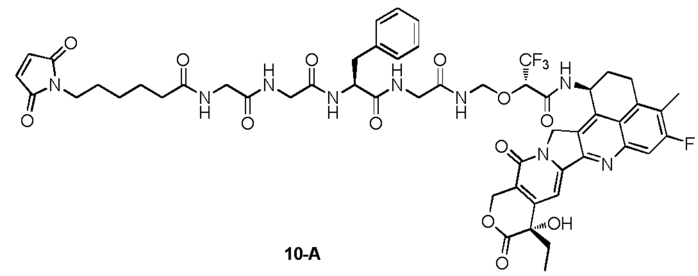

10-A

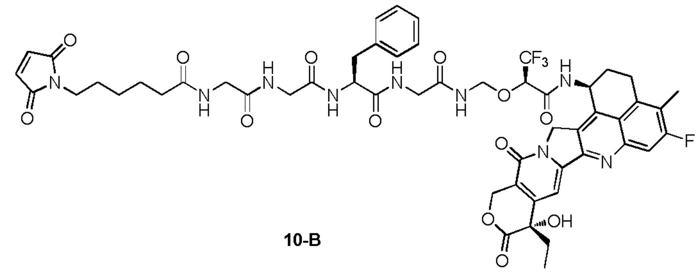

10-B

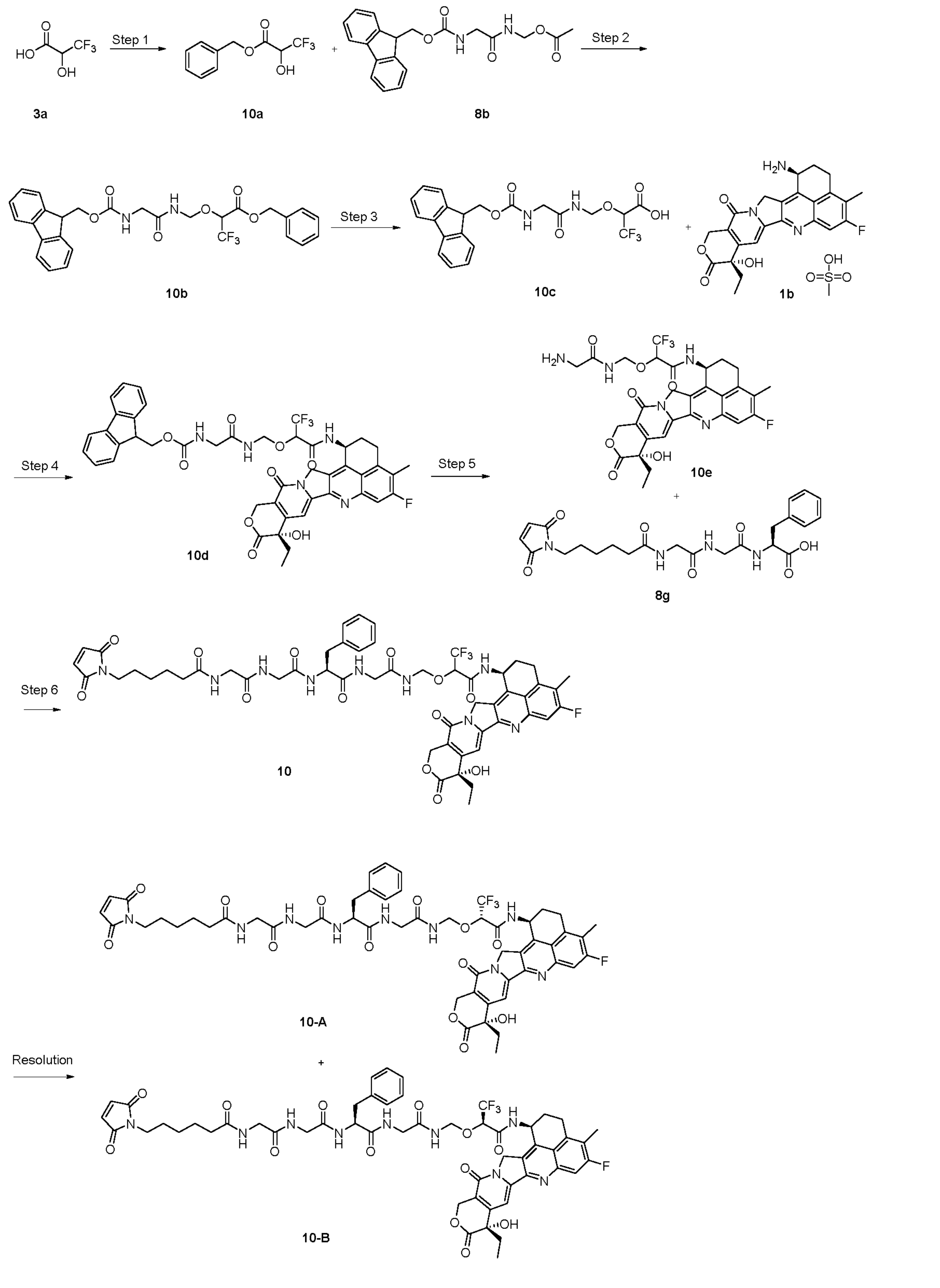


3a 10a 8b

10b 10c 1b

10d 10e 8g

10

10-A

10-B

Step 1

Benzyl 3,3,3-trifluoro-2-hydroxypropionate **10a**

**3a** (1.80 g, 12.5 mmol) was dissolved in 100 mL of acetonitrile, and potassium carbonate (5.17g, 37.5 mmol), benzyl bromide (4.48 mL, 37.5 mmol) and tetrabutylammonium iodide (231 mg, 0.63 mmol) were successively added. The reaction solution was heated to 60 °C and stirred for 5 h, and cooled to room temperature and filtered. The filtrate

was concentrated under reduced pressure, and the resulting residue was purified by silica gel column chromatography with developing solvent system C to give the title product **10a** (980 mg, 33.5% yield).
$^1$H NMR (400 MHz, $CDCl_3$): δ 7.43-7.36 (m, 5H), 5.34 (s, 2H), 4.53 (s, 1H), 3.44 (s, 1H).

Step 2

Benzyl 1-(9*H*-fluoren-9-yl)-3,6-dioxo-10-(trifluoromethyl)-2,9-dioxa-4,7-diazaundecan-11-oate **10b**

To a reaction flask were added **8b** (63 mg, 0.17 mmol) and **10a** (80 mg, 0.34 mmol), followed by addition of 3 mL of tetrahydrofuran. The reaction solution was purged with argon three times, cooled to 0-5 °C in an ice-water bath, and added with potassium *tert*-butoxide (38 mg, 0.34 mmol). The ice bath was removed, and the reaction solution was heated to room temperature and stirred for 20 min, added with 10 mL of ice water, and extracted with ethyl acetate (20 mL × 2) and chloroform (10 mL × 5). The organic phases were combined and concentrated, and the resulting residue was dissolved in 2 mL of dioxane, and added with 0.4 mL of water, followed by addition of sodium bicarbonate (19 mg, 0.23 mmol) and 9-fluorenylmethyl chloroformate (49 mg, 0.19 mmol). The reaction solution was stirred at room temperature for 1 h, added with 20 mL of water, and extracted with ethyl acetate (10 mL × 3). The organic phase was washed with saturated sodium chloride solution (20 mL), dried over anhydrous sodium sulfate, and filtered. The filtrate was concentrated under reduced pressure, and the resulting residue was purified by silica gel column chromatography with developing solvent system C to give the title product **10b** (51 mg, 55.3% yield).
MS m/z (ESI): 559.9 [M+18].

Step 3

1-(9*H*-fluoren-9-yl)-3,6-dioxo-10-(trifluoromethyl)-2,9-dioxa-4,7-diazaundecan-1 1-oic acid **10c**

**10b** (15 mg, 0.28 mmol) was dissolved in 3 mL of a solvent mixture of tetrahydrofuran and ethyl acetate (V:V = 2:1), and palladium on carbon (15 mg, 10% loading) was added. The reaction solution was purged with hydrogen three times and stirred at room temperature for 1 h. The reaction solution was filtered through celite, and the filter cake was rinsed with tetrahydrofuran. The filtrate was concentrated to give the title product **10c** (13 mg).
MS m/z (ESI): 452.9 [M+1].

Step 4

(9*H*-fluoren-9-yl)methyl(2-((((3-(((1*S*,9*S*)-9-ethyl-5-fluoro-9-hydroxy-4-methyl-10,13-d ioxo-2,3,9,10,13,15-hexahydro-1*H*,12*H*-benzo[*de*]pyrano[3',4':6,7]indolizino[1,2-*b*]quin olin-1-yl)amino)-1,1,1-trifluoro-3-oxoprop-2-yl)oxy)methyl)amino)-2-oxoethyl)carbam ate **10d**

To a reaction flask was added **1b** (10 mg, 18.8 μmol), followed by addition of 1 mL of N,N-dimethylformamide. The reaction solution was purged with argon three times, and cooled to 0-5 °C in an ice-water bath, followed by addition of a drop of triethylamine, a solution of 10c (13 mg, 28.7 μmol) in 0.5 mL of N,N-dimethylformamide, and 4-(4,6-dimethoxy-1,3,5-triazin-2-yl)-4-methylmorpholinium chloride (11 mg, 39.7 μmol). The reaction solution was stirred in an ice bath for 30 min, added with 10 mL of water, and extracted with ethyl acetate (10 mL × 3). The organic phases were combined, washed with saturated sodium chloride solution (10 mL × 2), dried over anhydrous sodium sulfate, and filtered. The filtrate was concentrated under reduced pressure, and the resulting residue was purified by thin layer chromatography with developing solvent system B to give the title product **10d** (16 mg, 97.8% yield).
MS m/z (ESI): 870.0 [M+1].

Step 5

2-((2-Aminoacetylamino)methoxy)-*N*-((1*S*,9*S*)-9-ethyl-5-fluoro-9-hydroxy-4-methyl-10 ,13-dioxo-2,3,9,10,13,15-hexahydro-1*H*,12*H*-benzo[*de*]pyrano[3',4':6,7]indolizino[1,2-*b* ]quinolin-1-yl)-3,3,3-trifluoropropionamide **10e**

**10d** (16 mg, 18.4 μmol) was dissolved in 0.6 mL of dichloromethane, and 0.3 mL of diethylamine was added. The reaction solution was stirred at room temperature for 2 h, and concentrated under reduced pressure. 2 mL of toluene was added, followed by concentration under reduced pressure; the procedures were repeated twice. The residue was slurried with 3 mL of n-hexane and let stand. Then, the supernatant was removed, and the solid was kept; the procedures were repeated three times. The solid residue was concentrated under reduced pressure and dried using an oil pump to give the crude title product **10e** (12 mg), which was directly used in the next step without purification.

MS m/z (ESI): 647.9 [M+1].

Step 6

*N*-((2*S*,10*S*)-10-benzyl-2-(((1*S*,9*S*)-9-ethyl-5-fluoro-9-hydroxy-4-methyl-10,13-dioxo-2, 3,9,10,13,15-hexahydro-1*H*,12*H*-benzo[*de*]pyrano[3',4':6,7]indolizino[1,2-*b*]quinolin-1-yl)aminocarbonyl)-1,1,1-trifluoro-6,9,12,15-tetraoxo-3-oxa-5,8,11,14-tetraazahexadec-1 6-yl)-6-(2,5-dioxo-2,5-dihydro-1*H*-pyrrol-1-yl)hexanamide **10-A**

*N*-((2*R*,10*S*)-10-benzyl-2-(((1*S*,9*S*)-9-ethyl-5-fluoro-9-hydroxy-4-methyl-10,13-dioxo-2, 3,9,10,13,15-hexahydro-1*H*,12*H*-benzo[*de*]pyrano[3',4':6,7]indolizino[1,2-*b*]quinolin-1-yl)aminocarbonyl)-1,1,1-trifluoro-6,9,12,15-tetraoxo-3-oxa-5,8,11,14-tetraazahexadec-1 6-yl)-6-(2,5-dioxo-2,5-dihydro-1*H*-pyrrol-1-yl)hexanamide **10-B**

Crude **10e** (12 mg, 18.5 μmol) was dissolved in 1.0 mL of *N,N*-dimethylformamide. The reaction solution was purged with argon three times, cooled to 0-5 °C in an ice-water bath, and added with a solution of **8g** (14 mg, 29.6 μmol) in 0.3 mL of *N,N*-dimethylformamide, followed by addition of 4-(4,6-dimethoxy-1,3,5-triazin-2-yl)-4-methylmorpholinium chloride (15 mg, 54.2 μmol). The reaction solution was stirred in an ice bath for 30 min, and the ice bath was removed. The reaction solution was heated to room temperature and stirred for 1 h to give compound **10.** The reaction solution was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product (2.7 mg, 2.6 mg).

MS m/z (ESI): 1102.0 [M+1].

Single-configuration compound (shorter retention time)

UPLC analysis: retention time: 1.18 min; purity: 91% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.97 (d, 1H), 8.85-8.76 (m, 1H), 8.37-8.27 (m, 1H), 8.12-8.02 (m, 1H), 8.02-7.95 (m, 1H), 7.80 (d, 1H), 7.31 (s, 1H), 7.26-7.10 (m, 4H), 6.99 (s, 1H), 6.66 (br, 1H), 6.52 (s, 1H), 5.65-5.54 (m, 1H), 5.41 (s, 1H), 5.37-5.25 (m, 3H), 5.23-5.13 (m, 1H), 4.81-4.68 (m, 2H), 4.51-4.41 (m, 1H), 3.78-3.45 (m, 6H), 3.21-3.13 (m, 1H), 3.02-2.93 (m, 1H), 2.77-2.63 (m, 2H), 2.45-2.29 (m, 3H), 2.24-2.05 (m, 3H), 2.04-1.93 (m, 5H), 1.90-1.75 (m, 2H), 1.52-1.38 (m, 4H), 0.90-0.78 (m, 5H).

Single-configuration compound (longer retention time)

UPLC analysis: retention time: 1.23 min; purity: 90% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 9.05 (d, 1H), 8.97-8.88 (m, 1H), 8.35-8.27 (m, 1H), 8.11-8.03 (m, 1H), 8.02-7.95 (m, 1H), 7.80 (d, 1H), 7.34 (s, 1H), 7.29-7.13 (m, 4H), 6.99 (s, 1H), 6.66 (br, 1H), 6.54 (s, 1H), 5.64-5.55 (m, 1H), 5.43 (s, 1H), 5.36-5.20 (m, 3H), 4.92-4.85 (m, 1H), 4.82-4.72 (m, 2H), 4.52-4.42 (m, 1H), 3.77-3.48 (m, 6H), 3.21-3.14 (m, 1H), 3.03-2.95 (m, 1H), 2.79-2.65 (m, 2H), 2.47-2.28 (m, 3H), 2.25-2.05 (m, 3H), 2.05-1.94 (m, 5H), 1.91-1.76 (m, 2H), 1.52-1.37 (m, 4H), 0.92-0.77 (m, 5H).

Example 1-11

1-(((*S*)-7-benzyl-20-(2,5-dioxo-2,5-dihydro-1*H*-pyrrol-1-yl)-3,6,9,12,15-pentaoxo-2,5,8, 11,14-pentaazaeicosyl)oxy)-*N*-((1*S*,9*S*)-9-ethyl-5-fluoro-9-hydroxy-4-methyl-10,13-dio xo-2,3,9,10,13,15-hexahydro-1*H*,12*H*-benzo[*de*]pyrano[3',4':6,7]indolizino[1,2-*b*]quinol in-1-yl)cyclobutane-1-carboxamide **11**

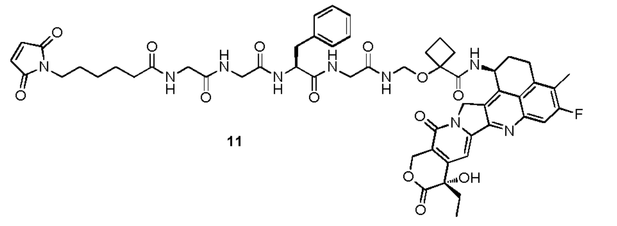

11

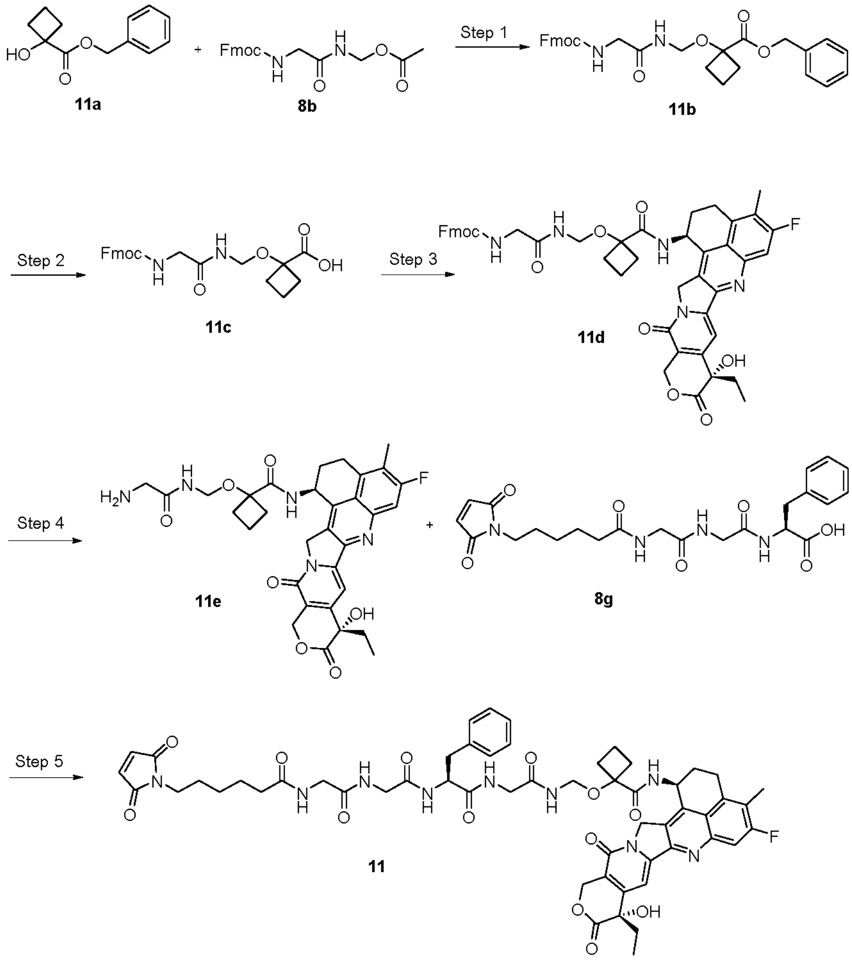


11a 8b 11b 11c 11d 11e 8g 11

Step 1

Benzyl 1-((2-((((9*H*-fluoren-9-yl)methoxy)carbonyl)amino)acetamido)methoxy)cyclobutane-1-carboxylate **11b**

To a reaction flask were added benzyl 1-hydroxycyclobutane-carboxylate **11a** (167 mg, 0.81 mmol, prepared using the method disclosed in the document "Journal of Medicinal Chemistry, 2013, vol. 56, # 13, p. 5541-5552") and **8b** (150 mg, 0.41 mmol), followed by addition of 5 mL of tetrahydrofuran. The reaction solution was purged with argon three times, cooled to 0-5 °C in an ice-water bath, and added with potassium *tert*-butoxide (92 mg, 0.82 mmol). The ice bath was removed, and the reaction solution was heated to room temperature and stirred for 10 min, added with 20 mL of ice water, and extracted with ethyl acetate (5 mL × 2) and chloroform (5 mL × 5). The organic phases were combined and concentrated, and the resulting residue was dissolved in 3 mL of dioxane, and added with 0.6 mL of water, followed by addition of sodium bicarbonate (41 mg, 0.48 mmol) and 9-fluorenylmethyl chloroformate (105 mg, 0.41 mmol). The reaction solution was stirred at room temperature for 1 h, added with 20 mL of water, and extracted with ethyl acetate (8 mL × 3). The organic phase was washed with saturated sodium chloride solution (20 mL), dried over anhydrous sodium sulfate, and filtered. The filtrate was concentrated under reduced pressure, and the resulting residue was purified by silica gel column chromatography with developing solvent system C to give the title product **11b** (37 mg, 17.6% yield).

MS m/z (ESI): 514.6 [M+1].

Step 2

1-((2-((((9*H*-fluoren-9-yl)methoxy)carbonyl)amino)acetamido)methoxy)cyclobutane-1-carboxylic acid **11c**

**11b** (37 mg, 71.9 μmol) was dissolved in 3 mL of a solvent mixture of tetrahydrofuran and ethyl acetate (V:V = 2:1), and palladium on carbon (15 mg, 10% loading) was added. The reaction solution was purged with hydrogen three times and stirred at room temperature for 2 h. The reaction solution was filtered through celite, and the filter cake was rinsed with tetrahydrofuran. The filtrate was concentrated to give the title product **11c** (35 mg, 82% yield), which was directly used in the next step.

Step 3

(9*H*-fluoren-9-yl)methyl(2-(((1-(((1*S*,9*S*)-9-ethyl-5-fluoro-9-hydroxy-4-methyl-10,13-di oxo-2,3,9,10,13,15-hexahydro-1*H*,12*H*-benzo[*de*]pyrano[3',4':6,7]indolizino[1,2-*b*]quin olin-1-yl)aminocarbonyl)cyclobutoxy)methyl)amino)-2-oxoethyl)carbamate **lid**

To a reaction flask was added **1b** (10 mg, 0.018 mmol), followed by addition of 1 mL of *N,N*-dimethylformamide. The reaction solution was purged with argon three times, and cooled to 0-5 °C in an ice-water bath, followed by addition of a drop of triethylamine, a solution of **11c** (13 mg, 0.031 mmol) in 0.5 mL of *N,N*-dimethylformamide, and 4-(4,6-dimethoxy-1,3,5-triazin-2-yl)-4-methylmorpholinium chloride (25 mg, 0.091 mmol). The reaction solution was stirred in an ice bath for 40 min, added with 8 mL of water, and extracted with ethyl acetate (5 mL × 3). The organic phase was washed with saturated sodium chloride solution (8 mL), dried over anhydrous sodium sulfate, and filtered. The filtrate was concentrated under reduced pressure, and the resulting residue was purified by thin layer chromatography with developing solvent system A to give the title product **lid** (19 mg, 73.9% yield).

MS m/z (ESI): 842.3 [M+1].

Step 4

1-((2-Aminoacetylamino)methoxy)-*N*-((1*S*,9*S*)-9-ethyl-5-fluoro-9-hydroxy-4-methyl-10 ,13-dioxo-2,3,9,10,13,15-hexahydro-1*H*,12*H*-benzo[*de*]pyrano[3',4':6,7]indolizino[1,2-*b* ]quinolin-1-yl)cyclobutane-1-carboxamide **11e**

**lid** (19 mg, 22.6 μmol) was dissolved in 2 mL of dichloromethane, and 1 mL of diethylamine was added. The reaction solution was stirred at room temperature for 1.5 h, and concentrated under reduced pressure. 1 mL of toluene was added, followed by concentration under reduced pressure; the procedures were repeated twice. The residue was slurried with 4 mL of *n*-hexane, and the upper *n*-hexane layer was removed; the procedures were repeated three times. The slurry was concentrated under reduced pressure to give the crude title product **lie** (15 mg), which was directly used in the next step without purification.

Step 5

1-(((*S*)-7-benzyl-20-(2,5-dioxo-2,5-dihydro-1*H*-pyrrol-1-yl)-3,6,9,12,15-pentaoxo-2,5,8, 11,14-pentaazaeicosyl)oxy)-*N*-((1*S*,9*S*)-9-ethyl-5-fluoro-9-hydroxy-4-methyl-10,13-dio xo-2,3,9,10,13,15-hexahydro-1*H*,12*H*-benzo[*de*]pyrano[3',4':6,7]indolizino[1,2-*b*]quinol in-1-yl)cyclobutane-1-carboxamide **11**

Crude **lie** (2 mg, 3.22 μmol) was dissolved in 0.5 mL of *N,N*-dimethylformamide. The reaction solution was purged with argon three times, cooled to 0-5 °C in an ice-water bath, and added with a solution of **8g** (1.5 mg, 3.17 μmol) in 0.3 mL of N,N-dimethylformamide, followed by addition of 4-(4,6-dimethoxy-1,3,5-triazin-2-yl)-4-methylmorpholinium chloride (2.7 mg, 9.67 μmol). The reaction solution was stirred at room temperature for 30 min, and concentrated to dryness with an oil pump to remove DMF, and the residue was dissolved in DCM and purified directly by thin layer chromatography twice (polarity of developing solvent: DCM/MeOH = 10/1) to give the title product **11** (1 mg, 28.8% yield).

MS m/z (ESI): 1073.6 [M+1].

$^1$H NMR (400 MHz, $CDCl_3$): δ 8.70-8.60 (m, 1H), 8.28-8.19 (m, 1H), 8.13-7.91 (m, 3H), 7.79-7.71 (d, 1H), 7.29 (s, 1H), 7.25-7.09 (m, 4H), 6.98 (s, 1H), 6.71-6.62 (m, 1H), 6.55-6.47 (m, 1H), 5.64-5.54 (m, 2H), 5.40 (s, 1H), 5.35-5.27 (t, 2H), 5.17-5.10 (m, 2H), 4.60-4.51 (m, 1H), 4.51-4.35 (m, 2H), 3.93-3.78 (m, 3H), 3.71-3.59 (m, 3H), 3.01-2.88 (m, 3H), 2.70-2.64 (m, 2H), 2.44-2.30 (m, 3H), 2.28-2.14 (m, 3H), 2.11-1.92 (m, 6H), 1.90-1.76 (m, 3H), 1.51-1.39 (m, 4H), 0.92-0.75 (m, 6H).

Example 1-12

(*S*)-3-cyclopropyl-*N*-((1*S*,9*S*)-9-ethyl-5-fluoro-9-hydroxy-4-methyl-10,13-dioxo-2,3,9,1 0,13,15-hexahydro-1*H*,12*H*-benzo[*de*]pyrano[3',4':6,7]indolizino[1,2-*b*]quinolin-1-yl)-2 -hydroxypropionamide **12-A**

(*R*)-3-cyclopropyl-*N*-((1*S*,9*S*)-9-ethyl-5-fluoro-9-hydroxy-4-methyl-10,13-dioxo-2,3,9,1 0,13,15-hexahydro-1*H*,12*H*-benzo[*de*]pyrano[3',4':6,7]indolizino[1,2-*b*]quinolin-1-yl)-2 -hydroxypropionamide **12-B**

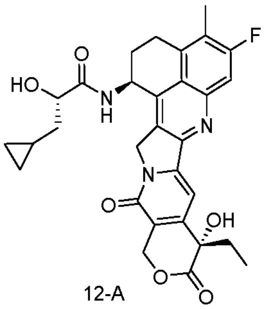

12-A

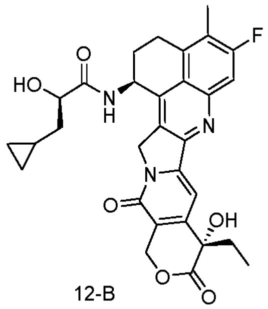

12-B

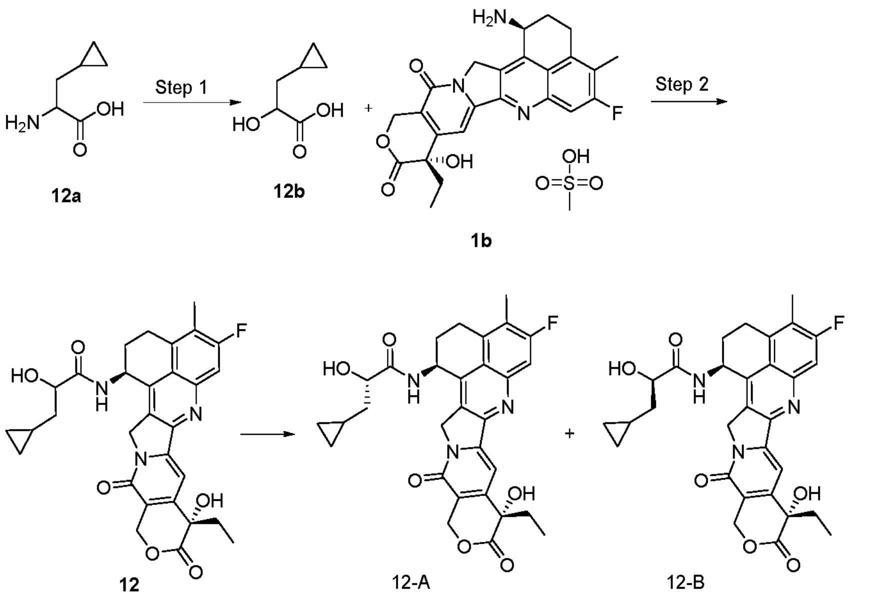


**12a** **12b** **1b**

**12** 12-A 12-B

Step 1

3-cyclopropyl-2-hydroxypropionic acid **12b**

**12a** (0.5 g, 3.87 mmol, supplier Adamas) was dissolved in 35 mL of a mixed solvent of water and acetic acid (V:V = 4:1), and the reaction solution was cooled to 0-5 °C in an ice-water bath, added dropwise with a 2 M aqueous solution of sodium nitrite (0.53 g, 7.74 mmol), and heated to room temperature and stirred for 3 h. Solid sodium chloride was added to the reaction solution to saturate the aqueous phase. The reaction solution was extracted with ethyl acetate (8 mL × 8), dried over anhydrous sodium sulfate, and filtered. The filtrate was concentrated to give the title product **12b** (0.45 g, 89.3% yield).

Step 2

(*S*)-3-cyclopropyl-*N*-((1*S*,9*S*)-9-ethyl-5-fluoro-9-hydroxy-4-methyl-10,13-dioxo-2,3,9,1 0,13,15-hexahydro-1*H*,12*H*-benzo[*de*]pyrano[3',4':6,7]indolizino[1,2-*b*]quinolin-1-yl)-2 -hydroxypropionamide **12-A**

(*R*)-3-cyclopropyl-*N*-((1*S*,9*S*)-9-ethyl-5-fluoro-9-hydroxy-4-methyl-10,13-dioxo-2,3,9,1 0,13,15-hexahydro-1*H*,12*H*-benzo[*de*]pyrano[3',4':6,7]indolizino[1,2-*b*]quinolin-1-yl)-2 -hydroxypropionamide **12-B**

To **1b** (45 mg, 0.085 mmol) were added 1.5 mL of ethanol and 1.5 mL of *N,N*-dimethylformamide, and the reaction solution was purged with argon three times, added dropwise with 0.1 mL of N-methylmorpholine, and stirred until it became clear. To the reaction solution were successively added **12b** (90 mg, 0.691 mmol), 1-hydroxybenzotriazole (34 mg, 0.251 mmol) and 1-(3-dimethylaminopropyl)-3-ethylcarbodiimide hydrochloride (49 mg, 0.256 mmol). After the addition was completed, the reaction solution was stirred at room temperature for 3 h, and concentrated under reduced pressure. The resulting crude compound **12** was purified by high performance liquid chromatography (separation conditions: chromatography column: Sharpsil-T C18 5 $\mu$m 21.2 $\times$ 250 mm; mobile phase: A-water (10 mmol of $NH_4OAc$), B-acetonitrile, gradient elution, flow rate: 18 mL/min) to give the title product (7 mg, 15 mg).
MS m/z (ESI): 547.9 [M+1].

Single-configuration compound (shorter retention time)

UPLC analysis: retention time: 1.345 min; purity: 72% (chromatography column: ZORBAX Ecliphase Plus C18 1.8 $\mu$m 2.1 $\times$ 50 mm; mobile phase: A-water (5 mmol of $NH_4OAc$), B-acetonitrile).
$^1H$ NMR (400 MHz, DMSO-$d_6$): $\delta$ 8.42 (d, 1H), 7.78 (d, 1H), 7.30 (s, 1H), 6.51 (s, 1H), 5.60-5.50 (m, 2H), 5.42 (s, 1H), 5.19 (q, 2H), 4.02-4.00 (m, 1H), 3.21-3.11 (m, 2H), 2.39 (s, 3H), 2.21-2.07 (m, 2H), 2.05-1.95 (m, 1H), 1.92-1.68 (m, 4H), 1.53-1.41 (m, 1H),0.87 (t, 3H), 0.48-0.34 (m, 2H), 0.14-0.01 (m, 2H).

Single-configuration compound (longer retention time)

UPLC analysis: retention time: 1.399 min; purity: 88% (chromatography column: ZORBAX Ecliphase Plus C18 1.8$\mu$m 2.1 $\times$ 50 mm; mobile phase: A-water (5 mmol of $NH_4OAc$), B-acetonitrile).
$^1H$ NMR (400 MHz, DMSO-$d_6$): $\delta$ 8.36 (d, 1H), 7.77 (d, 1H), 7.31 (s, 1H), 6.51 (s, 1H), 5.58-5.51 (m, 1H), 5.48 (d, 1H), 5.42 (s, 1H),5.20 (q, 2H), 4.09-4.02 (m, 1H), 3.22-3.11 (m, 2H), 2.39 (s, 3H), 2.27-2.06 (m, 2H), 2.05-1.95 (m, 1H), 1.93-1.81 (m, 2H), 1.65-1.43 (m, 2H), 1.32-1.21 (m, 1H), 0.87 (t, 3H), 0.48-0.33 (m, 2H), 0.14-0.01 (m, 2H).

Example 1-13 (reference example)

*N*-((1*S*,9*S*)-9-ethyl-5-fluoro-9-hydroxy-4-methyl-10,13-dioxo-2,3,9,10,13,15-hexa hydro-1*H*,12*H*-benzo[*de*]pyrano[3',4':6,7]indolizino[1,2-*b*]quinolin-1-yl)-2-hydroxyacet

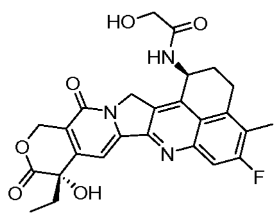

amide

**13**

The title compound **13** was prepared using the method disclosed in "Example 76 on page 147 of the specification in Patent EP2907824A1".

Example 1-14

*N*-((2*R*,10*S*)-10-benzyl-2-(cyclopropylmethyl)-1-(((1*S*,9*S*)-9-ethyl-5-fluoro-9-hydroxy-4 -methyl-10,13-dioxo-2,3,9,10,13,15-hexahydro-1*H*,12*H*-benzo[*de*]pyrano[3',4':6,7]indol izino[1,2-*b*]quinolin-1-yl)amino)-1,6,9,12,15-pentaoxo-3-oxa-5,8,11,14-tetraazahexadec -16-yl)-6-(2,5-dioxo-2,5-dihydro-1*H*-pyrrol-1-yl)hexanamide **14-A**

*N*-((2*S*,10*S*)-10-benzyl-2-(cyclopropylmethyl)-1-(((1*S*,9*S*)-9-ethyl-5-fluoro-9-hydroxy-4 -methyl-10,13-dioxo-2,3,9,10,13,15-hexahydro-1*H*,12*H*-benzo[*de*]pyrano[3',4':6,7]indol izino[1,2-*b*]quinolin-1-yl)amino)-1,6,9,12,15-pentaoxo-3-oxa-5,8,11,14-tetraazahexadec -16-yl)-6-(2,5-dioxo-2,5-dihydro-1*H*-pyrrol-1-yl)hexanamide **14-B**

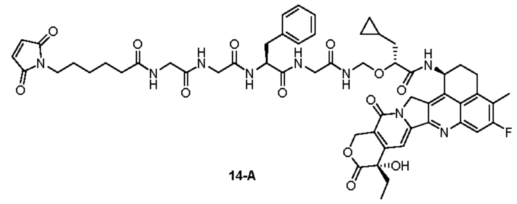

14-A

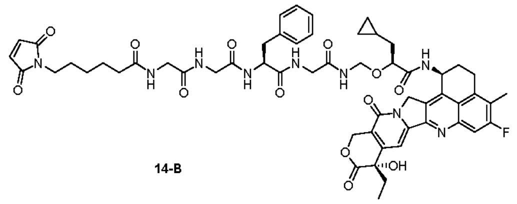

14-B

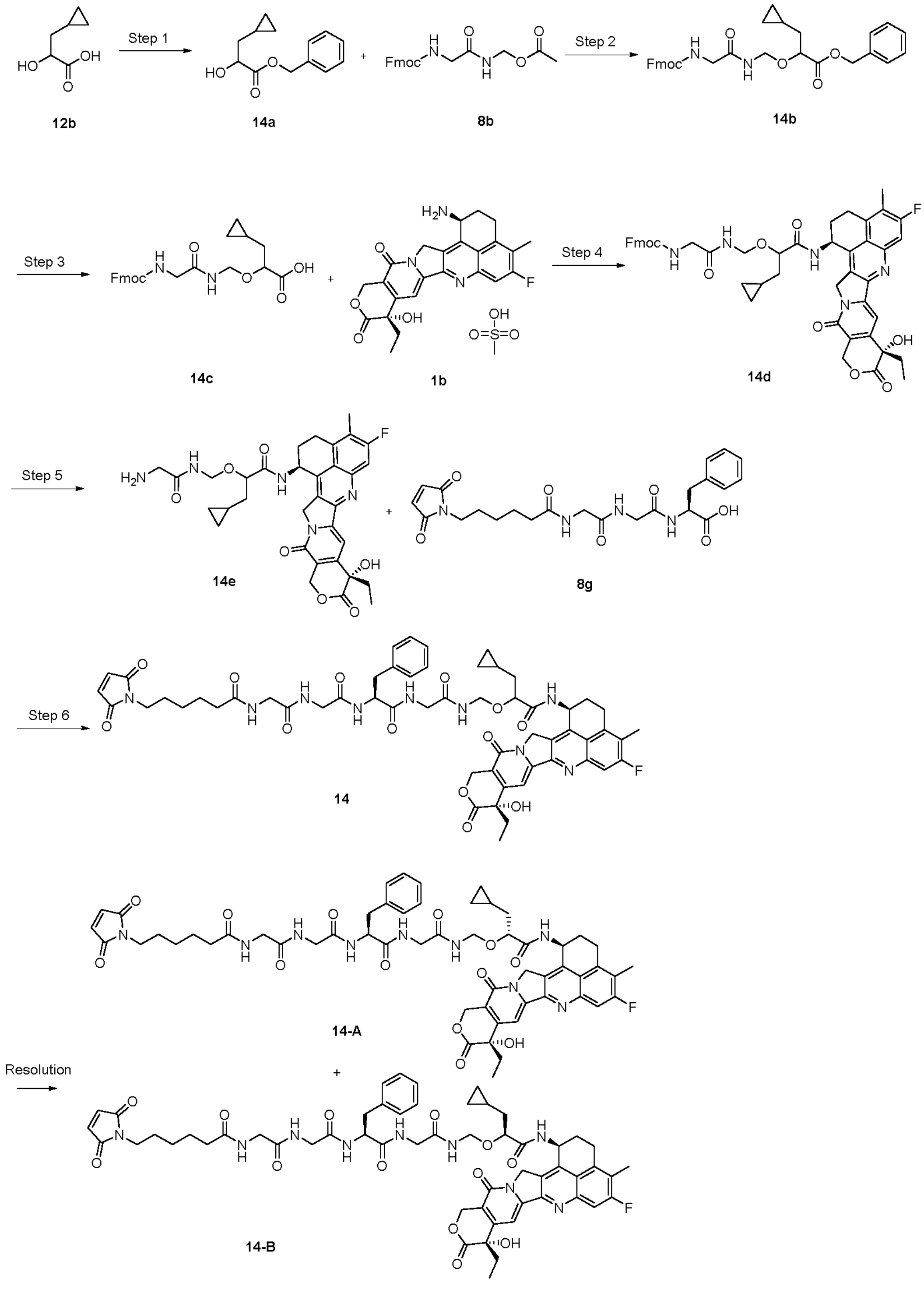


12b 14a 8b 14b

14c 1b 14d

14e 8g

14

14-A

+

14-B

Step 1

Benzyl 3-cyclopropyl-2-hydroxypropionate **14a**

**12b** (200 mg, 1.54 mmol) was dissolved in 20 mL of acetonitrile, and potassium carbonate (1.06 g, 7.68 mmol), benzyl bromide (0.16 mL, 1.34 mmol) and tetrabutylammonium iodide (28 mg, 0.07 mmol) were successively added. The reaction solution was stirred at room temperature for 48 h and filtered through celite, and the filter cake was rinsed with ethyl acetate (10 mL). The filtrates were combined and concentrated under reduced pressure, and the resulting residue was purified by silica gel column chromatography with developing solvent system C to give the title product **14a** (140 mg, 41.3% yield).

Step 2

Benzyl

10-(cyclopropylmethyl)-1-(9*H*-fluoren-9-yl)-3,6-dioxo-2,9-dioxa-4,7-diazaundecan-11-oate **14b**

To a reaction flask were added **14a** (94 mg, 0.427 mmol) and **8b** (130 mg, 0.353 mmol), followed by addition of 10 mL of tetrahydrofuran. The reaction solution was purged with argon three times, cooled to 0-5 °C in an ice-water bath, and added with potassium *tert*-butoxide (79 mg, 0.704 mmol), and the ice bath was removed. The reaction solution was heated to room temperature and stirred for 10 mi, added with 20 mL of ice water, and extracted with ethyl acetate (10 mL × 4). The organic phase was washed with saturated sodium chloride solution (20 mL), dried over anhydrous sodium sulfate, and filtered, and the filtrate was concentrated under reduced pressure. The resulting residue was purified by silica gel column chromatography with developing solvent system C to give the title product **14b** (50 mg, 26.8% yield).

MS m/z (ESI): 529.2 [M+1].

Step 3

10-(cyclopropylmethyl)-1-(9*H*-fluoren-9-yl)-3,6-dioxo-2,9-dioxa-4,7-diazaundecan-11-oic acid **14c**

**14b** (27 mg, 0.051 mmol) was dissolved in 3 mL of ethyl acetate, and palladium on carbon (7 mg, 10% loading, dry) was added. The reaction solution was purged with hydrogen three times and stirred at room temperature for 1 h. The reaction solution was filtered through celite, and the filter cake was rinsed with ethyl acetate. The filtrate was concentrated to give the crude title product **14e** (23 mg), which was directly used in the next step without purification.

MS m/z (ESI): 439.1 [M+1].

Step 4

(9*H*-fluoren-9-yl)methyl(2-((((3-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indoli zino[1,2-*b*]quinolin-1-yl)amino)-1-oxopropan-2-yl)oxy)methyl)amino)-2-oxoethyl)carb amate **14d**

To a reaction flask was added **1b** (22 mg, 42.38 μmol), followed by addition of 3 mL of *N*,*N*-dimethylformamide. The reaction solution was purged with argon three times, and cooled to 0-5 °C in an ice-water bath, followed by addition of a drop of triethylamine (4.3 mg, 42.49 μmol), crude product **14e** (23 mg, 51.1 μmol), and 4-(4,6-dimethoxy-1,3,5-triazin-2-yl)-4-methylmorpholinium chloride (17.6 mg, 63.6 μmol). The reaction solution was stirred in an ice bath for 40 min, added with 15 mL of water, and extracted with ethyl acetate (8 mL × 3). The organic phases were combined, washed with saturated sodium chloride solution (15 mL), dried over anhydrous sodium sulfate, and filtered. The filtrate was concentrated under reduced pressure. The resulting residue was purified by thin layer chromatography with developing solvent system B to give the title product **14d** (29 mg, 79.9% yield).

MS m/z (ESI): 856.1 [M+1].

Step 5

2-((2-aminoacetamido)methoxy)-3-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]ind olizino[1,2-*b*]quinolin-1-yl)propanamide **14e**

**14d** (29 mg, 33.9 μmol) was dissolved in 0.8 mL of dichloromethane, and 0.4 mL of diethylamine was added.

The reaction solution was stirred at room temperature for 1.5 h, and concentrated under reduced pressure. 1 mL of toluene was added, followed by concentration under reduced pressure; the procedures were repeated twice. The residue was slurried with 3 mL of *n*-hexane and let stand. Then, the supernatant was removed; and the procedures were repeated three times. The residue was concentrated under reduced pressure and dried using an oil pump to give the crude title product **14e** (22 mg), which was directly used in the next step without purification.

MS m/z (ESI): 634.1 [M+1].

Step 6

*N*-((2*R*,10*S*)-10-benzyl-2-(cyclopropylmethyl)-l-(((1*S*,9*S*)-9-ethyl-5-fluoro-9-hydroxy-4 -methyl-10,13-dioxo-2,3,9,10,13,15-hexahydro-1*H*,12*H*-benzo[*de*]pyrano[3',4':6,7]indol izino[1,2-*b*]quinolin-1-yl)amino)-1,6,9,12,15-pentaoxo-3-oxa-5,8,11,14-tetraazahexadec -16-yl)-6-(2,5-dioxo-2,5-dihydro-1*H*-pyrrol-1-yl)hexanamide **14-A**

N-((2*S*,10*S*)-10-benzyl-2-(cyclopropylmethyl)-1-(((1*S*,9*S*)-9-ethyl-5-fluoro-9-hydroxy-4 -methyl-10,13-dioxo-2,3,9,10,13,15-hexahydro-1*H*,12*H*-benzo[*de*]pyrano[3',4':6,7]indol izino[1,2-b]quinolin-1-yl)amino)-1,6,9,12,15-pentaoxo-3-oxa-5,8,11,14-tetraazahexadec -16-yl)-6-(2,5-dioxo-2,5-dihydro-1*H*-pyrrol-1-yl)hexanamide **14-B**

Crude **14e** (22 mg, 33.9 μmol) was dissolved in 2.5 mL of *N*,*N*-dimethylformamide. The reaction solution was purged with argon three times, cooled to 0-5 °C in an ice-water bath, and successively added with **8g** (24 mg, 50.8 μmol) and 4-(4,6-dimethoxy-1,3,5-triazin-2-yl)-4-methylmorpholinium chloride (14 mg, 50.6 μmol), and the ice bath was removed. The reaction solution was heated to room temperature and stirred for 1 h to give compound **14**. The reaction solution was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min) to give the title product (2 mg, 2 mg).

MS m/z (ESI): 1088.4 [M+1].

Single-configuration compound (shorter retention time)

UPLC analysis: retention time: 1.18 min; purity: 88% (chromatography column: ACQUITY UPLC BEHC18 1.7 μm 2.1 × 50 mm; mobile phase: A-water (5 mmol of $NH_4OAc$), B-acetonitrile).

Single-configuration compound (longer retention time)

UPLC analysis: retention time: 1.23 min; purity: 96% (chromatography column: ACQUITY UPLC BEHC18 1.7 μm 2.1 × 50 mm; mobile phase: A-water (5 mmol of $NH_4OAc$), B-acetonitrile).

Example 1-15

1-((*S*)-9-benzyl-22-(2,5-dioxo-2,5-dihydro-1*H*-pyrrol-1-yl)-5, 8,11,14,17-pentaoxo-2-ox a-4,7,10,13,16-pentaazadocosyl)-*N*-((1*S*,9*S*)-9-ethyl-5-fluoro-9-hydroxy-4-methyl-10,13 -dioxo-2,3,9,10,13,15-hexahydro-1*H*,12*H*-benzo[*de*]pyrano[3,4':6,7]indolizino[1,2-*b*]qu inolin-1-yl)cyclopropane-1-carboxamide **15**

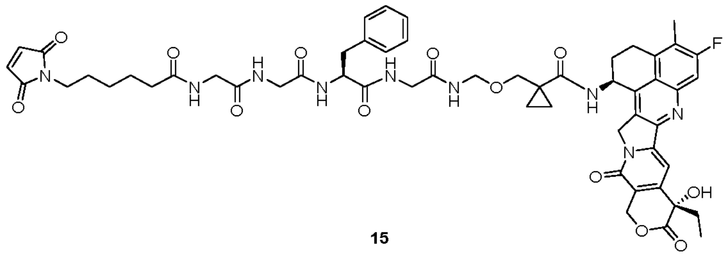

**15**

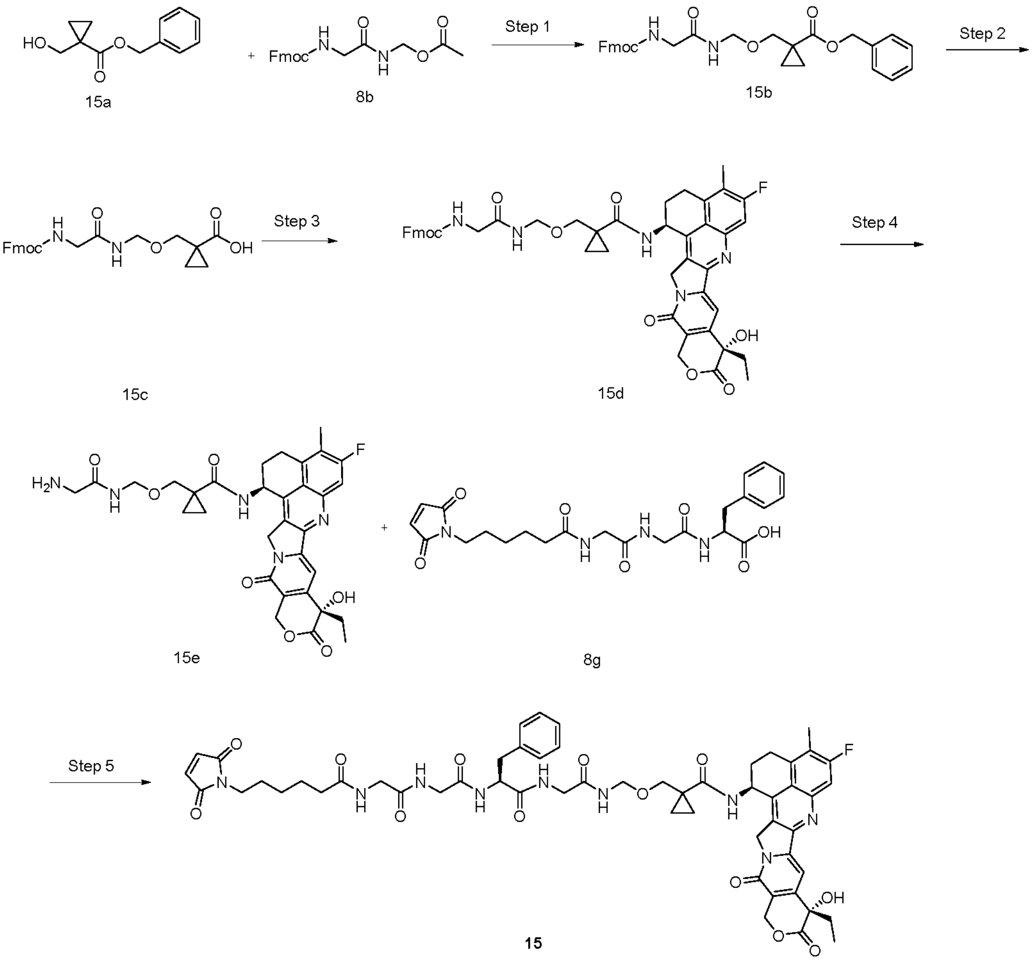


15a 8b 15b 15c 15d 15e 8g 15

Step 1

Benzyl

1-(10-(9H-fluoren-9-yl)-5,8-dioxo-2,9-dioxa-4,7-diazadecyl)cyclopropane-1-carboxylat e **15b**

To a reaction flask were added **8b** (500 mg, 1.35 mmol), 6 mL of tetrahydrofuran, and benzyl 1-hydroxymethyl cyclopropane-1-carbamate **15a** (233 mg, 1.13 mmol; prepared using the method disclosed in "Example 22-2 on page 262 of the specification in Patent Application EP2862856A1 "). The reaction solution was purged with argon three times, cooled to 0-5 °C in an ice-water bath, and added with sodium hydride (54 mg, 1.35 mmol), and the ice bath was removed. The reaction solution was heated to room temperature and stirred for 40 min, then cooled to 0 °C and added with 20 mL of ice water, and extracted with ethyl acetate (5 mL × 2) and chloroform (5 mL × 5). The organic phases were combined, washed with saturated sodium chloride solution (20 mL), dried over anhydrous sodium sulfate, and filtered. The filtrate was concentrated under reduced pressure, and the resulting residue was purified by silica gel column chromatography with developing solvent system B to give the title product **15b** (15 mg, 2.5% yield).
MS m/z (ESI): 515.2 [M+1].

Step 2

1-(10-(9*H*-fluoren-9-yl)-5,8-dioxo-2,9-dioxa-4,7-diazadecyl)cyclopropane-1-carboxylic acid **15c**

**15b** (15 mg, 0.029 mmol) was dissolved in 2 mL of ethyl acetate, and palladium on carbon (3 mg, 10% loading, dry) was added. The reaction solution was purged with hydrogen three times, stirred at room temperature for 4.5 h, and filtered through celite, and the filter cake was rinsed with ethyl acetate. The filtrate was concentrated to give the title product **15c** (11 mg, 89% yield).
MS m/z (ESI): 425.2 [M+1].

Step 3

(9*H*-fluoren-9-yl)methyl(2-((((1-(((1*S*,9*S*)-9-ethyl-5-fluoro-9-hydroxy-4-methyl-10,13-d ioxo-2,3,9,10,13,15-hexahydro-1*H*,12*H*-benzo[*de*]pyrano[3',4':6,7]indolizino[1,2-*b*]quin olin-1-yl)aminocarbonyl)cyclopropyl)methoxy)methyl)amino)2-oxoethyl)carbamate **15d**

To a reaction flask was added **1b** (10 mg, 0.021 mmol), followed by addition of 1 mL of *N*,*N*-dimethylformamide. The reaction solution was purged with argon three times, cooled to 0-5 °C in an ice-water bath, added with one drop of triethylamine, and added with **15c** (11 mg, 0.026 mmol), and with 4-(4,6-dimethoxy-1,3,5-triazine-2-yl)-4-methylmorpholinium chloride (10.7 mg, 0.039 mmol). After the addition was completed, the reaction solution was stirred at room temperature for 60 min, added with 10 mL of water, and extracted with ethyl acetate (5 mL × 3). The organic phase was washed with saturated sodium chloride solution (10 mL), dried over anhydrous sodium sulfate, and filtered. The filtrate was concentrated under reduced pressure, and the resulting residue was purified by thin layer chromatography with developing solvent system B to give the title product **15d** (19 mg, 87.0% yield).

MS m/z (ESI): 842.2 [M+1].

Step 4

1-(((2-aminoacetylamino)methoxy)methyl)-*N*-((1*S*,9*S*)-9-ethyl-5-fluoro-9-hydroxy-4-m ethyl-10,13-dioxo-2,3,9,10,13,15-hexahydro-1*H*,12*H*

benzo[*de*]pyrano[3',4':6,7]indolizino[1,2-*b*]quinolin-1-yl)cyclopropane-1-carboxamide **15e**

**15d** (19 mg, 22.56 μmol) was dissolved in 2 mL of dichloromethane, and 1 mL of diethylamine was added. The reaction solution was stirred at room temperature for 1.5 h, and concentrated under reduced pressure at 0 °C. 1 mL of toluene was added, followed by concentration under reduced pressure; the procedures were repeated twice. The residue was slurried with 3 mL of *n*-hexane, and the upper *n*-hexane layer was removed; the procedures were repeated three times. The slurry was concentrated under reduced pressure to give the crude title product **15e** (13.9 mg), which was directly used in the next step without purification.

MS m/z (ESI): 620.1 [M+1].

Step 5

1-((S)-9-benzyl-22-(2,5-dioxo-2,5-dihydro-1*H*-pyrrol-1-yl)-5, 8,11,14,17-pentaoxo-2-ox a-4,7,10,13,16-pentaazadocosyl)-*N*-((1*S*,9*S*)-9-ethyl-5-fluoro-9-hydroxy-4-methyl-10,13 -dioxo-2,3,9,10,13,15-hexahydro-1*H*,12*H*-benzo[*de*]pyrano[3',4':6,7]indolizino[1,2-*b*]q uinolin-1-yl)cyclopropane-1-carboxamide **15**

Crude **15e** (13.9 mg, 22.4 μmol) was dissolved in 1 mL of *N*,*N*-dimethylformamide. The reaction solution was purged with argon three times, cooled to 0-5 °C in an ice-water bath, and added with **8g** (15.8 mg, 33.4 μmol), and with 4-(4,6-dimethoxy-1,3,5-triazin-2-yl)-4-methylmorpholinium chloride (9.3 mg, 33.6 μmol). The reaction solution was heated to room temperature and stirred for 60 min, and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product **15** (2.5 mg, 10.3% yield).

MS m/z (ESI): 1074.2 [M+1].

$^1$H NMR (400 MHz, DMSO-*d*$_6$): δ 8.51-8.37 (m, 1H), 8.22 (t, 1H), 8.14-8.02 (m, 2H), 8.011-7.94 (m, 1H), 7.82-7.73 (m, 1H), 7.29 (s, 1H), 7.26-7.10 (m, 3H), 6.98 (s, 1H), 6.53-6.47 (m, 1H), 5.62-5.50 (m, 1H), 5.45-5.36 (m, 1H), 5.35-5.23 (m, 2H), 5.13-5.02 (m, 2H), 4.61-4.50 (m, 2H), 4.42-4.28 (m, 2H), 3.76-3.61 (m, 3H), 3.60-3.45 (m, 3H), 3.27-3.23 (m, 1H), 3.20-2.81 (m,7H), 2.75-2.61 (m, 3H), 241-2.28 (m, 3H), 2.23-2.13 (m, 2H), 2.11-2.01 (m, 1H), 2.03-1.94 (m, 1H), 1.90 (s, 1H), 1.87-1.74 (m, 2H), 1.53-1.36 (m, 3H), 1.29-1.08 (m, 4H), 0.90-0.68 (m, 4H).

Example 1-16

1-((*S*)-9-benzyl-22-(2,5-dioxo-2,5-dihydro-1*H*-pyrrol-1-yl)-5, 8,11,14,17-pentaoxo-2-ox a-4,7,10,13,16-pentaazadocosyl)-*N*-((1*S*,9*S*)-9-ethyl-5-fluoro-9-hydroxy-4-methyl-10,13 -dioxo-2,3,9,10,13,15-hexahydro-1*H*,12*H*-benzo[*de*]pyrano[3',4':6,7]indolizino[1,2-*b*]q uinolin-1-yl)cyclobutane-1-carboxamide **16**

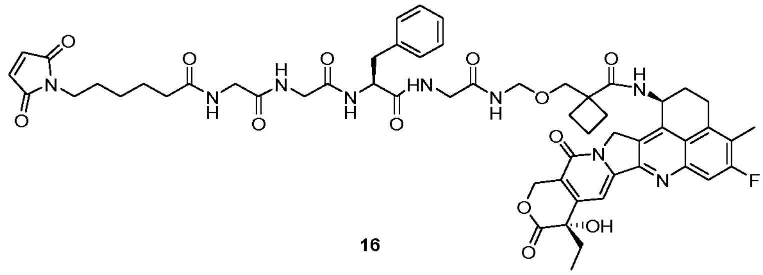

16

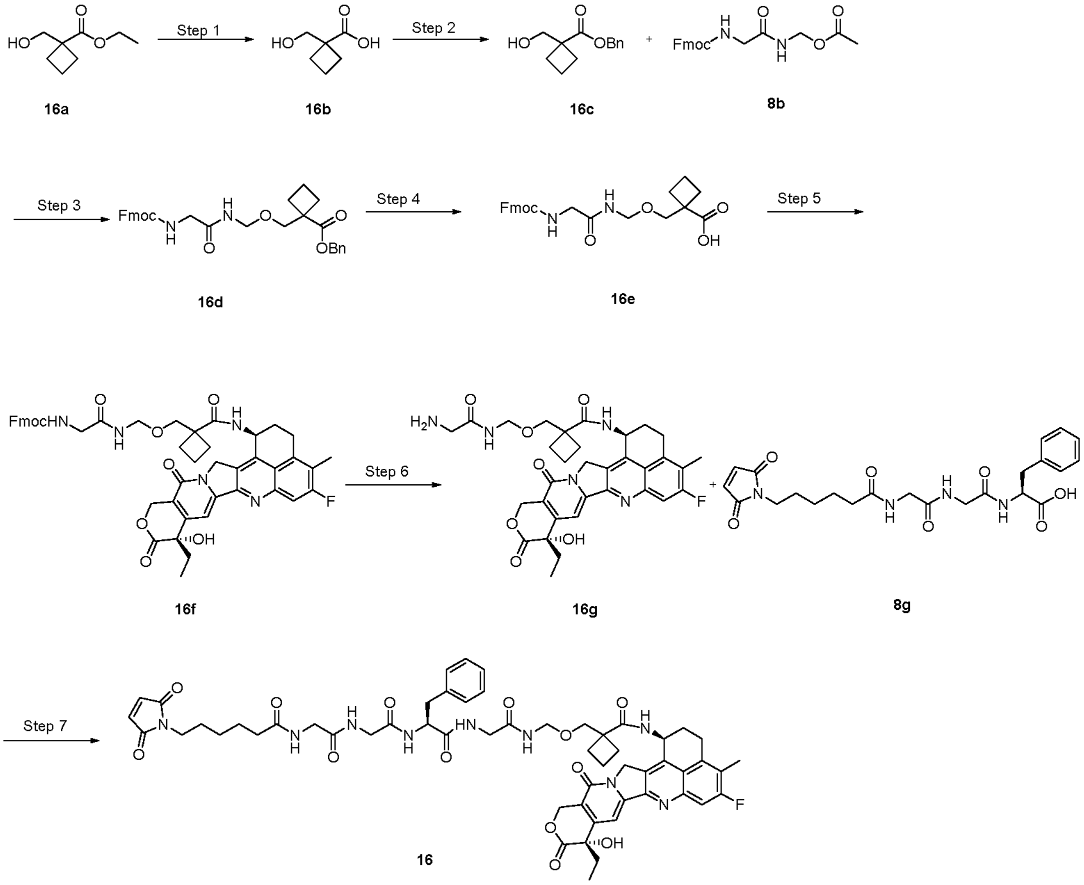


16a 16b 16c 8b 16d 16e 16f 16g 8g 16

Step 1

1-(hydroxymethyl)cyclobutane-1-carboxylic acid **16b**

Ethyl 1-(hydroxymethyl)cyclobutanecarboxylate **16a** (250 mg, 1.58 mmol, supplier Alfa) was dissolved in methanol (2 mL) and water (1 mL), and sodium hydroxide (126 mg, 3.15 mmol) was added. The reaction solution was heated to 40 °C, stirred for 3 h, cooled to room temperature, and concentrated under reduced pressure to remove the organic solvent. The reaction solution was reversely extracted with diethyl ether (10 mL) to collect the aqueous phase. The aqueous phase was adjusted to pH 3-4 with 6 N aqueous hydrochloric acid and concentrated under reduced pressure to give a solid. 3 mL of toluene was added, followed by concentration under reduced pressure to dryness; the procedures were repeated three times. The residue was dried using an oil pump to give the crude title product **16b** (206 mg), which was directly used in the next step without purification.

MS m/z (ESI, NEG): 129.2 [M-1].

Step 2

Benzyl 1-(hydroxymethyl)cyclobutane-1-carboxylate **16c**

Crude **16b** (206 mg, 1.58 mmol) was dissolved in acetonitrile (15 mL), anhydrous potassium carbonate (1.09 g, 7.90 mmol) and tetrabutylammonium iodide (29 mg, 78.51 μmol) were added, and benzyl bromide (216 mg, 1.26 mmol) was added. The reaction solution was stirred at room temperature overnight, and filtered. The filtrate was concentrated under reduced pressure, and the resulting residue was purified by silica gel column chromatography with developing solvent system C to give the title product **16e** (112 mg, 32.1% yield).
MS m/z (ESI): 221.1 [M+1].

Step 3

Benzyl

1-(10-(9H-fluoren-9-yl)-5,8-dioxo-2,9-dioxa-4,7-diazadecyl)cyclobutane-1-carboxylate **16d**

To a reaction flask were added **16c** (77 mg, 0.35 mmol) and **8b** (100 mg, 0.27mmol), followed by addition of 3 mL of tetrahydrofuran. The reaction solution was purged with argon three times, cooled to 0-5 °C in an ice-water bath, and added with potassium tert-butoxide (61 mg, 0.54 mmol). The reaction solution was stirred in an ice bath for 10 min, added with 20 mL of ice water, and extracted with ethyl acetate (5 mL) and chloroform (5 mL × 5). The organic phases were combined and concentrated. The resulting residue was dissolved in 3 mL of 1,4-dioxane, and 0.5 mL of water, sodium bicarbonate (27 mg, 0.32 mmol) and 9-fluorenylmethyl chloroformate (71 mg, 0.27 mmol) were added. The reaction solution was stirred at room temperature for 1 h, added with 20 mL of water, and extracted with ethyl acetate (10 mL × 3). The organic phase was washed with saturated sodium chloride solution (20 mL), dried over anhydrous sodium sulfate, and filtered. The filtrate was concentrated under reduced pressure, and the resulting residue was purified by silica gel column chromatography with developing solvent system C to give the title product **16d** (24 mg, 16.7% yield).
MS m/z (ESI): 551.3 [M+23].

Step 4

1-(10-(9H-fluoren-9-yl)-5,8-dioxo-2,9-dioxa-4,7-diazadecyl)cyclobutane-1-carboxylic acid **16e**

**16d** (12 mg, 22.7 μmol) was dissolved in 1.5 mL of a solvent mixture of tetrahydrofuran and ethyl acetate (V:V = 2:1), and palladium on carbon (5 mg, 10% loading) was added. The reaction solution was purged with hydrogen three times and stirred at room temperature for 2 h. The reaction solution was filtered through celite, and the filter cake was rinsed with ethyl acetate. The filtrate was concentrated under reduced pressure to give the crude title product **16e** (10 mg), which was directly used in the next step without purification.

Step 5

(9*H*-fluoren-9-yl)methyl(2-((((1-(((1*S*,9*S*)-9-ethyl-5-fluoro-9-hydroxy-4-methyl-10,13-d ioxo-2,3,9,10,13,15-hexahydro-1*H*,12*H*-benzo[*de*]pyrano[3',4':6,7]indolizino[1,2-*b*]quin olin-1-yl)aminocarbonyl)cyclobutyl)methoxy)methyl)amino)-2-oxoethyl)carbamate **16f** To a reaction flask was added **1b** (7.5 mg, 0.014 mmol), followed by addition of 1 mL of *N*,*N*-dimethylformamide. The reaction solution was purged with argon three times, cooled to 0-5 °C in an ice-water bath, added with a drop of triethylamine, added with a solution of crude **16e** (10 mg) in 0.5 mL of N,N-dimethylformamide, and then added with 4-(4,6-dimethoxy-1,3,5-triazin-2-yl)-4-methylmorpholinium chloride (6 mg, 0.026 mmol). The reaction solution was stirred in an ice bath for 30 min, added with 10 mL of water, and extracted with ethyl acetate (10 mL × 3). The organic phase was washed with saturated sodium chloride solution (10 mL), dried over anhydrous sodium sulfate, and filtered. The filtrate was concentrated under reduced pressure, and the resulting residue was purified by thin layer chromatography with developing solvent system B to give the title product **16f** (10.6 mg, 87.8% yield).
MS m/z (ESI): 856.2 [M+1].

Step 6

1-(((2-aminoacetylamino)methoxy)methyl)-*N*-((1*S*,9*S*)-9-ethyl-5-fluoro-9-hydroxy-4-m ethyl-10,13-dioxo-2,3,9,10,13,15-hexahydro-1*H*,12*H*-benzo[*de*]pyrano[3',4':6,7]indolizi no[1,2-*b*]quinolin-1-yl)cyclobutane-1-carboxamide **16g**

**16f** (10.6 mg, 12.4 μmol) was dissolved in 0.6 mL of dichloromethane, and 0.3 mL of diethylamine was added. The reaction solution was stirred at room temperature for 2 h, concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The residue was concentrated under reduced pressure to give the crude title product **16g** (8 mg), which was directly used in the next step without purification.

MS m/z (ESI): 634.1 [M+1].

Step 7

1-((*S*)-9-benzyl-22-(2,5-dioxo-2,5-dihydro-1*H*-pyrrol-1-yl)-5,8,11,14,17-pentaoxo-2-ox a-4,7,10,13,16-pentaazadocosyl)-*N*-((1*S*,9*S*)-9-ethyl-5-fluoro-9-hydroxy-4-methyl-10,13 -dioxo-2,3,9,10,13,15-hexahydro-1*H*,12*H*-benzo[*de*]pyrano[3',4':6,7]indolizino[1,2-*b*]q uinolin-1-yl)cyclobutane-1-carboxamide **16**

Crude **16g** (8 mg) was dissolved in 1 mL of *N*,*N*-dimethylformamide. The reaction solution was added with **8g** (8.8 mg, 18.6 μmol), and with 4-(4,6-dimethoxy-1,3,5-triazin-2-yl)-4-methylmorpholinium chloride (5.2 mg, 18.8 μmol). The reaction solution was stirred at room temperature for 30 min, and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min) to give the title product **16** (1.0 mg, 7.2% yield).

MS m/z (ESI): 1088.0 [M+1].

Example 1-17

(1r,4r)-*N*-((*S*)-7-benzyl-1-(1-(((1*S*,9*S*)-9-ethyl-5-fluoro-9-hydroxy-4-methyl-10,13-diox o-2,3,9,10,13,15-hexahydro-1*H*,12*H*-benzo[*de*]pyrano [3',4':6,7]indolizino[1,2-*b*]quinoli n-1-yl)aminocarbonyl)cyclopropoxy)-3,6,9,12,15-pentaoxo-17,20,23,26,29,32,35,38,41-nonaxo-2,5,8,11,14-pentaazatetratridec-43-yl)-4-((2,5-dioxo-2,5-dihydro-1*H*-pyrrol-1-yl )methyl)cyclohexane-1-carboxamide 17

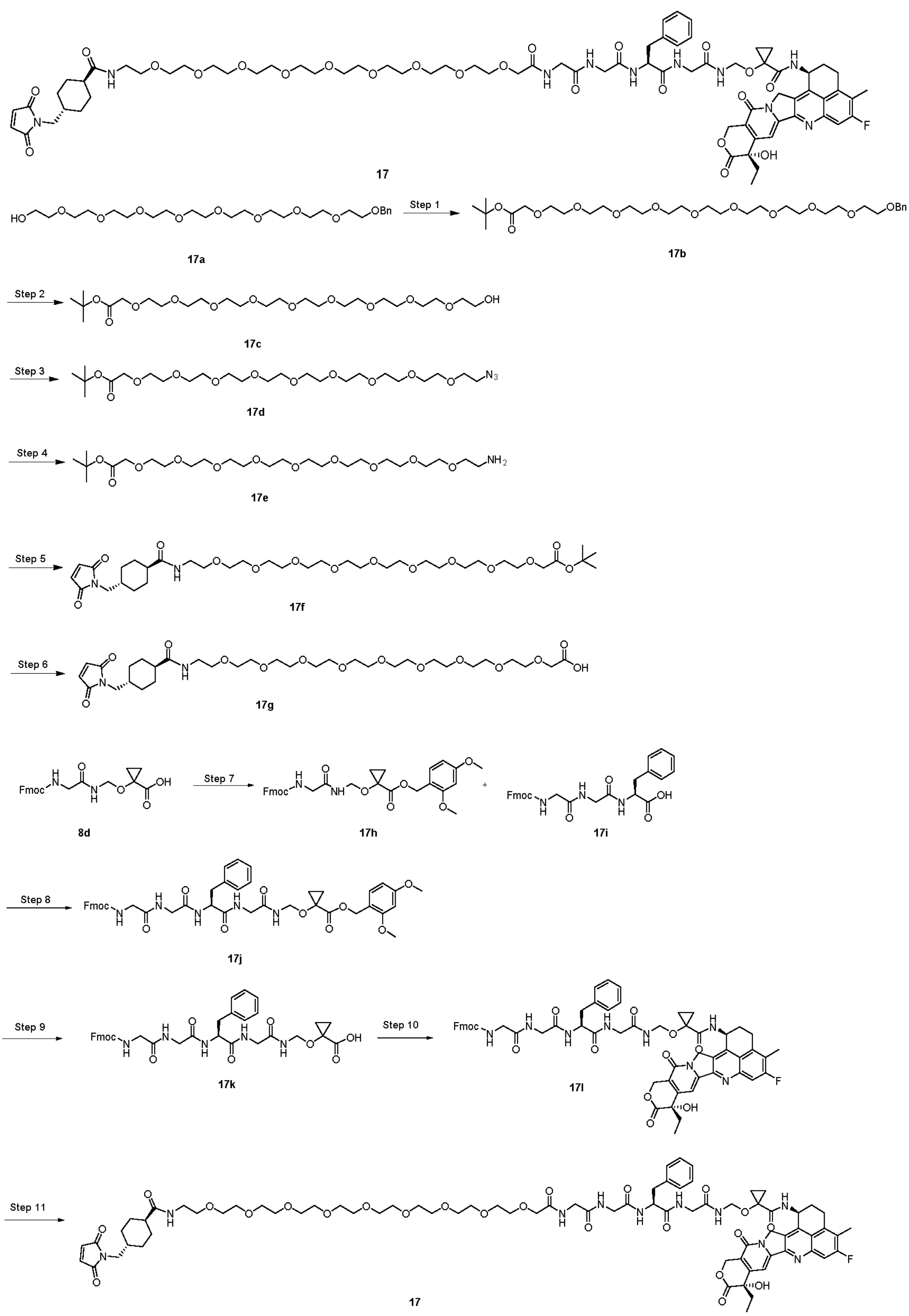


17

17a 17b

17c

17d

17e

17f

17g

8d 17h + 17i

17j

17k 17l

17

Step 1

*Tert*-butyl 1-phenyl-2,5,8,11,14,17,20,23,26,29-decaoxatriundec-31-oate **17b**

1-phenyl-2,5,8,11,14,17,20,23,26-nonaoxadioctadecyl-28-ol **17a** (0.34 g, 0.67 mmol, supplier Bide) was dissolved in 10 mL of dichloromethane, and silver oxide (0.24 g, 1.01 mmol), *tert*-butyl bromoacetate (0.16 g, 0.81 mmol)

and potassium iodide (0.07 g, 0.40 mmol) were successively added. The reaction solution was stirred at room temperature for 3 h, and filtered. The filtrate was concentrated under reduced pressure, and the resulting residue was purified by silica gel column chromatography with developing solvent system B to give the title product **17b** (0.42 g, 100% yield).
MS m/z (ESI): 636.3 [M+18].

Step 2

*Tert*-butyl 29-hydroxy-3,6,9, 12, 15, 18,21,24,27 -nonaxoheneicosano-1-oate **17c**

**17b** (417 mg, 0.67 mmol) was dissolved in 15 mL of tetrahydrofuran, and palladium on carbon (110 mg, 10% loading, dry) was added. The reaction solution was purged with hydrogen three times, stirred at 60 °C for 3 h, and filtered through celite, and the filter cake was rinsed with tetrahydrofuran. The filtrate was concentrated to give the crude title product **17c** (357 mg), which was directly used in the next step without purification. MS m/z (ESI): 546.2 [M+18].

Step 3

*Tert*-butyl 29-azido-3,6,9,12,15,18,21,24,27-nonaxomontanyl-1-oate **17d**

**17c** (357 mg, 0.675 mmol) was dissolved in 10 mL of toluene, and diphenyl phosphoryl azide (279 mg, 1.014 mmol) and 1,8-diazabicycloundec-7-ene (206 mg, 1.353 mmol) were added. The reaction solution was purged with argon three times, stirred at room temperature for 2 h, and then heated to 105 °C for 19 h. The reaction solution was cooled to room temperature, concentrated, added with 20 mL of water, and extracted with ethyl acetate (10 mL $\times$ 4). The organic phase was washed with saturated sodium chloride solution (20 mL), dried over anhydrous sodium sulfate, and filtered. The filtrate was concentrated under reduced pressure. The resulting residue was purified by silica gel column chromatography with developing solvent system B to give the title product **17d** (412 mg).
MS m/z (ESI): 571.3 [M+18].

Step 4

*Tert*-butyl 29-amino-3,6,9,12,15,18,21,24,27-nonaxomontanyl-1-oate **17e**

**17d** (230 mg, 0.415 mmol) was dissolved in 8 mL of tetrahydrofuran, and palladium on carbon (58 mg, 10% loading, dry) was added. The reaction solution was purged with hydrogen three times, stirred at room temperature for 2 h, and filtered through celite, and the filter cake was rinsed with tetrahydrofuran. The filtrate was concentrated to give the crude title product **17e** (220 mg), which was directly used in the next step without purification.
MS m/z (ESI): 528.2 [M+1].

Step 5

*Tert-butyl* 1-((1*r*,4*r*)-4-( (2,5-dioxo-2,5-dihydro-1*H*-pyrrol-1-yl)methyl)cyclohexyl)-1-oxo-5,8,11,1 4,17,20,23,26,29-nonaoxa-2-azatriundec-31-oate **17f**

(1*r*,4*r*)-4-((2,5-dioxo-2,5-dihydro-1*H*-pyrrol-1-yl)methyl)cyclohexane-1-carboxylic acid (98.5 mg, 0.415 mmol) was dissolved in 10 mL of dichloromethane, and 2-(7-benzotriazol oxide)-*N*,*N*,*N'*,*N'*-tetramethyluronium hexafluorophosphate (190 mg, 0.500 mmol) and *N*,*N*-diisopropylethylamine (162 mg, 1.253 mmol) were added. The reaction solution was purged with argon three times, added with crude **17e** (220 mg, 0.417 mmol), stirred at room temperature for 1 h, added with 15 mL of water, and extracted with dichloromethane (8 mL $\times$ 3). The organic phases were combined, washed with saturated sodium chloride solution (15 mL), dried over anhydrous sodium sulfate, and filtered. The filtrate was concentrated under reduced pressure. The resulting residue was purified by silica gel column chromatography with developing solvent system B to give the title product **17f** (122 mg, 39.2% yield).
MS m/z (ESI): 747.2 [M+1].

Step 6

1-((1*r*,4*r*)-4-((2,5-dioxo-2,5-dihydro-1*H*-pyrrol-1-yl)methyl)cyclohexyl)-1-oxo-5,8,11,1 4,17,20,23,26,29-nonaoxa-2-azatriundec-31-oic acid **17g**

**17f** (122 mg, 0.163 mmol) was dissolved in 0.8 mL of dichloromethane, and 0.4 mL of trifluoroacetic acid was

added. The reaction solution was stirred at room temperature for 1 h, diluted with 15 mL of dichloromethane, and concentrated under reduced pressure; 10 mL of n-hexane was added, followed by concentration under reduced pressure; the procedures were repeated twice. Then the reaction solution was added with 10 mL of toluene, concentrated under reduced pressure, then slurried with 10 mL of a mixed solvent of *n*-hexane:diethyl ether = 5:1 three times to adjust pH to approach 7, concentrated, and dried with an oil pump to give the title product **17g** (98 mg, 86.8% yield).
MS m/z (ESI): 691.2 [M+1].

Step 7

2,4-dimethoxybenzyl 1-((2-((((9*H*-fluoren-9-yl)methoxy)carbonyl)amino)acetamido)methoxy)cyclopropyl-1-carboxylate **17h**

**8d** (164 mg, 0.40 mmol) was dissolved in dichloromethane (5 mL), and 2,4-dimethoxybenzyl alcohol (81 mg, 0.48 mmol), 1-ethyl-(3-dimethylaminopropyl) carbonyldiimine hydrochloride (115 mg, 0.60 mmol) and 4-dimethylaminopyridine (5 mg, 0.041 mmol) were successively added. After the addition was completed, the reaction solution was stirred at room temperature for 1 h, and added with 20 mL of water, and the layers were separated after shaking. The aqueous phase was extracted with dichloromethane (8 mL × 3). The organic phases were combined, washed with saturated sodium chloride solution (20 mL), dried over anhydrous sodium sulfate, and filtered. The filtrate was concentrated under reduced pressure. The resulting residue was purified by silica gel column chromatography with developing solvent system C to give the title product **17h** (124 mg, 55.4% yield).
MS m/z (ESI): 583.1 [M+23].

Step 8

2,4-dimethoxybenzyl (S)-1-((11-benzyl-1-(9*H*-fluoren-9-yl)-3,6,9,12,15-pentaoxo-2-oxa-4,7,10,13,16-pentaaz epin-17-yl)oxy)cyclopropyl-1-carboxylate **17j**

**17h** (39 mg, 69.6 μmol) was dissolved in 0.6 mL of dichloromethane, and 0.3 mL of diethylamine was added. The reaction solution was stirred at room temperature for 1 h, concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times, followed by concentration under reduced pressure. The resulting crude product was dissolved in 2 mL of *N*,*N*-dimethylformamide, and (((9*H*-fluoren-9-yl)methoxy)carbonyl)glycyl-L-phenylalanine **17i** (35 mg, 69.8 μmol, prepared using the method disclosed in "Examples 7-12 on page 13 of the specification in Patent Application CN108853514A") and 4-(4,6-dimethoxy-1,3,5-triazin-2-yl)-4-methylmorpholinium chloride (23 mg, 83.1 μmol) were added. The reaction solution was stirred at room temperature for 1 h, added with 10 mL of water, and extracted with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **17j** (48 mg, 83.9% yield).
MS m/z (ESI): 822.0 [M+1].

Step 9

(5)-1-((11-benzyl-1-(9*H*-fluoren-9-yl)-3,6,9,12,15-pentaoxo-2-oxa-4,7,10,13,16-pentaaz epanyl-17-yl)oxy)cyclopropane-1-carboxylic acid **17k**

**17j** (48 mg, 58.4 μmol) was dissolved in 1.4 mL of a 3% (v/v) solution of dichloroacetic acid in dichloromethane. The reaction solution was cooled to 0-5 °C in an ice-water bath, added with triethylsilane (21 mg, 180.6 μmol), and stirred in an ice bath for 3 h. The reaction solution was concentrated under reduced pressure in an ice bath to remove half of the organic solvent, added with 5 mL of diethyl ether, then heated to room temperature naturally and slurried to precipitate a white solid, and filtered. The filter cake was collected and dried with an oil pump to give the title product **17k** (33 mg, 84.1% yield).

Step 10

(9*H*-fluoren-9-yl)methyl((*S*)-7-benzyl-1-(1-(((1*S*,9*S*)-9-ethyl-5-fluoro-9-hydroxy-4-met hyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)cyclopropoxy)-3,6,9,12-tetraoxo-2,5,8,11-tetraazatriden-13-yl)carba mate **171**

To a reaction flask was added **1b** (20 mg, 42.4 μmol), followed by addition of 1 mL of 10% (v/v) solution of methanol in dichloromethane solution. The reaction solution was purged with argon three times, cooled to 0-5 °C in an ice-water bath, added one drop of triethylamine, and stirred until **1b** was dissolved. **17k** (33 mg, 49.1 μmol) was dissolved in 1 mL of 10% (v/v) solution of methanol in dichloromethane, then the reaction mixture was added dropwise to the above reaction solution, followed by addition of 4-(4,6-dimethoxy-1,3,5-triazin-2-yl)-4-methylmorpholinium chloride (17.6 mg, 63.6 μmol). The reaction solution was heated to room temperature and stirred for 1 h, added with 10 mL of dichloromethane and 5 mL of water, stirred for 5 min, and let stand for layer separation. The organic phase was collected, and the aqueous phase was extracted with dichloromethane (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **17l** (37 mg, 80.2% yield).

MS m/z (ESI): 1090.1 [M+1].

Step 11

(1*r*,4*r*)-*N*-((*S*)-7-benzyl-1-(1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]qui nolin-1-yl)aminocarbonyl)cyclopropoxy)-3,6,9,12,15-pentaoxo-17,20,23,26,29,32,35,38 ,41-nonaxo-2,5,8,11,14-pentaazatetratridec-43-yl)-4-((2,5-dioxo-2,5-dihydro-1*H*-pyrrol-1-yl)methyl)cyclohexane-1-carboxamide **17**

**17l** (15.5 mg, 14.23 μmol) was dissolved in 0.6 mL of dichloromethane, and 0.3 mL of diethylamine was added. The reaction solution was stirred at room temperature for 1.5 h, concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The reaction solution was concentrated under reduced pressure and then dried with an oil pump. The resulting crude product was dissolved in 1 mL of *N*,*N*-dimethylformamide. The reaction solution was added with **17g** (11 mg, 15.92 μmol) and 4-(4,6-dimethoxy-1,3,5-triazin-2-yl)-4-methylmorpholinium chloride (6.0 mg, 21.68 μmol), purged with argon three times, stirred at room temperature for 30 min, and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product **17** (6 mg, 27.4% yield).

MS m/z (ESI): 1556.4 [M+18].

$^1$H NMR (400 MHz, DMSO-$d_6$): δ 8.98 (d, 1H), 8.76 (s, 1H), 8.20 (br, 1H), 8.12-7.95 (m, 3H), 7.93-7.76 (m, 2H), 7.75-7.66 (m, 2H), 7.24 (s, 1H), 7.20-7.05 (m, 6H), 6.97 (s, 1H), 6.64 (br, 1H), 6.55 (d, 1H), 6.47 (s, 1H), 5.61-5.52 (m, 2H), 5.37 (s, 1H), 5.33-5.23 (m, 2H), 5.18 (s, 1H), 5.13 (s, 1H), 5.05 (s, 1H), 5.00 (s, 1H), 4.65-4.55 (m, 2H), 4.53-4.45 (m, 1H), 4.38-4.28 (m, 2H), 3.84 (s, 2H), 3.67 (d, 3H), 3.60-3.40 (m, 33H), 3.18 (d, 1H), 3.15-3.08 (m, 3H), 2.28 (s, 3H), 2.00-1.92 (m, 3H), 1.85 (s, 2H), 1.82-1.73 (m, 2H), 1.68-1.52 (m, 4H), 1.29-1.15 (m, 3H), 0.86-0.76 (m, 5H).

Example 1-18

(1*r*,4*r*)-*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indoli zino[1,2-*b*]quinolin-1-yl)amino)-1,6,9,12,15,18-hexaoxo-3,20,23,26,29,32,35,38,41,44-decaoxa-5,8,11,14,17-pentaaza-tetrahexadec-46-yl)-4-((2,5-dioxo-2,5-dihydro-1*H*-pyrro 1-1-yl)methyl)cyclohexane-1-carboxamide **18**

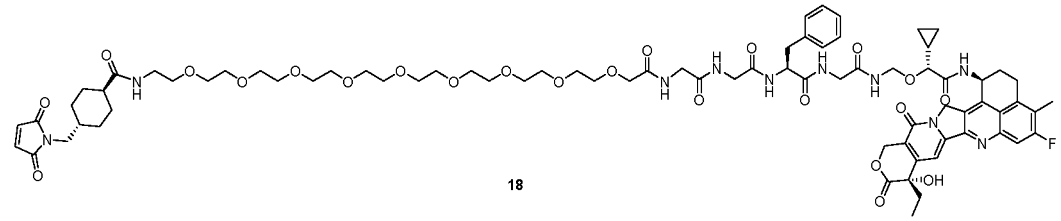

18

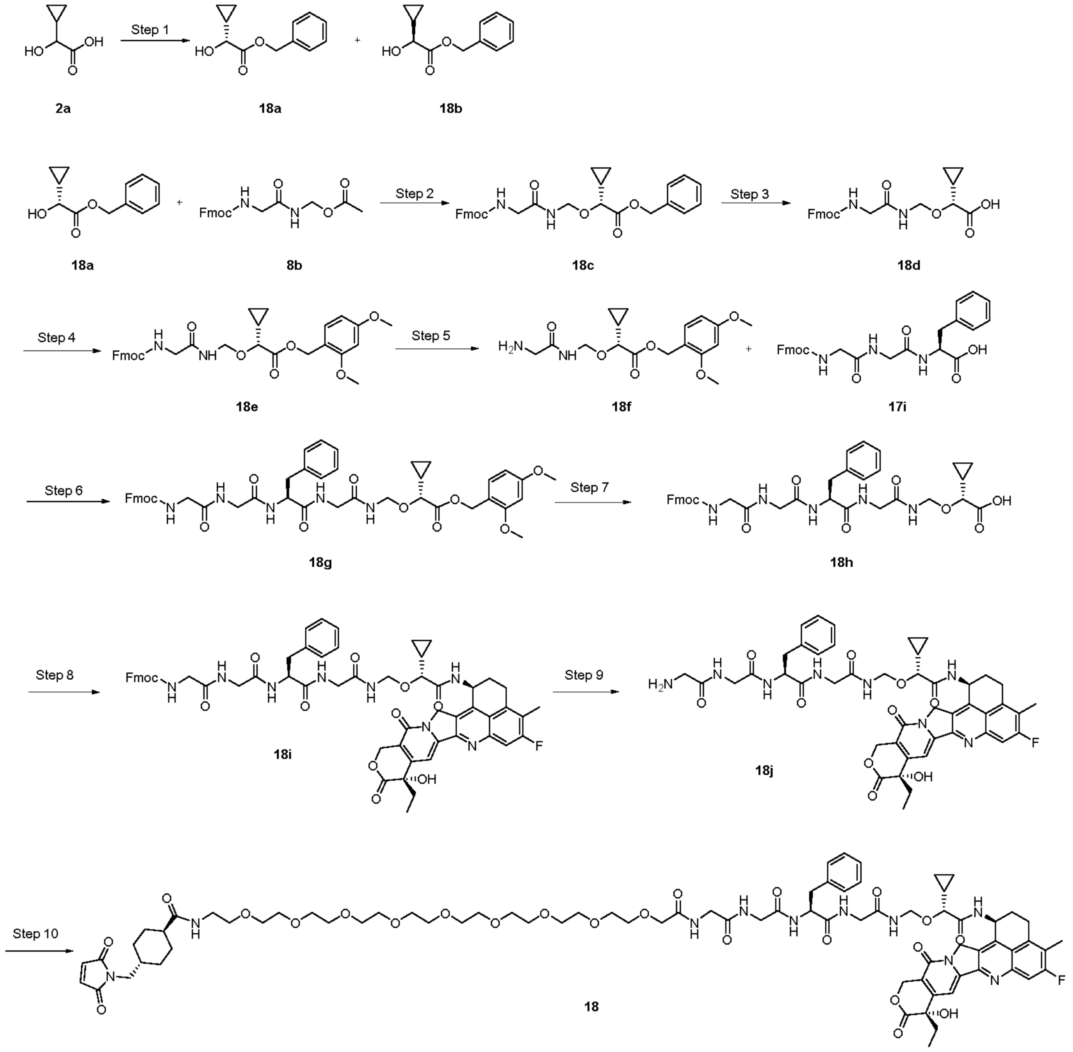


2a 18a 18b

18a 8b 18c 18d

18e 18f 17i

18g 18h

18i 18j

18

Step 1

Benzyl (*R*)-2-cyclopropyl-2-hydroxyacetate **18a**

Benzyl (*S*)-2-cyclopropyl-2-hydroxyacetate **18b**

**2a** (7.4 g, 63.7 mmol) was dissolved in 200 mL of acetonitrile, and potassium carbonate (35 g, 253.6 mmol), benzyl bromide (9.3 g, 54.4 mmol) and tetrabutylammonium iodide (500 mg, 1.36 mmol) were successively added. The reaction solution was stirred at room temperature for 16 h and filtered through celite, and the filter cake was rinsed with ethyl acetate (10 mL). The filtrates were combined and concentrated under reduced pressure, and the resulting residue was purified by silica gel column chromatography with developing solvent system C. The resulting residue (4.1 g) was further purified by chiral resolution to give the title products **18a** (1.1 g) and **18b** (1.2 g).

Step 2

Benzyl (*R*)-10-cyclopropyl-1-(9*H*-fluoren-9-yl)-3,6-dioxo-2,9-dioxa-4,7-diazaundecan-11-oate **18c**

**8b** (3.1 g, 8.41mmol) was dissolved in tetrahydrofuran (55 mL), **18a** (2.0 g, 9.70 mmol) was added. The reaction solution was cooled to 0-5 °C in an ice-water bath, added with potassium *tert*-butoxide (1.89 g, 16.84 mmol), stirred in an ice-water bath for 10 min, added with ethyl acetate (30 mL) and water (20 mL), and let stand for layer separation. The aqueous layer was extracted with chloroform (30 mL × 5), and the organic layers were combined, and concentrated under reduced pressure. The resulting residue was dissolved in 1,4-dioxane (32 mL) and water (8 mL), added with sodium carbonate (1.78 g, 16.79 mmol) and 9-fluorenylmethyl chloroformate (2.18 g, 8.42 mmol), stirred at room temperature for 2 h, added with water (30 mL), and extracted with ethyl acetate (50 mL × 3). The organic phases were combined, washed with saturated sodium chloride solution (30 mL × 2), dried over anhydrous sodium sulfate, and filtered. The filtrate was concentrated under reduced pressure. The resulting residue was purified by column chromatography with developing solvent system C to give the title product **18c** (1.3g, 30.0% yield).

MS m/z (ESI): 515.2 [M+1].

Step 3

(*R*)-10-cyclopropyl-1-(9*H*-fluoren-9-yl)-3,6-dioxo-2,9-dioxa-4,7-diazaundecan-11-oic acid **18d**

**18c** (1.29 g, 2.51 mmol) was dissolved in ethyl acetate (15 mL), and palladium on carbon (260 mg, 10% loading, dry) was added. The reaction solution was purged with hydrogen three times, stirred at room temperature for 5 h, and filtered through celite, and the filter cake was rinsed with ethyl acetate (20 mL) and methanol (20 mL). The filtrate was concentrated to give the crude title product **18d** (980 mg), which was directly used in the next step without purification.

MS m/z (ESI): 425.1 [M+1].

Step 4

2,4-dimethoxybenzyl(*R*)-10-cyclopropyl-1-(9*H*-fluoren-9-yl)-3,6-dioxo-2,9-dioxa-4,7-d iazaundecan-11-ester **18e**

Crude **18d** (980 mg, 2.31 mmol) was dissolved in dichloromethane (15 mL), and 2,4-dimethoxybenzyl alcohol (777 mg, 4.62 mmol), 1-ethyl-(3-dimethylaminopropyl) carbonyldiimine hydrochloride (664 mg, 3.46 mmol) and 4-dimethylaminopyridine (28 mg, 0.23 mmol) were added. The reaction solution was stirred at room temperature for 1 h, and concentrated under reduced pressure to remove the organic solvent. The reaction solution was added with 20 mL of water and extracted with ethyl acetate (50 mL × 3). The organic phases were combined, washed with saturated sodium chloride solution (30 mL × 2), dried over anhydrous sodium sulfate, and filtered. The filtrate was concentrated under reduced pressure. The resulting residue was purified by column chromatography with developing solvent system C to give the title product **18e** (810 mg, 61.1% yield).

MS m/z (ESI): 575.0 [M+1].

Step 5

2,4-dimethoxybenzyl(R)-2-((2-aminoacetylamino)methoxy)-2-cyclopropylacetate **18f**

**18e** (33 mg, 57.4 μmol) was dissolved in 0.6 mL of dichloromethane, and 0.3 mL of diethylamine was added. The reaction solution was stirred at room temperature for 1 h, concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The slurry was concentrated under reduced pressure to give the crude title product **18f** (21 mg), which was directly used in the next step without purification.

Step 6

2,4-dimethoxybenzyl(11*S*,19R)-11-benzyl-19-cyclopropyl-1-(9*H*-fluoren-9-yl)-3,6,9,12, 15-pentaoxo-2,18-dioxa-4,7,10,13,16-pentaazaeicosa-20-oate **18g**

Crude **18f** (21 mg, 57.4 μmol) was dissolved in 3 mL of *N*,*N*-dimethylformamide. The reaction solution was added with **17i** (29 mg, 57.8 μmol), and 4-(4,6-dimethoxy-1,3,5-triazin-2-yl)-4-methylmorpholinium chloride (19 mg, 68.7

μmol). The reaction solution was stirred at room temperature for 1 h, and added with 10 mL of water, and extracted with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **18g** (37 mg, 77.1% yield).

MS m/z (ESI): 853.0 [M+18].

Step 7

(11*S*,19*R*)-11-benzyl-19-cyclopropyl-1-(9*H*-fluoren-9-yl)-3,6,9,12,15-pentaoxo-2,18-di oxa-4,7,10,13,16-pentaazaeicosa-20-oic acid **18h**

**18g** (37 mg, 44.3 μmol) was dissolved in 1.4 mL of a 3% (v/v) solution of dichloroacetic acid in dichloromethane. The reaction solution was cooled to 0-5 °C in an ice-water bath, added with triethylsilane (15.4 mg, 132.4 μmol), and stirred in an ice bath for 3 h. The reaction solution was concentrated under reduced pressure in an ice bath to remove half of the organic solvent, added with 5 mL of diethyl ether, then heated to room temperature naturally and slurried to precipitate a white solid, and filtered. The filter cake was collected and dried with an oil pump to give the title product **18h** (24 mg, 79.1% yield).

MS m/z (ESI): 708.2 [M+23].

Step 8

(9*H*-fluoren-9-yl)methyl((2*R*,10*S*)-10-benzyl-2-cyclopropyl-1-(((1*S*,9*S*)-9-ethyl-5-fluor o-9-hydroxy-4-methyl-10,13-dioxo-2,3,9,10,13,15-hexahydro-1*H*,12*H*-benzo[*de*]pyrano [3',4':6,7]indolizino[1,2-*b*]quinolin-1-yl)amino)-1,6,9,12,15-pentaoxo-3-oxa-5,8,11,14-t etraazahexadec-16-yl)carbamate **18i**

To a reaction flask was added **1b** (30 mg, 63.6 μmol), followed by addition of 1 mL of 10% (v/v) solution of methanol in dichloromethane solution. The reaction solution was purged with argon three times, cooled to 0-5 °C in an ice-water bath, added one drop of triethylamine, and stirred until **1b** was dissolved. **18h** (65 mg, 94.8 μmol) was dissolved in 1 mL of 10% (v/v) solution of methanol in dichloromethane, then the reaction mixture was added dropwise to the above reaction solution, followed by addition of 4-(4,6-dimethoxy-1,3,5-triazin-2-yl)-4-methylmorpholinium chloride (27 mg, 97.6 μmol). The reaction solution was heated to room temperature and stirred for 1 h, added with 10 mL of dichloromethane and 5 mL of water, stirred for 5 min, and let stand for layer separation. The organic phase was collected, and the aqueous phase was extracted with dichloromethane (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **18i** (25 mg, 35.6% yield).

MS m/z (ESI): 1104.4 [M+1].

Step 9

(*S*)-2-(2-(2-aminoacetylamino)acetylamino)-*N*-(2-((((*R*)-1-cyclopropyl-2-(((1S,9S)-9-et hyl-5-fluoro-9-hydroxy-4-methyl-10, 13-dioxo-2,3,9, 10, 13, 15-hexahydro-1*H*,12*H*-benzo [de]pyrano[3',4':6,7]indolizino[1,2-*b*]quinolin-1-yl)amino)-2-oxoethoxy)methyl)amino)-2-oxoethoxy)-3-phenylpropanamide **18j**

**18i** (12 mg, 10.9 μmol) was dissolved in 0.6 mL of dichloromethane, and 0.3 mL of diethylamine was added. The reaction solution was stirred at room temperature for 1.5 h, concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The slurry was concentrated under reduced pressure to give the crude title product **18j** (10 mg), which was directly used in the next step without purification.

MS m/z (ESI): 881.0 [M+1].

Step 10

(1*r*,4*r*)-*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indoli zino[1,2-*b*]quinolin-1-yl)amino)-1,6,9,12,15,18-hexaoxo-3,20,23,26,29,32,35,38,41,44-decaoxa-5,8,11,14,17-pentaaza-tetrahexadec-46-yl)-4-((2,5-dioxo-2,5-dihydro-1*H*-pyrro 1-1-yl)methyl)cyclohexane-1-carboxamide **18**

Crude **18j** (10 mg) was dissolved in 1 mL of *N*,*N*-dimethylformamide. The reaction solution was added with **17g** (8.5 mg, 12.3 μmol), and with 4-(4,6-dimethoxy-1,3,5-triazin-2-yl)-4-methylmorpholinium chloride (4.6 mg, 16.6 μmol). The reaction solution was stirred at room temperature for 30 min, filtered, and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product **18** (9.5 mg, 56.2% yield).

MS m/z (ESI): 1570.2 [M+18].

$^1$H NMR (400 MHz, DMSO-$d_6$): δ 8.77 (d, 1H), 8.59-8.55 (m, 1H), 8.42 (d, 1H), 8.37-8.28 (m, 1H), 8.25-8.06 (m, 2H), 7.96-7.86 (m, 1H), 7.86-7.70 (m, 2H), 7.32-7.28 (m, 1H), 7.25-7.14 (m, 3H), 6.67 (m, 1H), 5.96 (s, 1H), 5.80-5.72 (m, 1H), 5.62-5.52 (m, 2H), 5.43-5.30 (m, 3H), 5.28-5.17 (m, 2H), 5.12-5.08 (m, 1H), 4.72-4.35 (m, 8H), 3.95-3.70 (m, 13H), 3.35-3.22 (m, 14H), 2.42-2.32 (m, 3H), 2.05-1.98 (m, 4H), 1.88-1.82 (m, 12H), 1.47-1.39 (m, 3H), 1.32-1.18 (m, 11H), 0.90-0.80 (m, 4H), 0.52-0.37 (m, 3H), 0.32-0.18 (m, 2H).

Example 1-19

(1*r*,4*r*)-N-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indoli zino[1,2-*b*]quinolin-1-yl)amino)-1,6,9,12,15,18-hexaoxo-3,20,23,26,29,32,35,38,41,44-decaoxa-5,8,11,14,17-pentaaza-tetrahexadec-46-yl)-4-((2,5-dioxo-2,5-dihydro-1*H*-pyrro l-1-yl)methyl)cyclohexane-1-carboxamide **19**

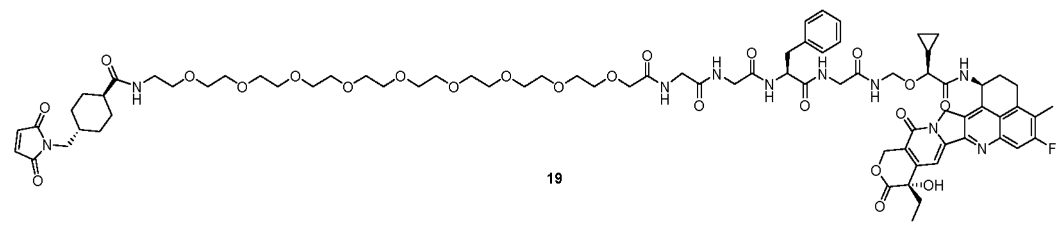

19

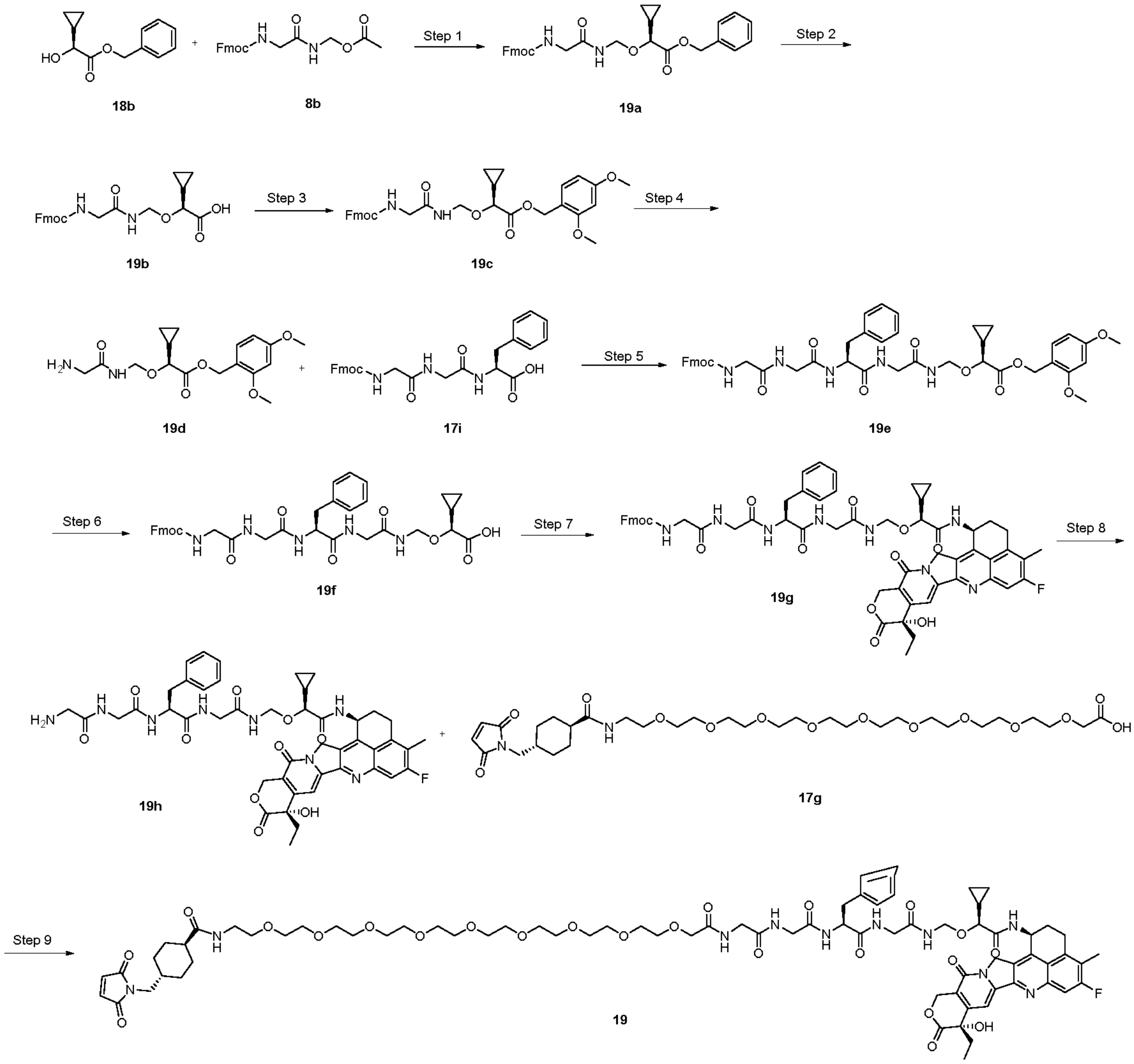


18b 8b 19a

19b 19c

19d 17i 19e

19f 19g

19h 17g

19

Step 1

Benzyl

(*S*)-10-cyclopropyl-1-(9*H*-fluoren-9-yl)-3,6-dioxo-2,9-dioxa-4,7-diazaundecan-11-oate **19a**

To a reaction flask was added **18b** (252 mg, 1.22 mmol), followed by addition of 4 mL of dichloromethane. The reaction solution was purged with argon three times, cooled to 0-5 °C in an ice-water bath, added with lithium *tert*-butoxide (98 mg, 1.22 mmol), stirred in an ice-water bath for 15 min until it became clear, then added with **8b** (300 mg, 814.3 μmol), and stirred in an ice-water bath for 2.5 h. Water (10mL) was added for liquid separation. The aqueous phase was extracted with dichloromethane (8 mL × 2), and the organic phases were combined and washed with water (10 mL × 1), washed with saturated brine (10 mL × 2), dried over anhydrous sodium sulfate, and filtered and concentrated to give the crude product. The resulting residue was purified by silica gel column chromatography with developing solvent system C to give the title product **19a** (282 mg, 67.2% yield).

Step 2

(*S*)-10-cyclopropyl-l-(9*H*-fluoren-9-yl)-3,6-dioxo-2,9-dioxa-4,7-diazaundecan-11-oic acid **19b**

**19a** (280 mg, 0.554 mmol) was dissolved in 8 mL of ethyl acetate, and palladium on carbon (84 mg, 10% loading, dry) was added. The reaction solution was purged with hydrogen three times, stirred at room temperature for 3 h, and filtered through celite, and the filter cake was rinsed with ethyl acetate. The filtrate was concentrated to give the crude title product **19b** (230 mg), which was directly used in the next step without purification.

Step 3

2,4-dimethoxybenzyl(*S*)-10-cyclopropyl-1-(9*H*-fluoren-9-yl)-3,6-dioxo-2,9-dioxa-4,7-di azaundecan-11-oate **19c**

Crude **19b** (230 mg, 541.8 μmol) was dissolved in 7 mL of dichloromethane, and 2,4-dimethoxybenzyl alcohol (136.7 mg, 812.7 μmol), 1-ethyl-(3-dimethylaminopropyl) carbodiimidate hydrochloride (155 mg, 808.5 μmol) and 4-dimethylaminopyridine (6.6 mg, 53.5 μmol) were successively added. The reaction solution was stirred at room temperature for 16 h, diluted with 10 mL of dichloromethane, washed with water (10 mL × 1) and saturated brine (10 mL × 2), dried over anhydrous sodium sulfate, and filtered and concentrated to give the crude product. The resulting residue was purified by thin layer chromatography with developing solvent system B to give the title product **19c** (159 mg, 51.0% yield).

Step 4

2,4-dimethoxybenzyl(*S*)-2-((2-aminoacetylamino)methoxy)-2-cyclopropylacetate **19d 19c** (60 mg, 104.4 μmol) was dissolved in 1 mL of dichloromethane, and 0.5 mL of diethylamine was added. The reaction solution was stirred at room temperature for 1 h, concentrated under reduced pressure. 2 mL of toluene was added, followed by concentration under reduced pressure; the procedures were repeated twice. The residue was slurried with 3 mL of n-hexane, and the upper n-hexane layer was removed; the procedures were repeated three times. The slurry was concentrated under reduced pressure to give the crude title product **19d** (21 mg), which was directly used in the next step without purification.

Step 5

2,4-dimethoxybenzyl(11*S*,19*S*)-11-benzyl-19-cyclopropyl-1-(9*H*-fluoren-9-yl)-3,6,9,12, 15-pentaoxo-2,18-dioxa-4,7,10,13,16-pentaazaeicosa-20-oate **19e**

Crude **19d** (36 mg, 102.2 μmol) was dissolved in 4 mL of *N*,*N*-dimethylformamide. The reaction solution was added with **17i** (52 mg, 103.6 μmol), and 4-(4,6-dimethoxy-1,3,5-triazin-2-yl)-4-methylmorpholinium chloride (34.6 mg, 125.0 μmol). The reaction solution was stirred at room temperature for 1 h, and added with 10 mL of water, and extracted with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **19e** (70 mg, 80.2% yield).

Step 6

(11*S*,19*S*)-11-benzyl-19-cyclopropyl-1-(9*H*-fluoren-9-yl)-3,6,9,12,15-pentaoxo-2,18-dio xa-4,7,10,13,16-pentaazaeicosa-20-oic acid **19f**

**19e** (70 mg, 83.7 μmol) was dissolved in 2.5 mL of a 3% (v/v) solution of dichloroacetic acid in dichloromethane. The reaction solution was cooled to 0-5 °C in an ice-water bath, added with triethylsilane (29 mg, 249.4 μmol), and stirred in an ice bath for 3 h. The reaction solution was concentrated under reduced pressure in an ice bath to remove half of the organic solvent, added with 5 mL of diethyl ether, then heated to room temperature naturally and slurried to precipitate a white solid, and filtered. The filter cake was collected and dried with an oil pump to give the title product **19f** (57 mg, 99.2% yield).

Step 7

(9*H*-fluoren-9-yl)methyl((2*S*,10*S*)-10-benzyl-2-cyclopropyl-1-(((1*S*,9*S*)-9-ethyl-5-fluoro -9-hydroxy-4-methyl-10,13-dioxo-2,3,9,10,13,15-hexahydro-1*H*,12*H*-benzo[*de*]pyrano[ 3',4':6,7]indolizino[1,2-*b*]quinolin-1-yl)amino)-1,6,9,12,15-pentaoxo-3-oxa-5,8,11,14-te traazahexadec-16-yl)carbamate **19g**

To a reaction flask was added **1b** (30 mg, 63.6 μmol), followed by addition of 1 mL of 10% (v/v) solution of methanol in dichloromethane solution. The reaction solution was purged with argon three times, cooled to 0-5 °C in an ice-water bath, added one drop of triethylamine, and stirred until **1b** was dissolved. **19f** (57 mg, 83.1 μmol) was dissolved in 1 mL of 10% (v/v) solution of methanol in dichloromethane, then the reaction mixture was added dropwise to the above reaction solution, followed by addition of 4-(4,6-dimethoxy-1,3,5-triazin-2-yl)-4-methylmorpholinium chloride (26 mg, 93.9 μmol). The reaction solution was heated to room temperature and stirred for 1 h, added with 10 mL of dichloromethane and 5 mL of water, stirred for 5 min, and let stand for layer separation. The organic phase was collected,

and the aqueous phase was extracted with dichloromethane (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **19g** (56 mg, 79.8% yield).

MS m/z (ESI): 1103.1 [M+1].

Step 8

(S)-2-(2-(2-aminoacetylamino)acetylamino)-*N*-(2-((((*S*)-1-cyclopropyl-2-((1*S*,9*S*)-9-eth yl-5-fluoro-9-hydroxy-4-methyl-10,13-dioxo-2,3,9,10,13,15-hexahydro-1*H*,12*H*-benzo[ *de*]pyrano[3',4':6,7]indolizino[1,2-*b*]quinolin-1-yl)amino)-2-oxoethoxy)methyl)amino)-2-oxoethyl)-3-phenylpropanamide **19h**

**19g** (4.6 mg, 4.16 μmol) was dissolved in 1.5 mL of dichloromethane, and 0.75 mL of diethylamine was added. The reaction solution was stirred at room temperature for 1.6 h, concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The slurry was concentrated under reduced pressure to give the crude title product **19h** (4.0 mg), which was directly used in the next step without purification.

Step 9

(1*r*,4*r*)-*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indoli zino[1,2-*b*]quinolin-1-yl)amino)-1,6,9,12,15,18-hexaoxo-3,20,23,26,29,32,35,38,41,44-decaoxa-5,8,11,14,17-pentaaza-tetrahexadec-46-yl)-4-((2,5-dioxo-2,5-dihydro-1*H*-pyrro 1-1-yl)methyl)cyclohexane-1-carboxamide **19**

Crude **19h** (4.0 mg) was dissolved in 1 mL of *N*,*N*-dimethylformamide. The reaction solution was added with **17g** (2.9 mg, 4.2 μmol), and with 4-(4,6-dimethoxy-1,3,5-triazin-2-yl)-4-methylmorpholinium chloride (1.5 mg, 5.4 μmol). The reaction solution was stirred at room temperature for 40 min, filtered, and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product **19** (2.1 mg, 32.4% yield).

$^1$H NMR (400 MHz, DMSO-$d_6$): δ 8.71-8.62 (m, 1H), 8.59-8.51 (m, 1H), 8.34-8.26 (m, 1H), 8.14-8.02 (m, 2H), 7.95-7.86 (m, 1H), 7.83-7.69 (m, 2H), 7.35-7.31 (m, 1H), 7.29-7.11 (m, 3H), 7.01 (s, 1H), 6.72-6.50 (m, 3H), 5.59-5.50 (m, 2H), 5.42 (s, 2H), 5.38-5.18 (m, 3H), 4.79-4.69 (m, 2H), 4.61-4.42 (m, 3H), 3.91 (s, 2H), 3.79-3.65 (m, 4H), 3.63-3.44 (m, 13H), 3.41-3.30 (m, 2H), 3.26-3.09 (m, 5H), 3.08-2.84 (m, 4H), 2.81-2.64 (m, 3H), 2.42-2.28 (m, 3H), 2.24-2.12 (m, 2H), 2.05-1.93 (m, 4H), 1.89-1.77 (m, 2H), 1.72-1.56 (m, 3H), 1.53-1.38 (m, 3H), 1.34-1.10 (m, 11H), 0.94-0.78 (m, 5H), 0.52-0.35 (m, 3H).

Example 1-20 (reference example)

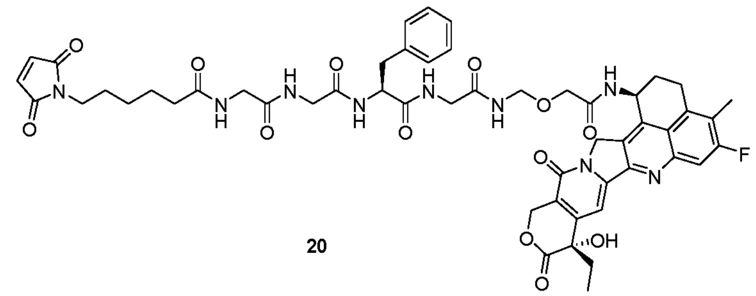

20

The title compound **20** was synthesized using the method provided in "Example 58 on page 163 of the specification in Patent CN104755494A".

The following antibodies can be prepared using a conventional preparation method for antibodies, and can be obtained by, for example, vector construction, transfection of eukaryotic cells such as HEK293 cells (Life Technologies

Cat. No. 11625019), and expression purification.

The following is the sequence of trastuzumab:

Light chain

DIQMTQSPSSLSASVGDRVTITCRASQDVNTAVAWYQQKPGKAPKLLIYSASFL
YSGVPSRFSGSRSGTDFTLTISSLQPEDFATYYCQQHYTTPPTFGQGTKVEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

SEQ ID NO: 1

Heavy chain

EVQLVESGGGLVQPGGSLRLSCAASGFNIKDTYIHWVRQAPGKGLEWVARIYP
TNGYTRYADSVKGRFTISADTSKNTAYLQMNSLRAEDTAVYYCSRWGGDGFY
AMDYWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVYTLPPSREEMTKNQVSLTCLV
KGFYPSDIAVEWESNGQPENNYKTTPPVLDSDGSFFLYSKLTVDKSRWQQGNV
FSCSVMHEALHNHYTQKSLSLSPGK

SEQ ID NO: 2

The following is the sequence of pertuzumab:

Light chain

DIQMTQSPSSLSASVGDRVTITCKASQDVSIGVAWYQQKPGKAPKLLIYSASYR
YTGVPSRFSGSGSGTDFTLTISSLQPEDFATYYCQQYYIYPYTFGQGTKVEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

SEQ ID NO: 3

Heavy chain

EVQLVESGGGLVQPGGSLRLSCAASGFTFTDYTMDWVRQAPGKGLEWVADV
NPNSGGSIYNQRFKGRFTLSVDRSKNTLYLQMNSLRAEDTAVYYCARNLGPSF
YFDYWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNG
KEYKCKVSNKALPAPIEKTISKAKGQPREPQVYTLPPSREEMTKNQVSLTCLVK
GFYPSDIAVEWESNGQPENNYKTTPPVLDSDGSFFLYSKLTVDKSRWQQGNVF
SCSVMHEALHNHYTQKSLSLSPGK

SEQ ID NO: 4

The following is the sequence of B7H3 antibody 1F9DS:

Light chain

DTVVTQEPSFSVSPGGTVTLTCGLSSGSVSTSHYPSWYQQTPGQAPRMLIYNTN
TRSSGVPDRFSGSILGNKAALTITGAQADDESDYYCAIHVDRDIWVFGGGTKLT
VLGQPKANPTVTLFPPSSEELQANKATLVCLISDFYPGAVTVAWKADGSPVKA
GVETTKPSKQSNNKYAASSYLSLTPEQWKSHRSYSCQVTHEGSTVEKTVAPTE
C

SEQ ID NO: 5

Heavy chain

QVQLVQSGGGVVQPGTSLRLSCAASGFIFSSSAMHWVRQAPGKGLEWVAVISY
DGSNKYYVDSVKGRFTISRDNSKNTLYLQMNSLRAEDTAVYYCARSARLYASF
DYWGQGALVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGK
EYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFS
CSVMHEALHNHYTQKSLSLSPGK

SEQ ID NO: 6

Example 1-21 ADC-1

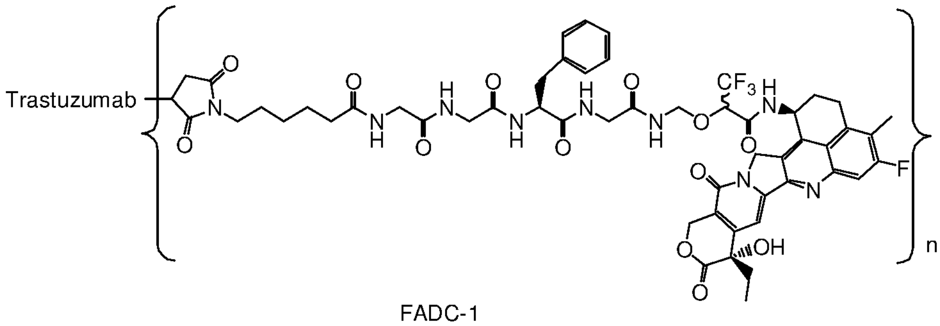

FADC-1

An aqueous solution of tris(2-carboxyethyl)phosphine (10 mM, 0.082 mL, 0.82 μmol) was added to an aqueous PBS buffer (0.05 M aqueous PBS buffer solution with pH = 6.5; 2.5 mL, 9.96 mg/mL, 0.168 μmol) of an antibody trastuzumab at 37 °C, and the reaction mixture was placed in a water bath shaker to react at 37 °C for 3 h with shaking, and the reaction was stopped; the reaction solution was cooled to 25 °C in a water bath, and diluted to 5.0 mg/mL, and 2.0 mL of the resulting solution was taken out and reacted.

Compound **10**-compound with shorter retention time (2.1 mg, 2.02 μmol) was dissolved in 0.10 mL of DMSO, and the reaction mixture was added to the above 2.0 mL solution, placed in a water bath shaker, and reacted at 25 °C for 3 h with shaking, and the reaction was stopped. The reaction solution was purified by desalting on a Sephadex G25

gel column (elution phase: 0.05 M PBS buffer solution at pH 6.5, containing 0.001 M EDTA) to give a PBS buffer (5.0 mg/mL, 1.1 mL) of the exemplary product **ADC-1** of general formula FADC-1, and the buffer was stored at 4 °C.
Mean calculated by UV-HPLC: n = 5.09.

Example 1-22 ADC-2

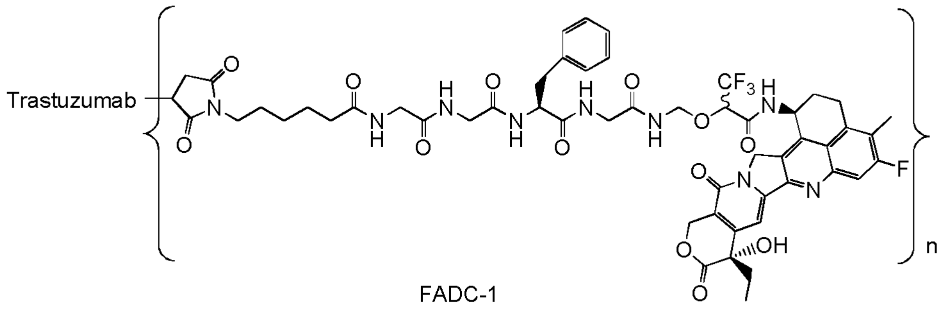

FADC-1

An aqueous solution of tris(2-carboxyethyl)phosphine (10 mM, 0.082 mL, 0.82 μmol) was added to an aqueous PBS buffer (0.05 M aqueous PBS buffer solution with pH = 6.5; 2.5 mL, 9.96 mg/mL, 0.168 μmol) of an antibody trastuzumab at 37 °C, and the reaction mixture was placed in a water bath shaker to react at 37 °C for 3 h with shaking, and the reaction was stopped; the reaction solution was cooled to 25 °C in a water bath, and diluted to 5.0 mg/mL, and 2.0 mL of the resulting solution was taken out and reacted.
Compound **10**-compound with longer retention time (2.1 mg, 2.02 μmol) was dissolved in 0.10 mL of DMSO, and the reaction mixture was added to the above 2.0 mL solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (4.95 mg/mL, 1.1 mL) of the exemplary product **ADC-2** of general formula FADC-1, and the buffer was stored at 4 °C.
Mean calculated by UV-HPLC: n = 7.39.

Example 1-23 ADC-3

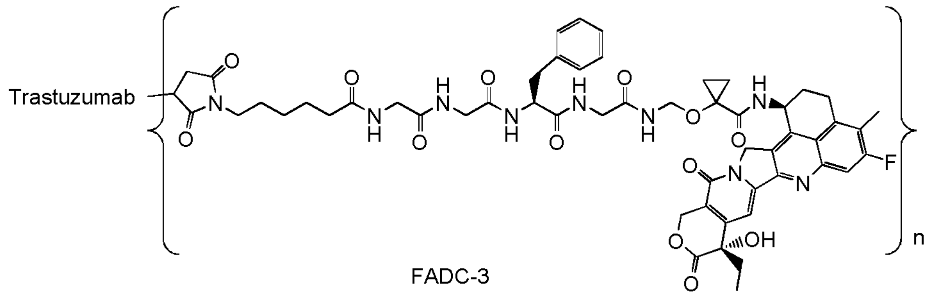

FADC-3

An aqueous solution of tris(2-carboxyethyl)phosphine (10 mM, 0.082 mL, 0.82 μmol) was added to an aqueous PBS buffer (0.05 M aqueous PBS buffer solution with pH = 6.5; 2.5 mL, 9.96 mg/mL, 0.168 μmol) of an antibody trastuzumab at 37 °C, and the reaction mixture was placed in a water bath shaker to react at 37 °C for 3 h with shaking, and the reaction was stopped; the reaction solution was cooled to 25 °C in a water bath, and diluted to 5.0 mg/mL, and 2.0 mL of the resulting solution was taken out and reacted.
Compound **8** (2.1 mg, 2.02 μmol) was dissolved in 0.10 mL of DMSO, and the reaction mixture was added to the above 2.0 mL solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (5.24 mg/mL, 1.1 mL) of the exemplary product **ADC-3** of general formula FADC-3, and the buffer was stored at 4 °C.
Mean calculated by UV-HPLC: n = 7.36.

Example 1-24 ADC-4

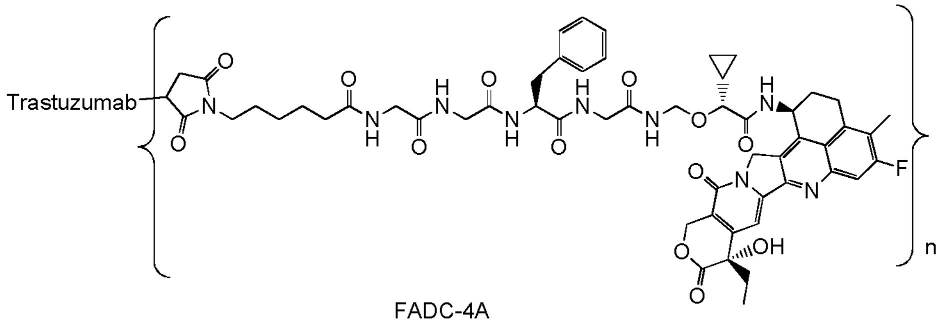

FADC-4A

An aqueous solution of tris(2-carboxyethyl)phosphine (10 mM, 0.173 mL, 1.73 μmol) was added to an aqueous PBS buffer (0.05 M aqueous PBS buffer solution with pH = 6.5; 3.74 mL, 13.38 mg/mL, 0.338 μmol) of an antibody trastuzumab at 37 °C, and the reaction mixture was placed in a water bath shaker to react at 37 °C for 3 h with shaking, and the reaction was stopped; the reaction solution was cooled to 25 °C in a water bath, and diluted to 6.7 mg/mL, and 1.3 mL of the resulting solution was taken out and reacted.

Compound **9**-compound 9-A with shorter retention time (1.0 mg, 0.93 μmol) was dissolved in 0.10 mL of DMSO, and the reaction mixture was added to the above 1.3 mL solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (1.72 mg/mL, 2.36 mL) of the exemplary product **ADC-4** of general formula FADC-4A, and the buffer was stored at 4 °C.

Mean calculated by UV-HPLC: n = 7.39.

Example 1-25 ADC-5

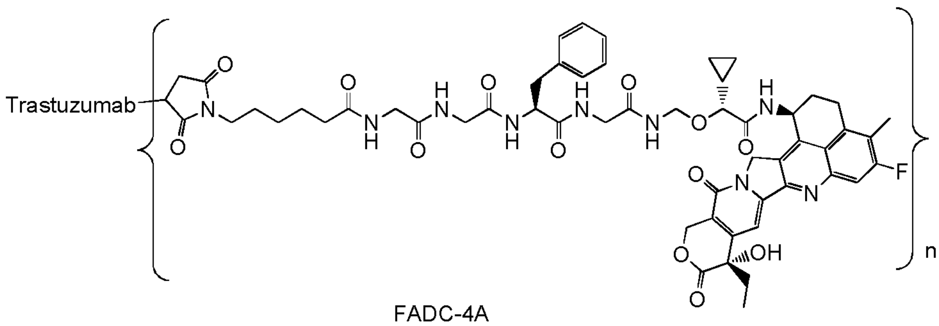

FADC-4A

An aqueous solution of tris(2-carboxyethyl)phosphine (10 mM, 0.067 mL, 0.67 μmol) was added to an aqueous PBS buffer (0.05 M aqueous PBS buffer solution with pH = 6.5; 3.0 mL, 6.70 mg/mL, 0.136 μmol) of an antibody trastuzumab at 37 °C, and the reaction mixture was placed in a water bath shaker to react at 37 °C for 3 h with shaking, and the reaction was stopped; the reaction solution was cooled to 25 °C in a water bath, and 0.614 mL of the resulting solution was taken out and reacted. Compound **9**-compound 9-A with shorter retention time (0.5 mg, 0.42 μmol) was dissolved in 0.031 mL of DMSO, and the reaction mixture was added to the above 0.614 mL solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (3.08 mg/mL, 0.82 mL) of the exemplary product **ADC-5** of general formula FADC-4A, and the buffer was stored at 4 °C.

Mean calculated by UV-HPLC: n = 3.16.

Example 1-26 ADC-6

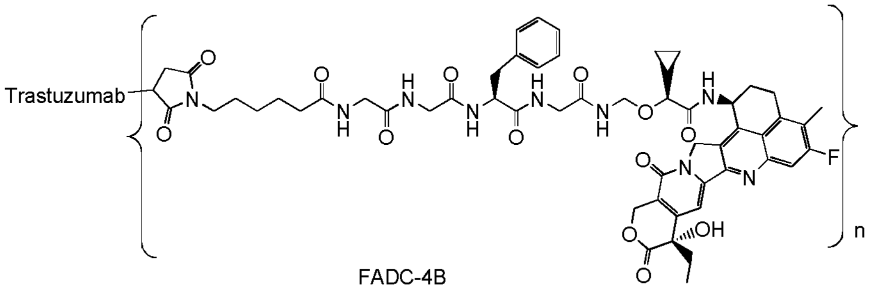

FADC-4B

An aqueous solution of tris(2-carboxyethyl)phosphine (10 mM, 0.173 mL, 1.73 μmol) was added to an aqueous PBS buffer (0.05 M aqueous PBS buffer solution with pH = 6.5; 3.74 mL, 13.38 mg/mL, 0.338 μmol) of an antibody trastuzumab at 37 °C, and the reaction mixture was placed in a water bath shaker to react at 37 °C for 3 h with shaking, and the reaction was stopped; the reaction solution was cooled to 25 °C in a water bath, and diluted to 6.7 mg/mL, and 0.75 mL of the resulting solution was taken out and reacted.
Compound **9**-compound 9-B with longer retention time (0.68 mg, 0.63 μmol) was dissolved in 0.10 mL of DMSO, and the reaction mixture was added to the above 0.75 mL solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (1.78 mg/mL, 1.78 mL) of the exemplary product **ADC-6** of general formula FADC-4A, and the buffer was stored at 4 °C.
Mean calculated by UV-HPLC: n = 3.94.

Example 1-27 ADC-7

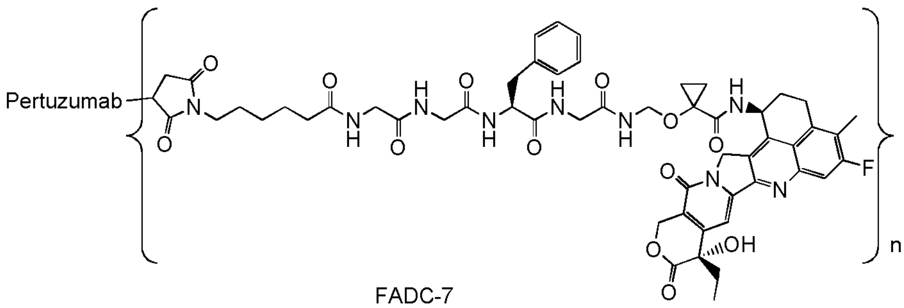

FADC-7

An aqueous solution of tris(2-carboxyethyl)phosphine (10 mM, 0.173 mL, 1.73 μmol) was added to an aqueous PBS buffer (0.05 M aqueous PBS buffer solution with pH = 6.5; 5.0 mL, 10 mg/mL, 0.338 μmol) of an antibody pertuzumab at 37 °C, and the reaction mixture was placed in a water bath shaker to react at 37 °C for 3 h with shaking, and the reaction was stopped; the reaction solution was cooled to 25 °C in a water bath, and diluted to 5.0 mg/mL, and 1.0 mL of the resulting solution was taken out and reacted.
Compound **8** (0.65 mg, 0.6 μmol) was dissolved in 0.1 mL of DMSO, and the reaction mixture was added to the above 1.0 mL solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (1.42 mg/mL, 2.15 mL) of the exemplary product **ADC-7** of general formula FADC-7, and the buffer was stored at 4 °C.
Mean calculated by UV-HPLC: n = 6.91.

Example 1-28 ADC-8

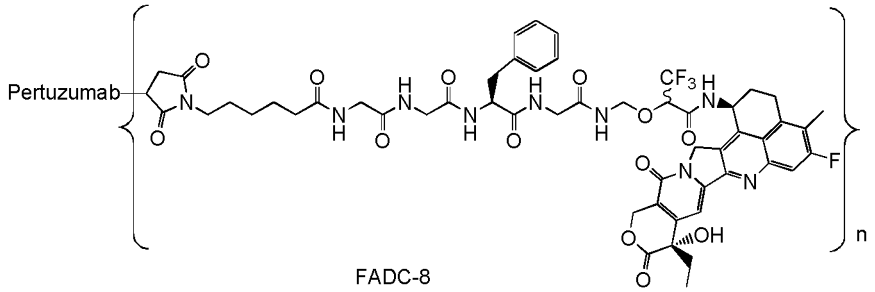

FADC-8

An aqueous solution of tris(2-carboxyethyl)phosphine (10 mM, 0.173 mL, 1.73 μmol) was added to an aqueous PBS buffer (0.05 M aqueous PBS buffer solution with pH = 6.5; 5.0 mL, 10 mg/mL, 0.338 μmol) of an antibody pertuzumab at 37 °C, and the reaction mixture was placed in a water bath shaker to react at 37 °C for 3 h with shaking, and the reaction was stopped; the reaction solution was cooled to 25 °C in a water bath, and diluted to 5.0 mg/mL, and 1.6 mL of the resulting solution was taken out and reacted.

Compound **10**-compound with shorter retention time (1.04 mg, 1.0 μmol) was dissolved in 0.1 mL of DMSO, and the reaction mixture was added to the above 1.6 mL solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (2.14 mg/mL, 2.31 mL) of the exemplary product **ADC-8** of general formula FADC-8, and the buffer was stored at 4 °C.

Mean calculated by UV-HPLC: n = 6.58.

Example 1-29 ADC-9

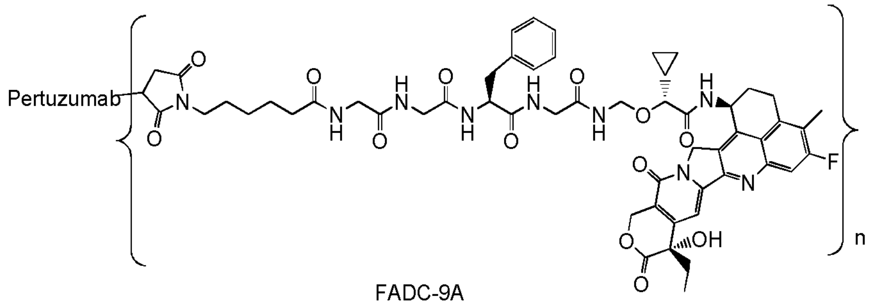

FADC-9A

An aqueous solution of tris(2-carboxyethyl)phosphine (10 mM, 0.173 mL, 1.73 μmol) was added to an aqueous PBS buffer (0.05 M aqueous PBS buffer solution with pH = 6.5; 5.0 mL, 10 mg/mL, 0.338 μmol) of an antibody pertuzumab at 37 °C, and the reaction mixture was placed in a water bath shaker to react at 37 °C for 3 h with shaking, and the reaction was stopped; the reaction solution was cooled to 25 °C in a water bath, and diluted to 5.0 mg/mL, and 0.8 mL of the resulting solution was taken out and reacted.

Compound **9**-compound 9-A with shorter retention time (0.55 mg, 0.5 μmol) was dissolved in 0.1 mL of DMSO, and the reaction mixture was added to the above 0.8 mL solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (2.27 mg/mL, 1.11 mL) of the exemplary product **ADC-9** of general formula FADC-9A, and the buffer was stored at 4 °C.

Mean calculated by UV-HPLC: n = 3.16.

Example 1-30 ADC-10

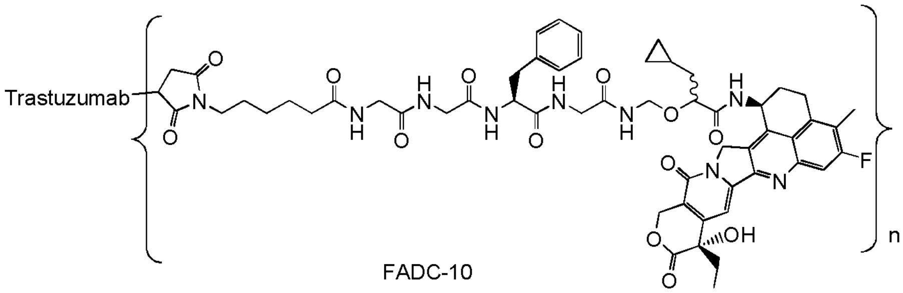

FADC-10

An aqueous solution of tris(2-carboxyethyl)phosphine (TCEP) (10 mM, 19.76 μL, 197.6 nmol) was added to an aqueous PBS buffer (0.05 M aqueous PBS buffer at pH 6.5; 10.0 mg/mL, 0.574 mL, 38.78 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath.

Compound **14**-compound with shorter retention time (0.64 mg, 588 nmol) was dissolved in 40 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (5.48 mg/mL, 1.03 mL) of the exemplary product **ADC-10** of general formula FADC-10, and the buffer was stored at 4 °C.

Mean calculated by UV-Vis: n = 6.25.

Example 1-31 ADC-11

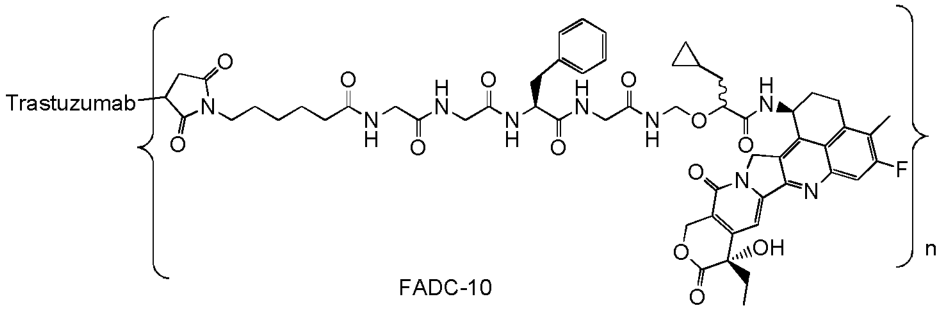

FADC-10

An aqueous solution of tris(2-carboxyethyl)phosphine (TCEP) (10 mM, 22.24 μL, 222.4 nmol) was added to an aqueous PBS buffer (0.05 M aqueous PBS buffer at pH 6.5; 10.0 mg/mL, 0.646 mL, 43.64 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath.

Compound **14**-compound with longer retention time (0.72 mg, 662 nmol) was dissolved in 40 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (2.13 mg/mL, 1.87 mL) of the exemplary product **ADC-11** of general formula FADC-10, and the buffer was stored at 4 °C.

Mean calculated by UV-Vis: n = 7.03.

Example 1-32 ADC-12

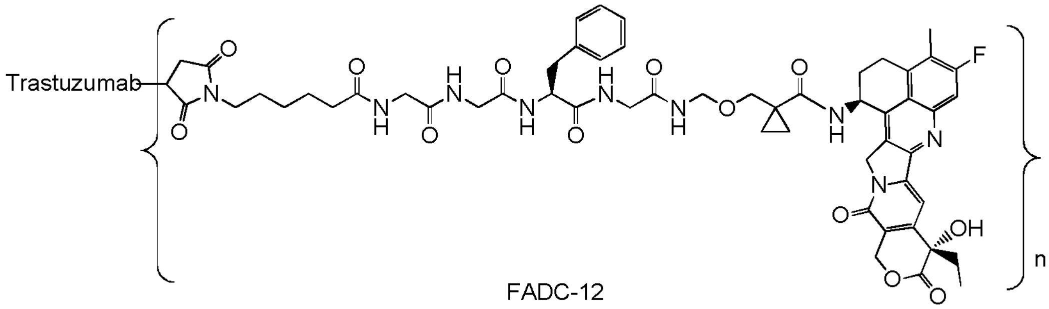

FADC-12

An aqueous solution of tris(2-carboxyethyl)phosphine (TCEP) (10 mM, 25.0 μL, 250.0 nmol) was added to an aqueous PBS buffer (0.05 M aqueous PBS buffer at pH 6.5; 10.0 mg/mL, 0.726 mL, 49.05 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath. Compound **15** (0.81 mg, 754 nmol) was dissolved in 40 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (3.34 mg/mL, 1.45 mL) of the exemplary product **ADC-12** of general formula FADC-12, and the buffer was stored at 4 °C.

Mean calculated by UV-Vis: n = 6.93.

Example 1-33 ADC-13

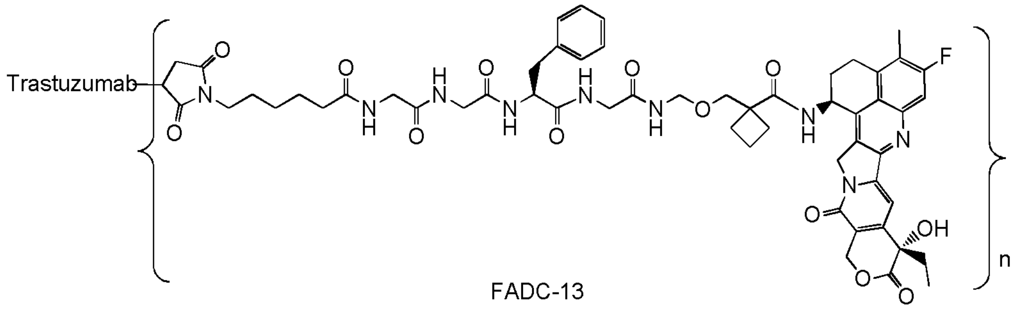

FADC-13

An aqueous solution of tris(2-carboxyethyl)phosphine (TCEP) (10 mM, 9.88 μL, 98.8 nmol) was added to an aqueous PBS buffer (0.05 M aqueous PBS buffer at pH 6.5; 10.0 mg/mL, 0.287 mL, 19.39 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath. Compound **16** (0.32 mg, 294 nmol) was dissolved in 20 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (2.37 mg/mL, 0.88 mL) of the exemplary product **ADC-13** of general formula FADC-13, and the buffer was stored at 4 °C.

Mean calculated by UV-Vis: n = 6.53.

Example 1-34 ADC-14

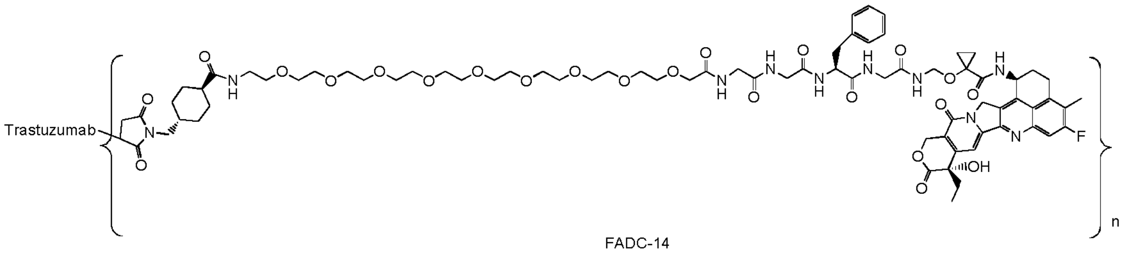

FADC-14

An aqueous solution of tris(2-carboxyethyl)phosphine (TCEP) (10 mM, 20.38 µL, 203.8 nmol) was added to an aqueous PBS buffer (0.05 M aqueous PBS buffer at pH 6.5; 10.0 mg/mL, 0.592 mL, 40.0 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath.

Compound **17** (0.92 mg, 598 nmol) was dissolved in 40 µL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (0.30 mg/mL, 12.0 mL) of the exemplary product **ADC-14** of general formula FADC-14, and the buffer was stored at 4 °C.

Mean calculated by UV-Vis: n = 7.61.

Example 1-35 ADC-15

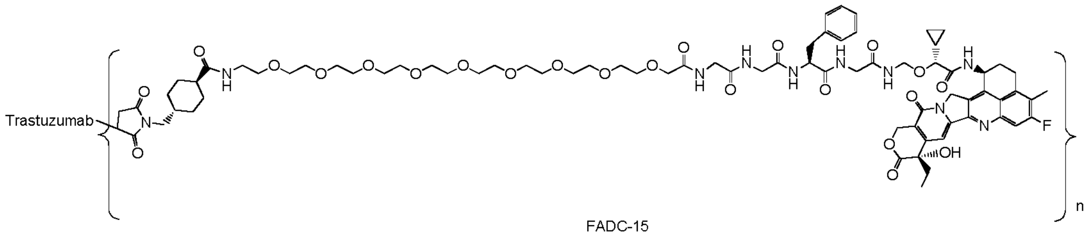

FADC-15

An aqueous solution of tris(2-carboxyethyl)phosphine (TCEP) (10 mM, 20.38 µL, 203.8 nmol) was added to an aqueous PBS buffer (0.05 M aqueous PBS buffer at pH 6.5; 10.0 mg/mL, 0.592 mL, 40.0 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath.

Compound **18** (0.93 mg, 599 nmol) was dissolved in 40 µL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (0.32 mg/mL, 11.8 mL) of the exemplary product **ADC-15** of general formula FADC-15, and the buffer was stored at 4 °C.

Mean calculated by UV-Vis: n = 7.89.

Example 1-36 ADC-16

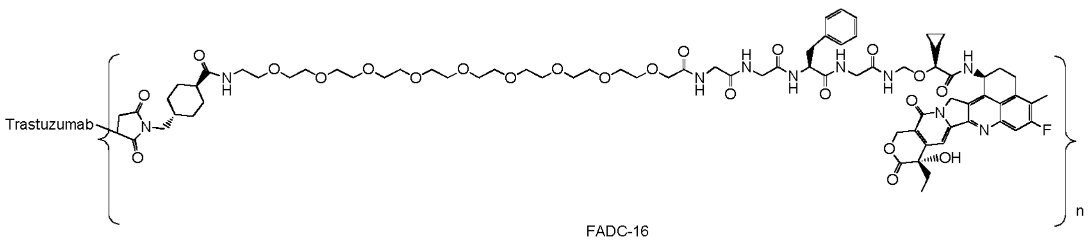

FADC-16

An aqueous solution of tris(2-carboxyethyl)phosphine (TCEP) (10 mM, 18.25 μL, 182.5 nmol) was added to an aqueous PBS buffer (0.05 M aqueous PBS buffer at pH 6.5; 10.0 mg/mL, 0.53 mL, 35.8 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath.

Compound **19** (0.83 mg, 534 nmol) was dissolved in 35 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (0.32 mg/mL, 12.0 mL) of the exemplary product **ADC-16** of general formula FADC-16, and the buffer was stored at 4 °C.

Mean calculated by UV-Vis: n = 7.43.

Example 1-37 ADC-17

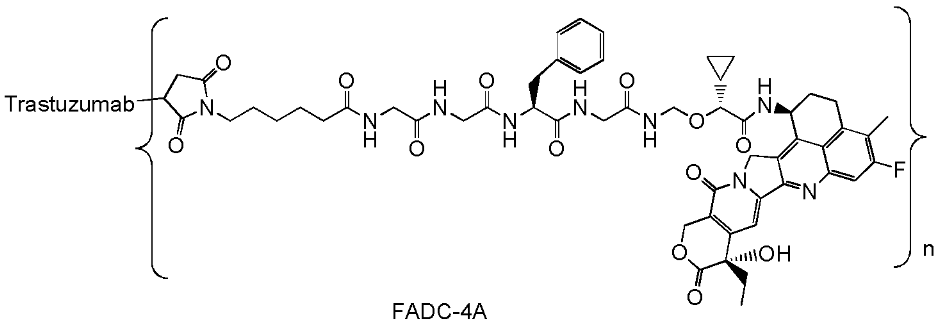

FADC-4A

An aqueous solution of tris(2-carboxyethyl)phosphine (TCEP) (10 mM, 43.2 μL, 432 nmol) was added to an aqueous PBS buffer (0.05 M aqueous PBS buffer at pH 6.5; 10.0 mg/mL, 2.0 mL, 135.12 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath. Compound **9**-compound **9-A** with shorter retention time (2.22 mg, 2067 nmol) was dissolved in 175 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (1.32 mg/mL, 12.0 mL) of the exemplary product **ADC-17** of general formula FADC-4A, and the buffer was stored at 4 °C.

Mean calculated by UV-Vis: n = 5.42.

Example 1-38 ADC-18 (reference example)

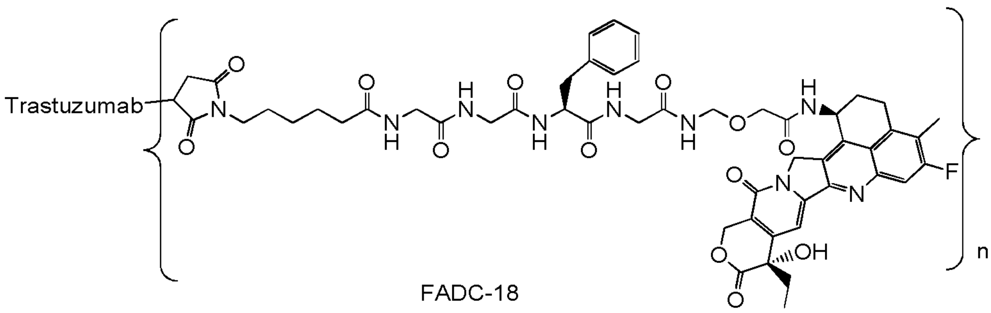

FADC-18

An aqueous solution of tris(2-carboxyethyl)phosphine (TCEP) (10 mM, 51.7 μL, 517 nmol) was added to an aqueous PBS buffer (0.05 M aqueous PBS buffer at pH 6.5; 10.0 mg/mL, 1.5 mL, 101.3 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath. Compound **20** (2.0 mg, 1934 nmol) was dissolved in 100 μL of DMSO, and the reaction mixture was added to the above reaction solution, placed in a water

bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (0.79 mg/mL, 13.0 mL) of the exemplary product **ADC-18** of general formula FADC-18, and the buffer was stored at 4 °C.

Mean calculated by UV-Vis: n = 7.23.

Example 1-39 ADC-19

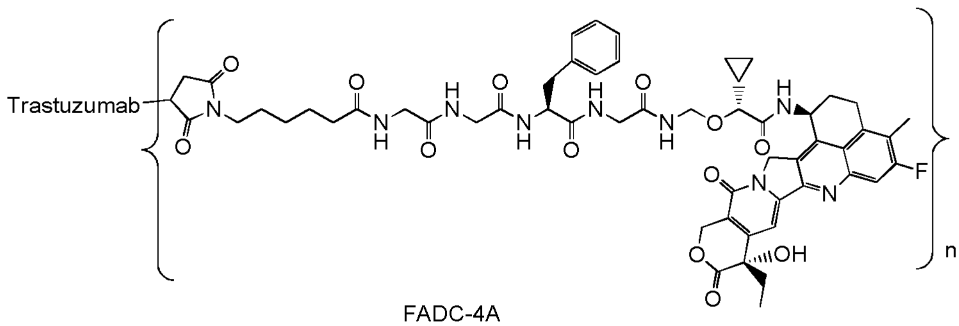

FADC-4A

An aqueous solution of tris(2-carboxyethyl)phosphine (TCEP) (10 mM, 46.9 μL, 469 nmol) was added to an aqueous PBS buffer (0.05 M aqueous PBS buffer at pH 6.5; 10.0 mg/mL, 1.36 mL, 91.9 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath. Compound **9**-compound **9-A** with shorter retention time (2.0 mg, 1862 nmol) was dissolved in 100 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (0.73 mg/mL, 13.0 mL) of the exemplary product **ADC-19** of general formula FADC-4A, and the buffer was stored at 4 °C.

Mean calculated by UV-Vis: n = 6.26.

Example 1-40 ADC-20

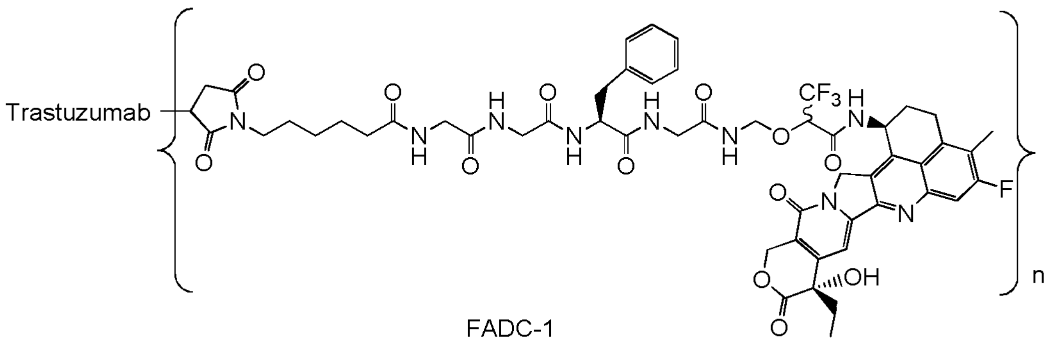

FADC-1

An aqueous solution of tris(2-carboxyethyl)phosphine (TCEP) (10 mM, 51.7 μL, 517 nmol) was added to an aqueous PBS buffer (0.05 M aqueous PBS buffer at pH 6.5; 10.0 mg/mL, 1.5 mL, 101.3 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath. Compound **10**-compound with longer retention time (2.0 mg, 1815 nmol) was dissolved in 100 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (0.73 mg/mL, 13.0 mL) of the exemplary product **ADC-20** of general formula FADC-1, and the buffer was stored at 4 °C.

Mean calculated by UV-Vis: n = 7.43.

Example 1-41 ADC-21 (reference example)

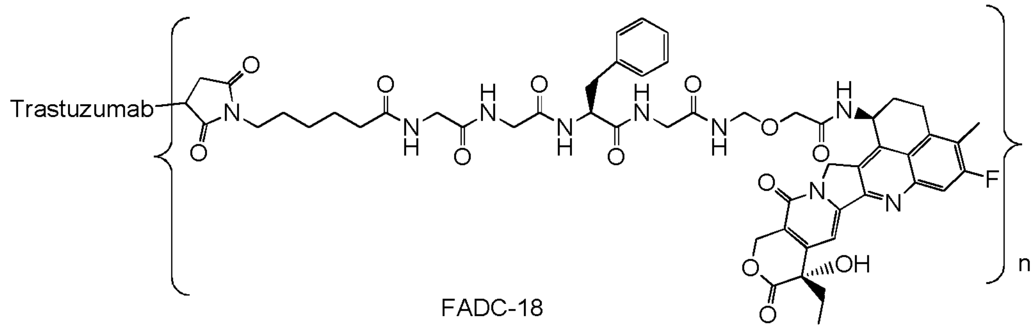

FADC-18

An aqueous solution of tris(2-carboxyethyl)phosphine (TCEP) (10 mM, 63.9 μL, 639 nmol) was added to an aqueous PBS buffer (0.05 M aqueous PBS buffer at pH 6.5; 10.0 mg/mL, 1.86 mL, 125.4 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath. Compound **20** (2.07 mg, 2001 nmol) was dissolved in 150 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (2.91 mg/mL, 4.44 mL) of the exemplary product **ADC-21** of general formula FADC-18, and the buffer was stored at 4 °C.

Mean calculated by UV-Vis: n = 7.23.

Example 1-42 ADC-22

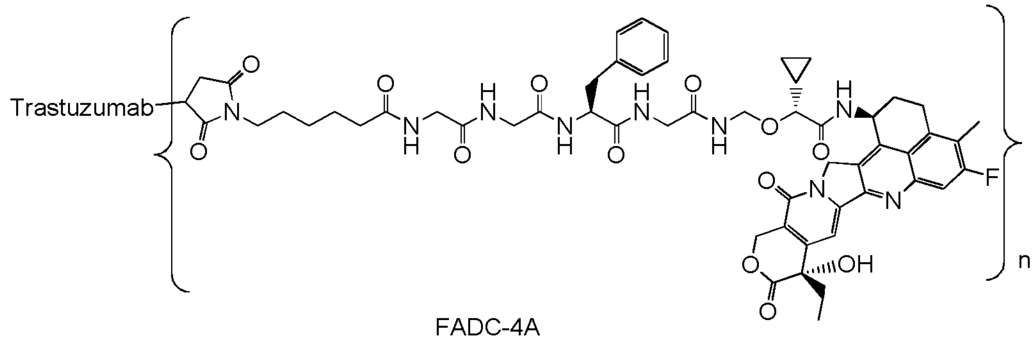

FADC-4A

An aqueous solution of tris(2-carboxyethyl)phosphine (TCEP) (10 mM, 64.9 μL, 649 nmol) was added to an aqueous PBS buffer (0.05 M aqueous PBS buffer at pH 6.5; 10.0 mg/mL, 1.88 mL, 127.2 nmol) of an antibody trastuzumab at 37 °C, and the reaction mixture was placed in a water bath shaker to react at 37 °C for 3 h with shaking, and the reaction was stopped. The reaction solution was cooled to 25 °C in a water bath. Compound **9**-compound **9-A** with shorter retention time (2.1 mg, 1955 nmol) was dissolved in 150 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (3.56 mg/mL, 3.98 mL) of the exemplary product **ADC-22** of general formula FADC-4A, and the buffer was stored at 4 °C.

Mean calculated by UV-Vis: n = 6.79.

Example 1-43 ADC-23 (reference example)

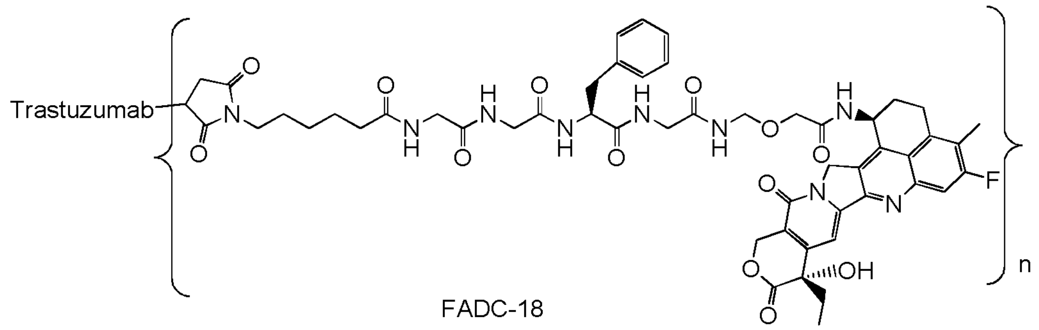

FADC-18

An aqueous solution of tris(2-carboxyethyl)phosphine (TCEP) (10 mM, 11.89 mL, 118.9 μmol) was added to an aqueous PBS buffer (0.05 M aqueous PBS buffer at pH 6.5; 10.0 mg/mL, 345 mL, 23.31 μmol) of an antibody trastuzumab at 37 °C, and the reaction mixture was placed in a water bath shaker to react at 37 °C for 3.5 h with shaking, and the reaction was stopped. The reaction solution was cooled to 25 °C in a water bath.

Compound **20** (362 mg, 350 μmol) was dissolved in 7.12 mL of MeCN and 3.56 mL of DMSO, and the reaction mixture was added to the above reaction solution, placed in a water bath shaker, and reacted at 25 °C for 3 h with shaking, and the reaction was stopped. The reaction solution was purified by desalting through ultrafiltration membranes with 2%(v/v) MeCN and 1%(v/v) DMSO-containing aqueous PBS buffer (0.05 M aqueous PBS buffer at pH = 6.5) and aqueous succinic acid buffer (0.01 M aqueous succinic acid buffer at pH = 5.3) successively, then sucrose was added to 60 mg/mL and tween 20 to 0.2 mg/mL, and the reaction solution was lyophilized to give a lyophilized powder sample of exemplary product **ADC-23** of general formula FADC-18, and the sample was stored at 4 °C.

Mean calculated by UV-Vis: n = 7.05.

Example 1-44 ADC-24

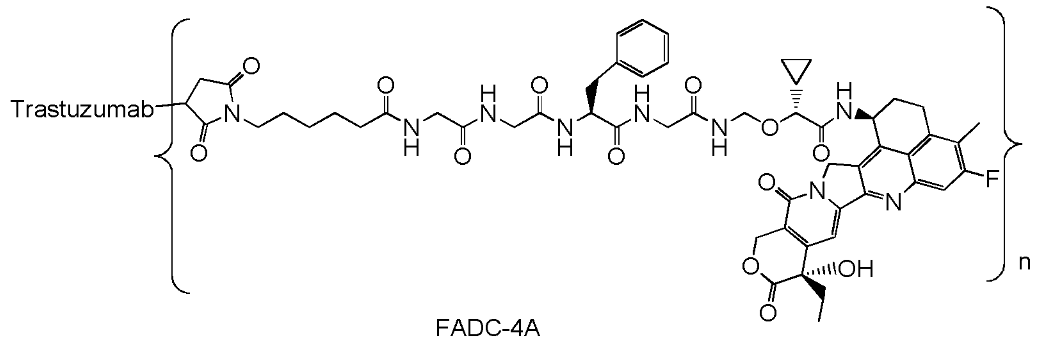

FADC-4A

An aqueous solution of tris(2-carboxyethyl)phosphine (TCEP) (10 mM, 11.44 mL, 114.4 μmol) was added to an aqueous PBS buffer (0.05 M aqueous PBS buffer at pH 6.5; 10.0 mg/mL, 332 mL, 22.43 μmol) of an antibody trastuzumab at 37 °C, and the reaction mixture was placed in a water bath shaker to react at 37 °C for 3.5 h with shaking, and the reaction was stopped. The reaction solution was cooled to 25 °C in a water bath.

Compound **9**-compound **9-A** with shorter retention time (241 mg, 224 μmol) was dissolved in 13.76 mL of MeCN and 6.88 mL of DMSO, and the reaction mixture was added to the above reaction solution, placed in a water bath shaker, and reacted at 25 °C for 3 h with shaking, and the reaction was stopped. The reaction solution was purified by desalting through ultrafiltration membranes with 4%(v/v) MeCN and 2%(v/v) DMSO-containing aqueous PBS buffer (0.05 M aqueous PBS buffer at pH = 6.5) and aqueous succinic acid buffer (0.01 M aqueous succinic acid buffer at pH = 5.3) successively, then sucrose was added to 60 mg/mL and tween 20 to 0.2 mg/mL, and the reaction solution was lyophilized to give a lyophilized powder sample of exemplary product **ADC-24** of general formula FADC-4A, and the sample was stored at 4 °C.

Mean calculated by UV-Vis: n = 7.07.

Example 1-45 ADC-25

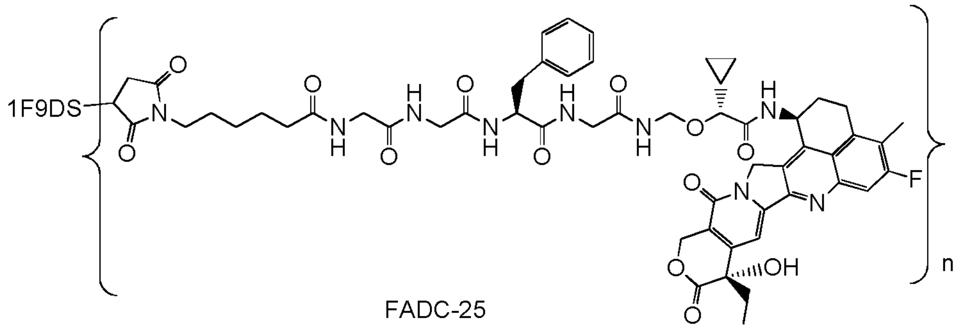

FADC-25

An aqueous solution of tris(2-carboxyethyl)phosphine (TCEP) (10 mM, 73.7 μL, 740 nmol) was added to an aqueous PBS buffer (0.05 M aqueous PBS buffer at pH 6.5; 10.0 mg/mL, 2.14 mL, 144.60 nmol) of an antibody B7H3 antibody 1F9DS at 37 °C, and the reaction mixture was placed in a water bath shaker to react at 37 °C for 3 h with shaking, and the reaction was stopped. The reaction solution was cooled to 25 °C in a water bath.

Compound **9**-compound **9-A** with shorter retention time (3.0 mg, 2793 nmol) was dissolved in 150 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (1.28 mg/mL, 13.0 mL) of the exemplary product **ADC-25** of general formula FADC-25, and the buffer was stored at 4 °C.

Mean calculated by UV-Vis: n = 6.87.

Example 1-46 ADC-26 (reference example)

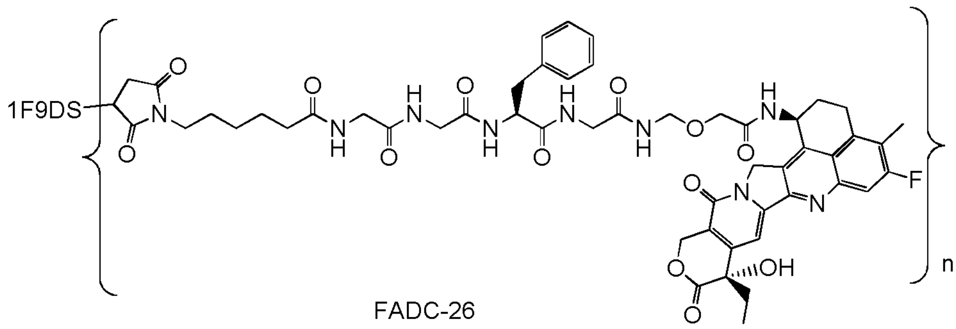

FADC-26

An aqueous solution of tris(2-carboxyethyl)phosphine (TCEP) (10 mM, 30.1 μL, 300 nmol) was added to an aqueous PBS buffer (0.05 M aqueous PBS buffer at pH 6.5; 10.0 mg/mL, 0.89 mL, 60.14 nmol) of an antibody B7H3 antibody 1F9DS at 37 °C, and the reaction mixture was placed in a water bath shaker to react at 37 °C for 3 h with shaking, and the reaction was stopped. The reaction solution was cooled to 25 °C in a water bath.

Compound **20** (1.0 mg, 967 nmol) was dissolved in 100 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (1.61 mg/mL, 4.0 mL) of the exemplary product **ADC-26** of general formula FADC-26, and the buffer was stored at 4 °C.

Mean calculated by UV-Vis: n = 6.15.

Example 1-47 ADC-27

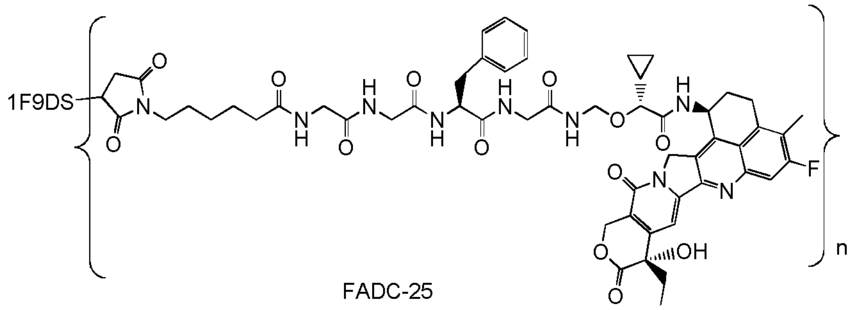

FADC-25

An aqueous solution of tris(2-carboxyethyl)phosphine (TCEP) (10 mM, 30.1 μL, 300 nmol) was added to an aqueous PBS buffer (0.05 M aqueous PBS buffer at pH 6.5; 10.0 mg/mL, 0.89 mL, 60.14 nmol) of an antibody B7H3 antibody 1F9DS at 37 °C, and the reaction mixture was placed in a water bath shaker to react at 37 °C for 3 h with shaking, and the reaction was stopped. The reaction solution was cooled to 25 °C in a water bath.

Compound **9**-compound **9-A** with shorter retention time (1.02 mg, 950 nmol) was dissolved in 100 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (1.94 mg/mL, 3.5 mL) of the exemplary product **ADC-27** of general formula FADC-25, and the buffer was stored at 4 °C.

Mean calculated by UV-Vis: n = 6.11.

Example 1-48 ADC-28 (reference example)

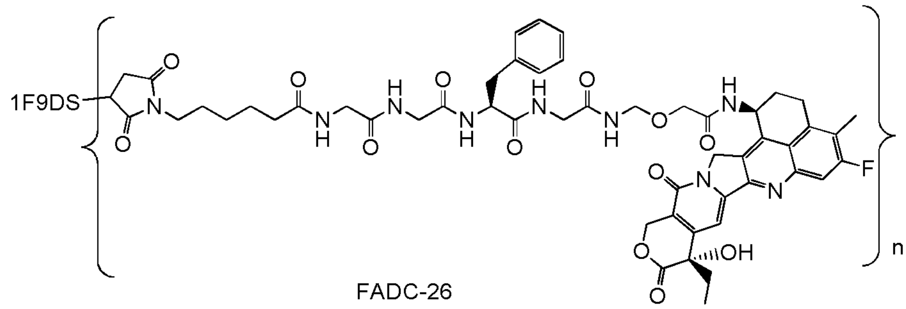

FADC-26

An aqueous solution of tris(2-carboxyethyl)phosphine (TCEP) (10 mM, 81.3 μL, 810 nmol) was added to an aqueous PBS buffer (0.05 M aqueous PBS buffer at pH 6.5; 10.0 mg/mL, 2.36 mL, 159.47nmol) of an antibody B7H3 antibody 1F9DS at 37 °C, and the reaction mixture was placed in a water bath shaker to react at 37 °C for 3 h with shaking, and the reaction was stopped. The reaction solution was cooled to 25 °C in a water bath.

Compound **20** (3.0 mg, 2901 nmol) was dissolved in 150 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (1.29 mg/mL, 13.0 mL) of the exemplary product **ADC-28** of general formula FADC-26, and the buffer was stored at 4 °C.

Mean calculated by UV-Vis: n = 7.46.

Example 1-49 ADC-29

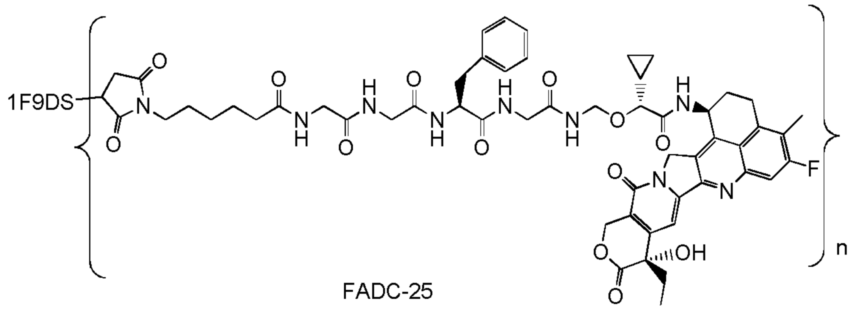

FADC-25

An aqueous solution of tris(2-carboxyethyl)phosphine (TCEP) (10 mM, 28.6 μL, 290 nmol) was added to an aqueous PBS buffer (0.05 M aqueous PBS buffer at pH 6.5; 10.0 mg/mL, 0.80 mL, 50.06 nmol) of an antibody B7H3 antibody 1F9DS at 37 °C, and the reaction mixture was placed in a water bath shaker to react at 37 °C for 3 h with shaking, and the reaction was stopped. The reaction solution was cooled to 25 °C in a water bath.

Compound **9**-compound **9-A** with shorter retention time (1.29 mg, 1201 nmol) was dissolved in 100 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (2.63 mg/mL, 2.4 mL) of the exemplary product **ADC-29** of general formula FADC-25, and the buffer was stored at 4 °C.

Mean calculated by UV-Vis: n = 7.24.

Example 1-50 ADC-30 (reference example)

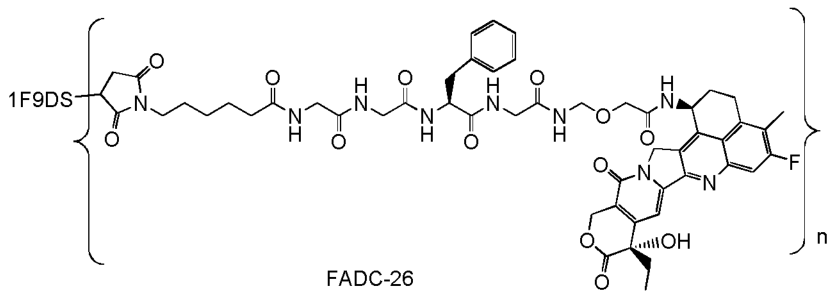

FADC-26

An aqueous solution of tris(2-carboxyethyl)phosphine (TCEP) (10 mM, 29.1 μL, 290 nmol) was added to an aqueous PBS buffer (0.05 M aqueous PBS buffer at pH 6.5; 10.0 mg/mL, 0.86 mL, 58.4nmol) of an antibody B7H3 antibody 1F9DS at 37 °C, and the reaction mixture was placed in a water bath shaker to react at 37 °C for 3 h with shaking, and the reaction was stopped. The reaction solution was cooled to 25 °C in a water bath.

Compound **20** (1.0 mg, 967 nmol) was dissolved in 100 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (1.61 mg/mL, 4.0 mL) of the exemplary product **ADC-30** of general formula FADC-26, and the buffer was stored at 4 °C.

Mean calculated by UV-Vis: n = 6.15.

Example 1-51 ADC-31

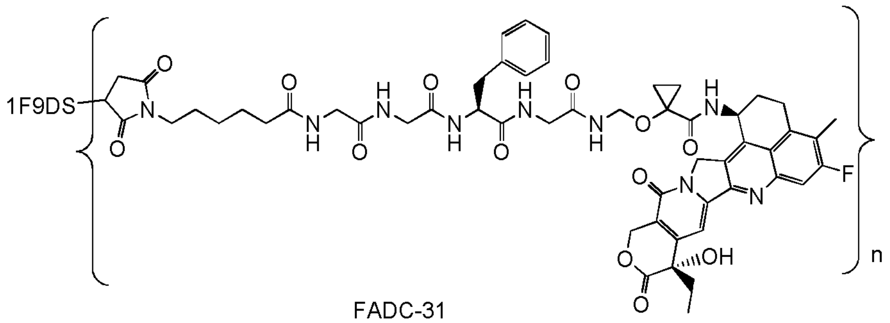

FADC-31

An aqueous solution of tris(2-carboxyethyl)phosphine (TCEP) (10 mM, 30.1 μL, 300 nmol) was added to an aqueous PBS buffer (0.05 M aqueous PBS buffer at pH 6.5; 10.0 mg/mL, 0.89 mL, 60.14 nmol) of an antibody B7H3 antibody 1F9DS at 37 °C, and the reaction mixture was placed in a water bath shaker to react at 37 °C for 3 h with shaking, and the reaction was stopped. The reaction solution was cooled to 25 °C in a water bath.

Compound **8** (1.0 mg, 943 nmol) was dissolved in 100 μL of DMSO, and the reaction mixture was added to the above reaction solution, placed in a water bath shaker, and reacted at 25 °C for 3 h with shaking, and the reaction was stopped. The reaction solution was purified by desalting on a Sephadex G25 gel column (elution phase: 0.05 M PBS buffer solution at pH 6.5, containing 0.001 M EDTA) to give a PBS buffer (1.47 mg/mL, 4.5 mL) of the exemplary product **ADC-31** of general formula FADC-31, and the buffer was stored at 4 °C.

Mean calculated by UV-Vis: n = 6.33.

Drug loading analysis of ADC stock solution

Purpose and principle of experiment

ADC stock solution is an antibody cross-linked drug, and the mechanism of treating diseases thereof is to transport toxin molecules into cells depending on the targeting performance of the antibody so as to kill the cells. The drug loading plays a decisive role in the drug efficacy. The drug loading of the ADC stock solution was determined using the UV method.

Experimental procedures

Cuvettes containing sodium succinate buffer were placed into the reference cell and sample cell, and the absorbance of the solvent blank was subtracted. Then, a cuvette containing test solution was placed into the sample cell, and the absorbances at 280 nm and 370 nm were determined.

Calculation for results: the loading capacity of the ADC stock solution was determined by ultraviolet spectrophotometry (instrument: Thermo nanodrop2000 ultraviolet spectrophotometer), based on the principle that the total absorbance of the ADC stock solution at a certain wavelength was the sum of the absorbance values of the cytotoxic drug and the monoclonal antibody at that wavelength, namely:

$$(1)\ A_{280\,nm} = \varepsilon_{mab\text{-}280} b C_{mab} + \varepsilon_{Drug\text{-}280} b C_{Drug}$$

$\varepsilon_{Drug\text{-}280}$: the mean molar extinction coefficient of the drug at 280 nm was 5,100;
$C_{Drug}$: the concentration of the drug;
$\varepsilon_{mab\text{-}280}$: the mean molar extinction coefficient of the monoclonal antibody stock solution of trastuzumab or pertuzumab at 280 nm is 214,600;
$C_{mab}$: the concentration of the monoclonal antibody stock solution of trastuzumab or pertuzumab;
b: the optical path length is 1 cm.

Similarly, an equation for the total absorbance of the sample at 370 nm can be given as:

$$(2)\ A_{370\,nm} = \varepsilon_{mab\text{-}370} b C_{mab} + \varepsilon_{Drug\text{-}370} b C_{Drug}$$

$\varepsilon_{Drug\text{-}370}$: the mean molar extinction coefficient of the drug at 370 nm was 19,000;

$C_{Drug}$: the concentration of the drug;
$\varepsilon_{mab-370}$: the attenuation coefficient of the monoclonal antibody stock solution of trastuzumab or pertuzumab at 370 nm was 0;
$C_{mab}$: the concentration of the monoclonal antibody stock solution of trastuzumab;
b: the optical path length is 1 cm.

The drug loading can be calculated using both equations (1) and (2) as well as the attenuation coefficients of the monoclonal antibody and the drug at both wavelengths and their concentrations.

$$\text{Drug loading} = C_{Drug}/C_{mab}.$$

Biological Evaluation

Test Example 1-1: Test for Inhibition of *In Vitro* Proliferation of Tumor Cells by Compounds Disclosed Herein

I. Purpose

This experiment was intended to detect the inhibitory activity of the pharmaceutical compounds of the present disclosure against the *in vitro* proliferation of U87MG cells (Cell Bank, Chinese Academy of Sciences, Catalog # TCHu138) and SK-BR-3 tumor cells (human breast cancer cells, ATCC, Cat # HTB-30). The cells were treated *in vitro* with a compound at different concentrations. After 6 days of culture, the proliferation of cells was tested using CTG (CellTiter-Glo® Luminescent Cell Viability Assay, Promega, Cat # G7573) reagents, and the *in vitro* activity of the compound was evaluated according to $IC_{50}$ value.

II. Method

The method for testing the inhibition of the *in vitro* proliferation of U87MG cells was described below as an example for the method for assaying for the inhibitory activity of the compounds of the present disclosure against the *in vitro* proliferation of tumor cells. The method was also applicable to, but not limited to, the test for the inhibitory activity against the *in vitro* proliferation of other tumor cells.

1. Cell culture: U87MG and SK-BR-3 cells were cultured in EMEM medium (GE, Cat # SH30024.01) containing 10% FBS and McCoy's 5A medium (Gibco, Cat # 16600-108) containing 10% FBS, respectively.
2. Cell preparation: U87MG and SK-BR-3 cells growing at log phase were washed once with PBS (phosphate buffer, Shanghai BasalMedia Technologies Co., Ltd.) and then digested with 2-3 mL of trypsin (0.25% Trypsin-EDTA (1×), Gibico, Life Technologies) for 2-3 min. After the cells were completely digested, 10-15 mL of cell culture media were added to elute the digested cells. The mixtures were centrifuged at 1000 rpm for 5 min, and the supernatants were discarded. Then the cells were resuspended in 10-20 mL of cell culture media to give single-cell suspensions.
3. Cell plating: the U87MG and SK-BR-3 single-cell suspensions were each well mixed and adjusted with cell culture media to cell densities of $2.75 \times 10^3$ cells/mL and $8.25 \times 10^3$ cells/mL, respectively. The adjusted cell suspensions were each well mixed and added to 96-well cell culture plates at 180 μL/well. To each of the peripheral wells of the 96-well plates was added 200 μL of media only. The plate was incubated in an incubator for 24 h (37 °C, 5% $CO_2$).
4. Compound preparation: the compound was dissolved in DMSO (dimethyl sulfoxide, Shanghai Titan Scientific Co., Ltd.) to prepare a stock solution at an initial concentration of 10 mM.

Small molecule compounds were prepared at an initial concentration of 500 nM as follows.
Different test samples at 100 μM (30 μL) were added to the first column of a 96-well U-bottom plate, and 20 μL of DMSO was added to each well of the second column through the eleventh column. The samples in the first column (10 μL) were added to the 20 μL of DMSO in the second column, and the mixtures were well mixed. The mixtures (10 μL) were added to the third column, and so on to the tenth column. The drugs in the plate (5 μL per well) were transferred to EMEM media (95 μL), and the mixtures were well mixed for later use.
ADCs were prepared at an initial concentration of 10 nM or 500 nM as follows.
Different test samples at concentration 100 nM or 5 μM (100 μL) were added to the first column of a 96-well plate, and 100 μL of PBS was added to each well of the second column through the eleventh column. The samples in the first column (50 μL) were added to the 100 μL of PBS in the second column, and the mixtures were well mixed. The mixtures (50 μL) were added to the third column, and so on, by 3-fold dilution, to the tenth column.
5. Sample adding: the test samples prepared at different concentrations (20 μL) were added to the culture

plate, with two duplicate wells set for each sample. The plate was incubated in an incubator for 6 days (37 °C, 5% $CO_2$).

6. Color developing: the 96-well cell culture plate was taken out, and 90 μL of CTG solution was added to each well, followed by 10 min of incubation at room temperature.

7. Plate reading: the 96-well cell culture plate was taken out and tested in a microplate reader (BMG labtech, PHERAstar FS) for chemiluminescence.

III. Data analysis

Data were processed and analyzed using Microsoft Excel and Graphpad Prism 5. The experimental results are shown in Table 1 below.

Table 1. $IC_{50}$ values of the small molecule fragments of the present disclosure in inhibiting *in vitro* proliferation of SK-BR-3 cells and U87 cells

| Compound No. | $IC_{50}$ (nM) | |
|---|---|---|
| | SK-BR-3 | U87 |
| 1 | 0.12 | 0.23 |
| 2-shorter retention time 2-B | 0.33 | 0.86 |
| 2-longer retention time 2-A | 8.11 | 2.31 |
| 3-shorter retention time | 0.36 | 0.83 |
| 3-longer retention time | 1.67 | 2.98 |
| 4 | 1.9 | / |
| 5 | / | 4.81 |
| 6 | / | 1.83 |
| 7 | / | 1.95 |

Conclusion: The small molecular fragments of the present disclosure have significant inhibitory activity against the proliferation of SK-BR-3 cells and U87 cells, and the chiral centers have certain influence on the inhibitory activity of the compounds.

Test Example 1-2: Test for Inhibition of *In Vitro* Proliferation of HER 2-targeted Tumor Cells by Antibody Drug Conjugates of the Present Disclosure

This experiment was intended to detect the inhibitory activity of the antibody drug conjugates aiming at HER2 targets of the present disclosure against the *in vitro* proliferation of SK-BR-3 (human breast cancer cells, ATCC, Cat # HTB-30) and MDA-MB-468 (human breast cancer cells, ATCC, Cat # HTB-132). The cells were treated *in vitro* with a compound at different concentrations. After 6 days of culture, the proliferation of cells was tested using CTG reagents, and the *in vitro* activity of the compound was evaluated according to $IC_{50}$ value.

According to the test method of Test Example 1, the test cells were SK-BR-3 and MDA-MB-468, and the cell culture media were 10% FBS-containing McCoy's 5A medium (Gibco, Cat # 16600-108), 10% FBS-containing EMEM medium (GE, Cat # SH 30024.01), and 10% FBS-containing L-15 medium (ThermoFisher, Cat # 11415-114), respectively. The cell densities of the three strains of cells were adjusted to $8.33 \times 10^3$ cells/mL, $8.33 \times 10^3$ cells/mL and $1.39 \times 10^4$ cells/mL with cell culture media, respectively, and the cell suspensions after the density adjustment were well mixed and added to 96-well cell culture plates at 180 μL/well. The results of the tests on the related compounds are shown in Table 2 below.

Table 2. $IC_{50}$ values for inhibition of *in vitro* proliferation of HER2-targeted tumor cells by antibody drug conjugates of the present disclosure

| Compound No. | $IC_{50}$ (nM) | |
|---|---|---|
| | SK-BR-3 | MDA-MB-468 |
| ADC-3 | 0.43 | >50 |

(continued)

| Compound No. | $IC_{50}$ (nM) | |
|---|---|---|
| | SK-BR-3 | MDA-MB-468 |
| ADC-4 | 0.30 | >50 |
| ADC-6 | 0.48 | >50 |
| ADC-7 | 0.14 | >50 |
| ADC-9 | 0.95 | >50 |
| ADC-10 | 1.36 | >50 |
| ADC-11 | 0.73 | >50 |
| ADC-12 | 0.82 | >50 |
| ADC-13 | 0.47 | >50 |
| ADC-14 | 0.53 | >50 |
| ADC-15 | 0.38 | >50 |
| ADC-16 | 0.49 | >50 |
| ADC-17 | 0.37 | >50 |

Conclusion: the antibody drug conjugates aiming at HER2 targets of the present disclosure have significant proliferation inhibition activity on HER2 positive cells SK-BR-3; meanwhile, the proliferation inhibition activity of the compounds on HER2 negative cells MDA-MB-468 is weak; the compounds have good selectivity.

Test Example 1-3: Plasma Stability Test for Her2-ADC

Samples ADC-19, ADC-18 and ADC-20, human plasma, monkey plasma ( Shanghai Medicilon Inc.) and 1% BSA (Sigma) PBS solution (Sangon Biotech (Shanghai)) were each filtered through a 0.22 μm filter for sterilization. ADC-19, ADC-18 and ADC-20 were added to the sterile plasma or a solution of 1% BSA in PBS, respectively, at a final concentration of 200 μg/mL, and the reaction solution was incubated in an incubator at 37 °C; the day of incubation was noted as day 0, and samples were taken out on days 7, 14 and 21, respectively, for detection of free toxin.

25 μL of samples were taken out to a 96-well plate; 50 μL of internal standard working solution (100 ng/mL camptothecine acetonitrile solution) and 150 μL of acetonitrile were added; the mixture was vortexed for 5 min and centrifuged for 10 min (4000 rpm), and 5 μL of the resulting samples were used for LC/MS/MS (Applied Biosystems, USA) analysis.

The results showed that: ADC-19 was fairly stable in both human and monkey plasma, as well as a solution of 1% BSA in PBS, with a release rate of free toxin of no more than 2.1% at the highest, and tended to be stable on day 14, see FIG. 1A.

ADC-18 was poorly stable in human and monkey plasma with release rates of free toxin of 14.5% and 8.10% at the highest, respectively. It was relatively stable in the solution of 1% BSA in PBS, see FIG. 1B.

ADC-20 was poorly stable in human plasma, monkey plasma, and a solution of 1% BSA in PBS, with release rates of free toxin of 21.7%, 29.7% and 21.7% at the highest, respectively. It was in a degraded state in the solution of 1% BSA in PBS, see FIG. 1C.

Test Example 1-4: Evaluation of Drug Efficacy on JIMT-1 Tumor-Bearing Mice

1. Objective

nu/nu nude mice were used as test animals, and the therapeutic effect of Her2-ADC antibodies T-DM1, ADC-21 and ADC-24 on human breast cancer cells trastuzumab drug-resistant strain (herceptin) JIMT-1 xenograft tumor nude mice after intraperitoneal injection was evaluated.

2. Test drugs and materials

2-1. Test drugs

T-DM1 (prepared by reference US20050169933)
ADC-21: 3 mg/kg
ADC-21: 10 mg/kg
ADC-24: 3 mg/kg
ADC-24: 10 mg/kg
Blank control (Blank): PBS

2-2. Preparation method: all were diluted with PBS.

2-3. Test animals

nu/nu nude mice, purchased from Beijing Vital River.

3. Test method

Mice were inoculated subcutaneously on the right flank with JIMT-1 cells (Nanjing Kebai) ($5 \times 10^6$/mouse with 50% artificial basement membrane), and the tumors grew for 8 days to $203.09 \pm 11.94$ $mm^3$ before animals were randomly grouped (d1), 8/group, for 6 groups.

The drug was administrated by intraperitoneal injection for a total of 2 times. The tumor volumes and body weights were measured twice a week and the results were recorded. Excel 2003 statistical software was used. The mean values were calculated as avg; the SD values were calculated as STDEV; the SEM values were calculated as STDEV/SQRT; and the inter-group difference P-value was calculated as TTEST.

$$\text{Tumor volume (V) was calculated as: } V = 1/2 \times L_{long} \times L_{short}^2$$

$$\text{Relative volume (RTV)} = V_T/V_0$$

$$\text{Tumor inhibition rate (\%)} = (C_{RTV}\text{-}T_{RTV})/C_{RTV} \text{ (\%)}$$

Where $V_0$ and $V_T$ are the tumor volume at the beginning and end of the experiment, respectively. $C_{RTV}$ and $T_{RTV}$ are the relative tumor volumes of the blank group (Vehicle, PBS) and the experimental groups, respectively, at the end of the experiment.

4. Test results

The experimental results are shown in FIG. 2. The experiment ended after two intraperitoneal injections were performed and then observation was performed for 34 days. T-DM1 (10 mg/kg) had no inhibition effect on tumors; 3 mg/kg of ADC-21 had the tumor inhibition rate of 46.22% ($P < 0.01$); 10 mg/kg of ADC-21 had the tumor inhibition rate of 56.77% ($P < 0.001$); 3 mg/kg of ADC-24 had the tumor inhibition rate of 62.77% ($P < 0.001$); 10 mg/kg of ADC-24 had the tumor inhibition rate of 76.32% ($P < 0.001$). Under the condition of the same dosage, the tumor inhibition effect of the ADC-24 was significantly better than that of the ADC-21.

Test Example 1-5: Evaluation of Drug Efficacy on SK-BR-3 Tumor-Bearing Mice

1. Objective

nu/nu nude mice were used as test animals, and the therapeutic effect of Her2-ADC antibodies ADC-21 and ADC-22 on human breast cancer cells SK-BR-3 xenograft tumor nude mice after intraperitoneal injection was evaluated.

2. Test drugs and materials

2-1. Test drugs

ADC-21: 1 mg/kg
ADC-21: 6 mg/kg
ADC-22: 1 mg/kg
ADC-22: 6 mg/kg
Blank control (Blank): PBS.

2-2. Preparation method: all were diluted with PBS.

2-3. Test animals

nu/nu nude mice, purchased from Beijing Vital River.

3. Test method

Mice were inoculated subcutaneously on the right flank with SK-BR-3 cells (ATCC) ($5 \times 10^6$/mouse with 50% artificial basement membrane), and the tumors grew for 20 days to $153.34 \pm 11.73$ mm$^3$ before animals were randomly grouped (d0), 8/group, for 5 groups.

The drug was administrated by intraperitoneal injection for a total of 1 time. The tumor volumes and body weights were measured twice a week and the results were recorded. Excel 2003 statistical software was used. The mean values were calculated as avg; the SD values were calculated as STDEV; the SEM values were calculated as STDEV/SQRT; and the inter-group difference P-value was calculated as TTEST.

$$\text{Tumor volume (V) was calculated as: } V = 1/2 \times L_{long} \times L_{short}^2$$

$$\text{Relative volume (RTV)} = V_T/V_0$$

$$\text{Tumor inhibition rate (\%)} = (C_{RTV}-T_{RTV})/C_{RTV}\ (\%)$$

Where $V_0$ and $V_T$ are the tumor volume at the beginning and end of the experiment, respectively. $C_{RTV}$ and $T_{RTV}$ are the relative tumor volumes of the blank control group and the experimental groups, respectively, at the end of the experiment.

4. Test results

The experimental results are shown in FIG. 3. The experiment ended after one intraperitoneal injection was performed and then observation was performed for 28 days. 1 mg/kg of ADC-21 had the tumor inhibition rate of 15.01%; 6 mg/kg of ADC-21 had the tumor inhibition rate of 77.4%, and had significant difference compared with that of the blank control ($P < 0.001$). 1 mg/kg of ADC-22 had the tumor inhibition rate of 19.82%; 6 mg/kg of ADC-22 had the tumor inhibition rate of 98.38% ($P < 0.001$). Under the condition of the same dosage of 6 mg/kg, the tumor inhibition effect of the ADC-22 was significantly better than that of the ADC-21.

Test Example 1-6: Plasma Stability

Sample ADC-25 was mixed uniformly with human plasma, monkey plasma and a solution of 1% BSA in PBS at a final concentration of 100 μg/mL, and the mixture was filtered for sterilization, and then incubated in a water bath at 37 °C; the day of incubation was noted as day 0, and samples were taken out on days 7, 14 and 21, respectively, for detection of free toxin.

Samples were taken out at different time points then placed at room temperature, and vortexed and mixed well; 25 μL of samples were taken out to a 96-well plate; 50 μL of internal standard working solution (100 ng/mL camptothecine

acetonitrile solution) and 150 μL of acetonitrile were added; the mixture was vortexed for 5 min and centrifuged for 10 min (4000 rpm), and 5 μL of supernatant was taken out for LC/MS/MS analysis. The results showed that: ADC-25 was fairly stable in both human and monkey plasma, as well as a solution of 1% BSA in PBS, with a release rate of free toxin of no more than 2% at the highest, and tended to be stable on day 14, see FIG. 4.

Test Example 1-7: Evaluation of Therapeutic Effect of ADCs on Human Brain Astrocytoma U87MG Nude Mouse Xenograft Tumor

1. Objective

In this experiment, BALB/cA-nude mice were used as test animals, and the therapeutic effect of the ADC compounds disclosed herein on human brain astrocytoma U87MG nude mouse xenograft tumor was evaluated.

2. Test drugs and materials

2-1. Test drugs

ADC-27 (3 mg/kg)
ADC-26 (3 mg/kg)
Blank control (Blank): PBS buffer at pH 7.4.

2-2. Preparation method: PBS buffer at pH 7.4.

2-3. Test animals

BALB/cA-nude mice: purchased from Shanghai Jiesijie Experimental Animal Company.

3. Test method

In the experiment, female BALB/cA-nude mice aged 6-7 weeks were inoculated subcutaneously with human brain astrocytoma U87MG cells (human brain astrocytoma, Cell Bank, Chinese Academy of Sciences, Catalog # TCHu138). On the tenth day after inoculation of the cells, the animals were randomly grouped (D0), 8 animals per group, administered by intraperitoneal injection once every week for a total of 3 times, and tumor volume and body weight were measured 2-3 times per week and the data were recorded. Tumor volume (V) was calculated as follows:

$$V = 1/2 \times a \times b^2$$

wherein: a and b represent length and width, respectively.

$$\text{Relative volume (RTV)} = V_T/V_0$$

$$\text{Tumor inhibition rate (\%)} = (C_{RTV}-T_{RTV})/C_{RTV}\ (\%)$$

Where $V_0$ and $V_T$ are the tumor volume at the beginning and end of the experiment, respectively. $C_{RTV}$ and $T_{RTV}$ are the relative tumor volumes of the blank control group (Blank) and the experimental groups, respectively, at the end of the experiment.

4. Test results

Intraperitoneal injection (i.p.) administration was given once every week for a total of 3 times; after observation for 22 days, 3 mg/kg of ADC-27 had the tumor inhibition rate of 63.3% ($P < 0.0001$), and 3 mg/kg of ADC-26 had the inhibition rate of 49.1%. ADC-27 showed a stronger antitumor effect than ADC-26.
The weights of all animals in each group were normal in the administration process, and the ADCs had no

obvious toxic or side effect. The detection results are shown in Table 3 and FIG. 5. The detected antibodies could effectively inhibit the growth of U87MG xenograft tumor in tumor-bearing nude mice, and showed dose dependency.

Table 3. Therapeutic effects of antibodies administrated on human brain astrocytoma U87MG nude mouse xenograft tumor (D22)

| Group | Mean tumor volume ($mm^3$) | | | | Relative tumor volume | | Tumor inhibition rate (%) |
|---|---|---|---|---|---|---|---|
| | Day 0 | SEM | Day 22 | SEM | Day 22 | SEM | Day 22 |
| Blank control | 167.06 | 17.74 | 2906.96 | 327.6 | 17.76 | 1.63 | - |
| ADC-27 3mpk | 167.07 | 16.06 | 1172.48 | 80.27 | 7.55 | 0.95 | 63.3*** |
| ADC-26 3mpk | 167.73 | 17.63 | 1561.03 | 303.37 | 8.83 | 1.17 | 49.1*** |
| *** indicates P < 0.001 | | | | | | | |

Test Example 1-8: Evaluation of therapeutic effect of ADCs on human pharyngeal cancer hydrothorax metastatic cell Detroit 562 nude mouse xenograft tumor

1. Objective

In this experiment, BALB/cA-nude mice were used as test animals, and the therapeutic effect of the ADC compounds disclosed herein on human pharyngeal cancer hydrothorax metastatic cell Detroit 562 nude mouse xenograft tumor was evaluated.

2. Test drugs and materials

2-1. Test drugs

ADC-29 (3 mg/kg)
ADC-28 (3 mg/kg)
Negative control ADC (3 mg/kg): antibody drug conjugate formed by coupling non-B7H3-targeted antibodies to compound 20.

2-2. Preparation method: all were diluted with PBS.

2-3. Test animals

BALB/cA-nude mice: purchased from Changzhou Cavens Laboratory Animal Co., Ltd.

3. Test method

In the experiment, female BALB/cA-nude mice aged 6-7 weeks were inoculated subcutaneously with human pharyngeal cancer hydrothorax metastatic cells Detroit 562 (ATCC, Catalog #ATCC® CCL-138™). On the tenth day after inoculation of the cells, the animals were randomly grouped (D0), 8 animals per group, administered by intraperitoneal injection once every week for a total of 3 times, and tumor volume and body weight were measured 2-3 times per week and the data were recorded. Tumor volume (V) was calculated as follows:

$$V = 1/2 \times a \times b^2$$

wherein: a and b represent length and width, respectively.

$$\text{Relative volume (RTV)} = V_T/V_0$$

$$\text{Tumor inhibition rate (\%)} = (C_{RTV}-T_{RTV})/C_{RTV} \text{ (\%)}$$

Where $V_0$ and $V_T$ are the tumor volume at the beginning and end of the experiment, respectively. $C_{RTV}$ and $T_{RTV}$ are the relative tumor volumes of the control group (negative control) and the experimental groups, respectively, at the end of the experiment.

4. Test results

Intraperitoneal injection administration was given once every week for a total of 3 times; after observation for 28 days, 3 mg/kg of ADC-29 (3mpk) had the tumor inhibition rate of 72.27% (P < 0.001), and 3 mg/kg of ADC-28 (3mpk) had the inhibition rate of 56.2% (P < 0.001). ADC-29 showed a stronger antitumor effect than ADC-28.

The weights of all animals in each group were normal in the administration process, and the ADCs had no obvious toxic or side effect. The detection results are shown in Table 4 and FIG. 6. The detected antibodies could effectively inhibit the growth of Detroit 562 xenograft tumor in tumor-bearing nude mice, and showed dose dependency.

Table 4. Therapeutic effects of antibodies administrated on tumor-bearing nude mouse Detroit 562 xenograft tumor (D28)

| Group | Mean tumor volume ($mm^3$) | | Mean tumor volume (mm3) | | Relative tumor volume | | Tumor inhibition rate (%) on day 28 |
|---|---|---|---|---|---|---|---|
| | Day 0 | SEM | Day 28 | SEM | Day 28 | SEM | |
| Negative control | 182.70 | 6.79 | 1317.99 | 223.20 | 7.47 | 1.46 | - |
| ADC-29 3mpk | 182.59 | 6.50 | 381.48 | 105.76 | 2.07 | 0.58 | 72.27*** |
| ADC-28 3mpk | 182.57 | 6.92 | 578.07 | 160.13 | 3.43 | 1.09 | 56.2*** |
| *** indicates P < 0.001 | | | | | | | |

Test Example 1-9: Evaluation of Drug Efficacy on U87-MG Tumor-Bearing Mice

1. Objective

Balb/c nude mice were used as test animals, and the therapeutic effect of the B7H3-antibody drug conjugate after intraperitoneal injection was evaluated on a human glioblastoma cell U87MG xenograft tumor model of the mice.

2. Test drugs and materials

2-1. Test drugs

ADC-30 1 mg/kg
ADC-30 3 mg/kg
ADC-31 1 mg/kg
ADC-31 3 mg/kg
Blank control (Blank): PBS

2-2. Preparation method: all were diluted with PBS.

2-3. Test animals

BALB/cA-nude mice: purchased from Shanghai Slac Laboratory Animal Co., Ltd.

3. Test method

Mice were inoculated subcutaneously on the right flank with U87MG cells (human brain astrocytoma, Cell Bank, Chinese Academy of Sciences, Catalog # TCHu138) ($2.5 \times 10^6$/mouse), and the tumors grew for 14 days to 167.49 $mm^3$ before animals were randomly grouped (d1), 8/group, for 5 groups.

Intraperitoneal injection administration was given once every week for a total of 3 times. The tumor volumes and body weights were measured twice a week and the results were recorded.

Excel 2003 statistical software was used. The mean values were calculated as avg; the SD values were calculated as STDEV; the SEM values were calculated as STDEV/SQRT; and the inter-group difference P-value was calculated as TTEST.

Tumor volume (V) was calculated as:

$$V = 1/2 \times L_{long} \times L_{short}^2$$

$$\text{Relative volume (RTV)} = V_T/V_0$$

$$\text{Tumor inhibition rate (\%)} = (C_{RTV}-T_{RTV})/C_{RTV}\ (\%)$$

Where $V_0$ and $V_T$ are the tumor volume at the beginning and end of the experiment, respectively. $C_{RTV}$ and $T_{RTV}$ are the relative tumor volumes of the blank control group (Vehicle) and the experimental groups, respectively, at the end of the experiment.

4. Test results

The experimental results are shown in FIG. 7. Intraperitoneal injection administration was given once every week for a total of 3 times; after observation for 18 days, tested ADCs had the following tumor inhibition rates: 1 mg/kg of ADC-30 had the tumor inhibition rate of 0.31%; 3 mg/kg of ADC-30 had the tumor inhibition rate of 45.23% (P

< 0.0001); 1 mg/kg of ADC-31 had the tumor inhibition rate of 39.22% (P < 0.01); 3 mg/kg of ADC-31 had the tumor inhibition rate of 80.24% (P < 0.0001). Under the condition of the same dosage, the tumor inhibition effect of the ADC-31 was significantly better than that of the ADC-30.

II. Optimization of preparation process

Exemplary products of the following examples have the structure shown below:

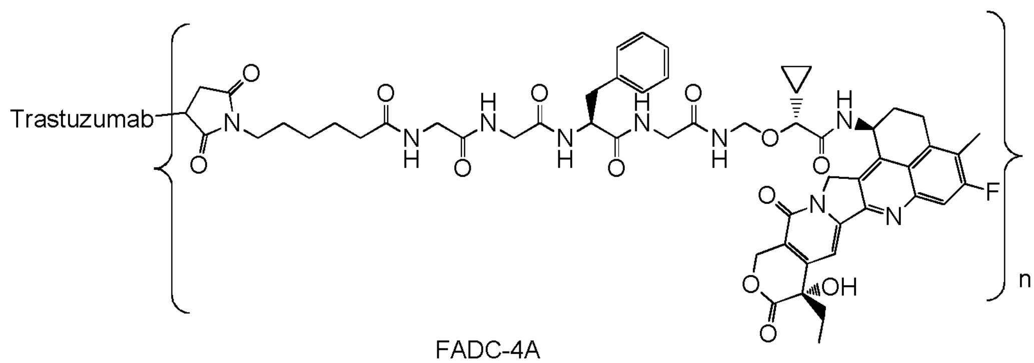

FADC-4A

Example 2-1

An antibody stock solution (0.0004251 mmol, trastuzumab antibody diluted with 20 mM histidine-hydrochloric acid buffer to a final antibody concentration of 15 mg/mL) containing 61.71 mg of trastuzumab was reacted with 0.7311 mg of tris(2-carboxyethyl)phosphine hydrochloride (Sigma, 0.002550 mmol) in 20 mM histidine-hydrochloric acid buffer (pH 5.6) containing 2.5 mM EDTA under stirring in a constant temperature water bath at 0 °C for 3 h to give an intermediate I solution.

Compound **9**-compound **9-A** with shorter retention time (3.196 mg, 0.002975 mmol) was dissolved in 0.2062 mL of DMSO to give a DMSO solution of compound **9-A.** 0.2052 mL of DMSO was added to the above intermediate I solution, and the above DMSO solution of compound **9-A** was added to the resulting solution which was stirred in a water bath at 25 °C for 1 h and quenched with excess cysteine. An exemplary product **ADC-2-1** of general formula FADC-4A was obtained.

Mean values were calculated by reverse phase chromatography-mass spectrometry: n = 5.45.

Example 2-2

An antibody stock solution (0.0004251 mmol, trastuzumab antibody diluted with 20 mM histidine-hydrochloric acid buffer to a final antibody concentration of 15 mg/mL) containing 61.71 mg of trastuzumab was reacted with 0.5118 mg of tris(2-carboxyethyl)phosphine hydrochloride (Sigma, 0.001785 mmol) in 20 mM histidine-hydrochloric acid buffer (pH 5.6) containing 2.5 mM EDTA under stirring in a constant temperature water bath at 13 °C for 3 h to give an intermediate I solution.

Compound **9**-compound **9-A** with shorter retention time (3.196 mg, 0.002975 mmol) was dissolved in 0.2062 mL of DMSO to give a DMSO solution of compound **9-A.** 0.2052 mL of DMSO was added to the above intermediate I solution, and the above DMSO solution of compound **9-A** was added to the resulting solution which was stirred in a water bath at 25 °C for 1 h and quenched with excess cysteine. An exemplary product **ADC-2-2** of general formula FADC-4A was obtained.

Mean values were calculated by reverse phase chromatography-mass spectrometry: n = 5.49.

Example 2-3

An antibody stock solution (0.0004251 mmol, trastuzumab antibody diluted with 20 mM histidine-hydrochloric acid buffer to a final antibody concentration of 15 mg/mL) containing 61.71 mg of trastuzumab was reacted with 0.4021 mg of tris(2-carboxyethyl)phosphine hydrochloride (Sigma, 0.001403 mmol) in 20 mM histidine-hydrochloric acid buffer (pH 5.6) containing 2.5 mM EDTA under stirring in a constant temperature water bath at 25 °C for 3 h to give an intermediate I solution.

Compound **9**-compound **9-A** with shorter retention time (3.196 mg, 0.002975 mmol) was dissolved in 0.2062 mL of DMSO to give a DMSO solution of compound **9-A.** 0.2052 mL of DMSO was added to the above intermediate I solution, and the above DMSO solution of compound **9-A** was added to the resulting solution which was stirred in a water bath at 25 °C for 1 h and quenched with excess cysteine. An exemplary product **ADC-2-3** of general formula FADC-4A was obtained.

Mean values were calculated by reverse phase chromatography-mass spectrometry: n = 5.39.

Example 2-4

An antibody stock solution (0.0004251 mmol, trastuzumab antibody diluted with 20 mM histidine-hydrochloric acid buffer to a final antibody concentration of 15 mg/mL) containing 61.71 mg of trastuzumab was reacted with 0.3899 mg of tris(2-carboxyethyl)phosphine hydrochloride (Sigma, 0.001360 mmol) in 20 mM histidine-hydrochloric acid buffer (pH 5.6) containing 2.5 mM EDTA under stirring in a constant temperature water bath at 28 °C for 3 h to give an intermediate I solution.

Compound **9**-compound **9-A** with shorter retention time (3.196 mg, 0.002975 mmol) was dissolved in 0.2062 mL of DMSO to give a DMSO solution of compound **9-A.** 0.2052 mL of DMSO was added to the above intermediate I solution, and the above DMSO solution of compound **9-A** was added to the resulting solution which was stirred in a water bath at 25 °C for 1 h and quenched with excess cysteine. An exemplary product **ADC-2-4** of general formula FADC-4A was obtained.

Mean values were calculated by reverse phase chromatography-mass spectrometry: n = 5.44.

Example 2-5

An antibody stock solution (0.0004251 mmol, trastuzumab antibody diluted with 20 mM histidine-hydrochloric acid buffer to a final antibody concentration of 15 mg/mL) containing 61.71 mg of trastuzumab was reacted with 0.3778 mg of tris(2-carboxyethyl)phosphine hydrochloride (Sigma, 0.001318 mmol) in 20 mM histidine-hydrochloric acid buffer (pH 5.6) containing 2.5 mM EDTA under stirring in a constant temperature water bath at 37 °C for 3 h to give an intermediate I solution.

Compound **9**-compound **9-A** with shorter retention time (3.196 mg, 0.002975 mmol) was dissolved in 0.2062 mL of DMSO to give a DMSO solution of compound **9-A.** 0.2052 mL of DMSO was added to the above intermediate I solution, and the above DMSO solution of compound **9-A** was added to the resulting solution which was stirred in a water bath at 25 °C for 1 h and quenched with excess cysteine. An exemplary product **ADC-2-5** of general formula FADC-4A was obtained.

Mean values were calculated by reverse phase chromatography-mass spectrometry: n = 5.46.

Example 2-6

An antibody stock solution (0.0003790 mmol, trastuzumab antibody diluted with 50 mM PBS buffer to a final antibody concentration of 15 mg/mL) containing 55.02mg of trastuzumab was reacted with 0.4563 mg of tris(2-carboxyethyl)phosphine hydrochloride (Sigma, 0.001592 mmol) in 50 mM PBS buffer (pH 6.5) containing 2.5 mM EDTA under stirring in a constant temperature water bath at 13 °C for 3 h to give an intermediate I solution.

Compound **9**-compound **9-A** with shorter retention time (2.850 mg, 0.002653 mmol) was dissolved in 0.1839 mL of DMSO to give a DMSO solution of compound **9-A.** 0.1829 mL of DMSO was added to the above intermediate I solution, and the above DMSO solution of compound **9-A** was added to the resulting solution which was stirred in a water bath at 25 °C for 1 h and quenched with excess cysteine. An exemplary product **ADC-2-6** of general formula FADC-4A was obtained.

Mean values were calculated by reverse phase chromatography-mass spectrometry: n = 5.39.

Example 2-7

An antibody stock solution (0.0003790 mmol, trastuzumab antibody diluted with 50 mM PBS buffer to a final antibody concentration of 15 mg/mL) containing 55.02mg of trastuzumab was reacted with 0.3477 mg of tris(2-carboxyethyl)phosphine hydrochloride (Sigma, 0.001213 mmol) in 50 mM PBS buffer (pH 6.5) containing 2.5 mM EDTA under stirring in a constant temperature water bath at 25 °C for 3 h to give an intermediate I solution.

Compound **9**-compound **9-A** with shorter retention time (2.850 mg, 0.002653 mmol) was dissolved in 0.1839 mL of DMSO to give a DMSO solution of compound **9-A.** 0.1829 mL of DMSO was added to the above intermediate I solution, and the above DMSO solution of compound **9-A** was added to the resulting solution which was stirred in a water bath at 25 °C for 1 h and quenched with excess cysteine. An exemplary product **ADC-2-7** of general formula FADC-4A was obtained.

Mean values were calculated by reverse phase chromatography-mass spectrometry: n = 5.50.

Example 2-8

An antibody stock solution (0.0003790 mmol, trastuzumab antibody diluted with 50 mM PBS buffer to a final antibody concentration of 15 mg/mL) containing 55.02mg of trastuzumab was reacted with 0.3259 mg of tris(2-carboxyethyl)phosphine hydrochloride (Sigma, 0.001137 mmol) in 50 mM PBS buffer (pH 6.5) containing 2.5 mM EDTA under stirring in a constant temperature water bath at 37 °C for 3 h to give an intermediate I solution.

Compound **9**-compound **9-A** with shorter retention time (2.850 mg, 0.002653 mmol) was dissolved in 0.1839 mL of DMSO to give a DMSO solution of compound **9-A.** 0.1829 mL of DMSO was added to the above intermediate I solution, and the above DMSO solution of compound **9-A** was added to the resulting solution which was stirred in a water bath at 25 °C for 1 h and quenched with excess cysteine. An exemplary product **ADC-2-8** of general formula FADC-4A was obtained.

Mean values were calculated by reverse phase chromatography-mass spectrometry: n = 5.47.

Example 2-9

An antibody stock solution (0.88 mmol, trastuzumab antibody diluted with 20 mM histidine-hydrochloric acid buffer to a final antibody concentration of 15 mg/mL) containing 127.4g of trastuzumab was reacted with 0.83g of tris(2-carboxyethyl)phosphine hydrochloride (Sigma, 2.90 mmol) in 20 mM histidine-hydrochloric acid buffer (pH 5.6) containing

2.5 mM EDTA under stirring in a constant temperature water bath at 25 °C for 3 h to give an intermediate I solution.

Compound **9-**compound **9-A** with shorter retention time (6.6 g, 6.14 mmol) was dissolved in 0.43 L of DMSO to give a DMSO solution of compound **9-A.** 0.43 L of DMSO was added to the above intermediate I solution, and the above DMSO solution of compound **9-A** was added to the resulting solution which was stirred in a water bath at 25 °C for 1 h, and the reaction was stopped.

The above reaction solution was purified through a Capto S Impact (GE) cation chromatography column, and washed with not less than 9 column volumes of 0.05 M acetate buffer containing 10% (v/v) DMSO (pH = 5.5) and with 6 column volumes of 0.05 M acetate buffer (pH = 5.5), followed by elution with 0.05 M acetate buffer (pH = 5.5, containing 0.39 M sodium chloride) to remove free toxins and the residual solvent from the reaction solution. The cation eluate was subjected to 7-fold volume equal-volume ultrafiltration (30 kd ultrafiltration membrane pack) and to buffer exchange to 0.01 M succinic acid buffer (pH 5.0) at 25 °C to give an exemplary product **ADC-A1** of general formula FADC-4A. Samples of 4 batches were prepared using the method described above.

The drug loading of the samples of 4 batches determined by the reverse phase chromatography-mass spectrometry was 5.7. The yields of the four batches were 100.8%, 98.9%, 97.4% and 99.0%, respectively.

Example 2-10

A prepared aqueous solution of tris(2-carboxyethyl)phosphine (TCEP) (10 mM, 3.55 mL, 35.5 μmol) was added to an aqueous PBS buffer (0.05 M aqueous PBS buffer at pH 6.5; 10.0 mg/mL, 164 mL, 11.08 μmol) of an antibody trastuzumab at 37 °C, and the reaction mixture was placed in a water bath shaker to react at 37 °C for 3.5 h with shaking, and the reaction was stopped. The reaction solution was cooled to 25 °C in a water bath.

Compound **9-**compound **9-A** with shorter retention time (185 mg, 172 μmol) was dissolved in 3.88 mL of acetonitrile and 1.94 mL of DMSO, and the reaction mixture was added to the above reaction solution, placed in a water bath shaker, and reacted at 25 °C for 3 h with shaking, and the reaction was stopped. The reaction solution was purified by desalting through ultrafiltration membranes with an aqueous PBS buffer of 2% (v/v) acetonitrile and 1% (v/v) DMSO (0.05 M aqueous PBS buffer at pH 6.5) and an aqueous succinic buffer (0.01 M aqueous succinic buffer at pH 5.3) successively to remove small molecules, and an exemplary product **ADC-B14** sample of general formula FADC-4A was obtained and stored at 4 °C.

Mean values were calculated by reverse phase chromatography-mass spectrometry: n = 5.3.

Test Example 2-1: Drug Loading Distribution Test

1. RP-DAR determination method

1.1. RP-DAR analysis was performed under the following measurement conditions:

UPLC system: Waters H-Class ultra-high performance liquid chromatograph UPLC system
Detector: TUV detector (measuring wavelength: 280 nm)
Chromatographic column: Waters ACQUITY UPLC Protein BEH C4 (2.1 mm × 150 mm, 1.7 μm)
Column temperature: 80 °C
Flow rate: 0.3 mL/min
Temperature of sample chamber: 20 °C
Operating time: 25 min
Mobile phase A: 0.1% aqueous difluoroacetic acid (DFA) solution
Mobile phase B: 0.1% DFA acetonitrile solution
Gradient program: 27.0% B-44.0% B (0.00 min-12.00 min), 44.0% B-100% B (12.00 min-13.00 min), 100% B-100% B (13.00 min-20.00 min), 100% B-27.0% B (20.00 min-20.04 min), and 27.0% B-27.0% B (20.04 min-25 min)
Volume of sample injected: 1.0 μL

1.2. Data analysis

In the case of a drug-bound light chain (one drug-bound light chain: $L_1$) and drug-bound heavy chains (one drug-bound heavy chain: $H_1$, two drug-bound heavy chains: $H_2$, three drug-bound heavy chains: $H_3$, and four drug-bound heavy chains: H4), hydrophobicity increased in proportion to the number of bound drugs, and the retention time was prolonged, when compared to a light chain ($L_0$) and a heavy chain ($H_0$) of an antibody not binding to drugs. Therefore, elution was performed in the order of $L_0$, $L_1$, $H_0$, $H_1$, $H_2$, $H_3$ and $H_4$.

Since the drug linkers absorbed UV, the resulting peak area was corrected according to the number of bound drugs using molar absorption coefficients of the light chains, the heavy chains and the drug linkers according to the following expression. The calculation formula is as follows:

$$\text{Light chain } (\varepsilon \text{ LC-280})/(\varepsilon \text{ LC-280} + \text{number of linked drugs} \times \varepsilon \text{ drug-280})$$

$$\text{Heavy chain } (\varepsilon \text{ HC-280})/(\varepsilon \text{ HC-280} + \text{number of linked drugs} \times \varepsilon \text{ drug-280})$$

Note: ε LC-280: molar extinction coefficient of light chain at 280 nm;
ε HC-28: molar extinction coefficient of heavy chain at 280 nm;
ε drug-280: molar extinction coefficient of toxin at 280 nm.

Table 5. Calculation table of drug loading from reverse phase chromatography

| Name | Number of linked drugs | Corrected peak area percentage |
|---|---|---|
| $L_0$ | 0 | 100 × $L_0$ corrected peak area/total LC corrected peak area |
| $L_1$ | 1 | 100 × $L_1$ corrected peak area/total LC corrected peak area |
| $H_0$ | 0 | 100 × $H_0$ corrected peak area/total HC corrected peak area |
| $H_1$ | 1 | 100 × $H_1$ corrected peak area/total HC corrected peak area |
| $H_2$ | 2 | 100 × $H_2$ corrected peak area/total HC corrected peak area |
| $H_3$ | 3 | 100 × $H_3$ corrected peak area/total HC corrected peak area |
| $H_4$ | 4 | 100 × $H_4$ corrected peak area/total HC corrected peak area |
| Note: total LC corrected peak area = $L_0$ corrected peak area + $L_1$ corrected peak area Total HC corrected peak area = $H_0$ corrected peak area + $H_1$ corrected peak area + $H_2$ corrected peak area + $H_3$ corrected peak area + $H_4$ corrected peak area Drug loading n = 2 × Σ(number of linked drugs × corrected peak area percentage)/100 2. Measurement results | | |

Table 6. Determination of drug loading distribution

| Sample | Buffer system | Temperature of reduction reaction | Ratio of heavy chains binding to different number of drugs (%) | | | | Ratio of light chains binding to different number of drugs (%) | |
|---|---|---|---|---|---|---|---|---|
| | | | $H_0$ | $H_1$ | $H_2$ | $H_3$ | $L_0$ | $L_1$ |
| ADC-2-1 | Histidine-hydrochloric acid | 0 °C | 1.80 | 30.25 | 52.31 | 15.64 | 9.42 | 90.58 |
| ADC-2-2 | | 13 °C | 2.53 | 33.18 | 36.86 | 27.43 | 14.57 | 85.43 |
| ADC-2-3 | | 25 °C | 3.52 | 31.90 | 29.67 | 34.90 | 26.37 | 73.63 |
| ADC-2-4 | | 28 °C | 3.36 | 28.50 | 30.96 | 37.18 | 29.76 | 70.24 |
| ADC-2-5 | | 37 °C | 3.32 | 21.38 | 36.47 | 38.83 | 37.69 | 62.31 |

(continued)

| Sample | Buffer system | Temperature of reduction reaction | Ratio of heavy chains binding to different number of drugs (%) | | | | Ratio of light chains binding to different number of drugs (%) | |
|---|---|---|---|---|---|---|---|---|
| | | | $H_0$ | $H_1$ | $H_2$ | $H_3$ | $L_0$ | $L_1$ |
| ADC-2-6 | PBS | 13 °C | 2.96 | 34.36 | 25.87 | 36.81 | 16.87 | 83.13 |
| ADC-2-7 | | 25 °C | 4.18 | 28.36 | 27.56 | 39.90 | 28.17 | 71.83 |
| ADC-2-8 | | 37 °C | 6.00 | 19.78 | 32.50 | 41.73 | 36.53 | 63.47 |
| Note: ADC-2-1 to ADC-2-8 had the content of $H_4$ lower than the detection limit (<<1%) | | | | | | | | |

The results showed that, for the samples prepared by adopting the same buffer system and different temperatures of reduction reaction, the uniform degree of the drug loading distribution of the samples was significantly improved along with the reduction of the reduction reaction temperature; for samples prepared by adopting the same temperature of reduction reaction and different buffer systems, the drug loading distribution of the samples adopting the histidine-hydrochloric acid buffer system was more uniform.

Test Example 2-2: Free Toxin Test

1. Free toxin determination method

1.1. HPLC analysis was performed under the following measurement conditions:

HPLC system: Waters H-Class ultra-high performance liquid chromatograph UPLC system
Detector: TUV detector (measuring wavelength: 370 nm)
Chromatographic column: Waters ACQUITY UPLC Petide BEH C18 (130Å, 2.1 mm × 150 mm, 1.7 μm)
Column temperature: 40 °C
Flow rate: 0.3 mL/min
Temperature of sample chamber: 10 °C
Mobile phase A: 0.1% aqueous trifluoroacetic acid (TFA) solution
Mobile phase B: 0.1% TFA acetonitrile solution
Gradient program: 25.0% B-25.0% B (0.00 min-1.00 min), 25.0% B-55.0% B (1.00 min-17.00 min), 55.0% B-25.0% B (17.00 min-17.10 min), 25.0% B-25.0% B (17.10 min-20.00 min)
Volume of sample injected: 5.0 μL

1.2. Data analysis

The LOD limit of the toxin was calculated as follows:

$$\text{Toxin limit (ppm)} = 0.1 \times 4 \times 1000/C$$

Note:

a) 0.1 was the toxin LOD solution concentration (μg/mL), 4 was the dilution factor at the time of sample pretreatment, 1000 was the unit conversion factor, and C was the protein concentration (mg/mlmL) of the sample to be measured.
b) If the peak area of the free toxin in the sample was smaller than the peak area of the LOD solution, it was determined to be smaller than the limit or undetectable.

2. Measurement results

The detection of free toxin was performed on ADC-A1 (batches 1-4) and ADC-B14, and the results (see Table 7) indicated that cation column chromatography was not only suitable for large-scale preparation, but also significantly reduced free toxin.

Table 7. Free toxin determination

| Sample | Drug loading | Peak area of free toxin |
|---|---|---|
| ADC-A1 (batch 1) | 5.7 | 760 |
| ADC-A1 (batch 2) | 5.7 | 123 |
| ADC-A1 (batch 3) | 5.7 | 70 |
| ADC-A1 (batch 4) | 5.7 | 37 |
| ADC-B14 | 5.3 | 5423 |

## Claims

**1.** A method for preparing an antibody drug conjugate, wherein the antibody drug conjugate has a structure as shown in general formula ($Pc-L_a-Y-D$):

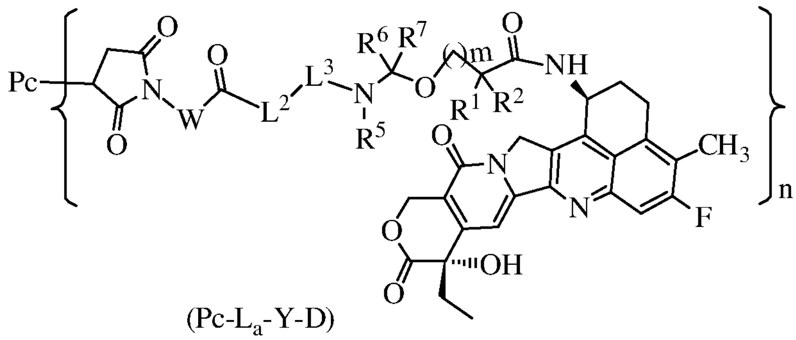

($Pc-L_a-Y-D$)

;

wherein:

W is selected from the group consisting of $C_{1-8}$ alkyl, $C_{1-8}$ alkyl-$C_{3-7}$ cycloalkyl and linear heteroalkyl of 1 to 8 atoms, the linear heteroalkyl comprising 1 to 3 heteroatoms selected from the group consisting of N, O and S, wherein the $C_{1-8}$ alkyl, the $C_{3-7}$ cycloalkyl and the linear heteroalkyl are each independently optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-7}$ cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;

$L^3$ is a peptide residue consisting of 2 to 7 amino acid residues, wherein the amino acid residues are selected from the group consisting of amino acid residues formed from amino acids from phenylalanine, glycine, valine, lysine, citrulline, serine, glutamic acid and aspartic acid, and are optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-7}$ cycloalkyl;

$R^1$ is $C_{1-6}$ haloalkyl or $C_{3-7}$ cycloalkyl;

$R^2$ is selected from the group consisting of hydrogen, $C_{1-6}$ haloalkyl and $C_{3-7}$ cycloalkyl; or, $R^1$ and $R^2$, together with carbon atoms linked thereto, form $C_{3-7}$ cycloalkyl;

$R^5$ is selected from the group consisting of hydrogen, $C_{1-6}$ alkyl, $C_{1-6}$ haloalkyl, deuterated $C_{1-6}$ alkyl and hydroxy $C_{1-6}$ alkyl;

$R^6$ and $R^7$ are identical or different and are each independently selected from the group consisting of hydrogen, halogen, $C_{1-6}$ alkyl, $C_{1-6}$ haloalkyl, deuterated $C_{1-6}$ alkyl and hydroxy $C_{1-6}$ alkyl;

m is 0 or 1;

n is a decimal or an integer from 3 to 8;
Pc is an antibody or an antigen-binding fragment thereof;
the preparation method comprises the following steps:

step (a): reacting the antibody or the antigen-binding fragment thereof with a reducing agent at a reaction temperature of about 1 °C to about 36 °C; and
step (b): reacting the product of the step (a) with a compound of the following formula (La-Y-D);

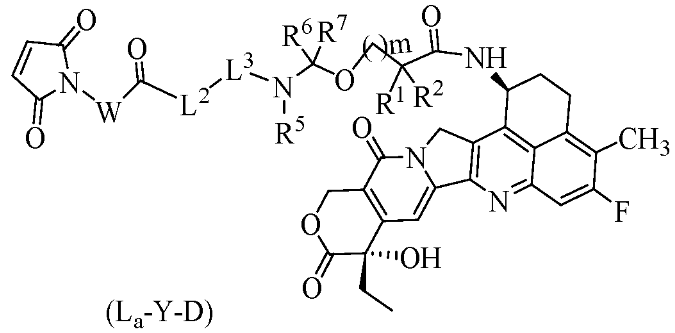

($L_a$-Y-D)

;

wherein: W, $L^2$, $L^3$, $R^1$, $R^2$, $R^5$, $R^6$, $R^7$ and m are as defined above.

2. The method for preparing an antibody drug conjugate according to claim 1, wherein the reaction temperature condition in the step (a) is about 4 °C to about 30 °C, preferably about 20 °C to about 30 °C, and more preferably about 25 °C.

3. The method for preparing an antibody drug conjugate according to claim 1 or 2, wherein the reaction in the step (a) is performed at a pH of about 4.5 to about 6.5; preferably, the reaction is performed at a pH of about 5.0 to about 6.0; more preferably, the reaction is performed at a pH of about 5.6.

4. The method for preparing an antibody drug conjugate according to any one of claims 1 to 3, wherein the reaction in the step (a) is performed in a buffer; preferably, the buffer is selected from the group consisting of histidine salt buffers, phosphate buffers and acetate buffers; more preferably, the buffer is an EDTA-containing histidine salt buffer.

5. The method for preparing an antibody drug conjugate according to any one of claims 1 to 4, wherein the reducing agent in the step (a) is selected from the group consisting of tris(2-carboxyethyl)phosphine or a salt thereof, 1,4-dimercaptothreitol and β-mercaptoethanol, and preferably tris(2-carboxyethyl)phosphine hydrochloride.

6. The method for preparing an antibody drug conjugate according to any one of claims 1 to 5, wherein the preparation method further comprises a step (c) comprising purifying the product of the step (b) by cation column chromatography or affinity column chromatography; preferably, the step (c) comprises subjecting the product of the step (b) to cation column chromatography using a packing material selected from the group consisting of Capto S Impact and Poros XS, and preferably Capto S Impact.

7. The method for preparing an antibody drug conjugate according to any one of claims 1 to 6, wherein the antibody or the antigen-binding fragment thereof is selected from the group consisting of an anti-HER2 (ErbB2) antibody, an anti-EGFR antibody, an anti-B7-H3 antibody, an anti-c-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-Integrin antibody, an anti-PSMA antibody, an anti-Tenascin-C antibody, an anti-SLC44A4 antibody, an anti-Mesothelin antibody and an antigen-binding fragment thereof;
preferably, the antibody or the antigen-binding fragment thereof is selected from the group consisting of trastuzumab, pertuzumab, nimotuzumab, enoblituzumab, emibetuzumab, inotuzumab, pinatuzumab, brentuximab, gemtuzumab, bivatuzumab, lorvotuzumab, cBR96, glematumamab and an antigen-binding fragment thereof.

8. The method for preparing an antibody drug conjugate according to any one of claims 1 to 7, wherein the antibody drug conjugate has a structure as shown in the following formula:

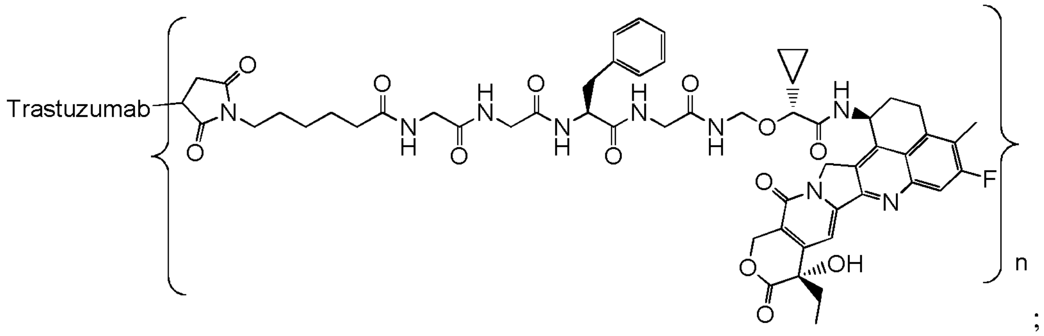

wherein n is a decimal or an integer from 4 to 8.

**9.** The method for preparing an antibody drug conjugate according to any one of claims 1 to 8, wherein n is a decimal or an integer from 4 to 8, preferably a decimal or an integer from 5 to 7, and more preferably a decimal or an integer from 5.3 to 6.1.

**10.** The method for preparing an antibody drug conjugate according to any one of claims 1 to 9, wherein drug loading distribution of the antibody drug conjugate is as follows: in a population of antibody heavy chains, a proportion of antibody heavy chains binding to 4 drugs is 4% or less, and a proportion of antibody heavy chains not binding to drugs is 5% or less;
preferably, drug loading distribution of the antibody drug conjugate is as follows: in a population of antibody heavy chains, a proportion of antibody heavy chains binding to four drugs is 4% or less, and a proportion of antibody heavy chains not binding to drugs is 5% or less; and in a population of antibody light chains, a proportion of antibody light chains binding to one drug is 65% or more.

**11.** A method for preparing an antibody drug conjugate, wherein the antibody drug conjugate has a structure as shown in the following formula:

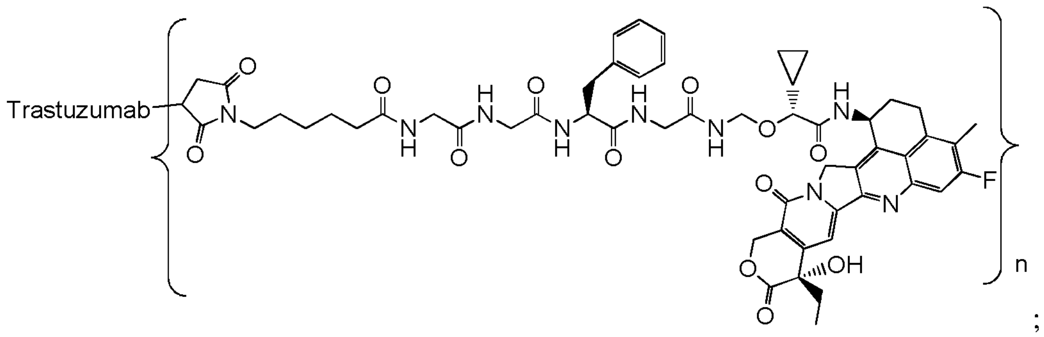

wherein n is a decimal or an integer from 4 to 8;
the preparation method comprises the following steps:

step (a): reacting trastuzumab with TCEP at a reaction temperature of about 4 °C to about 30 °C and a pH of about 5.0 to about 6.0; and
step (b): reacting the product of the step (a) with a compound of the following formula;

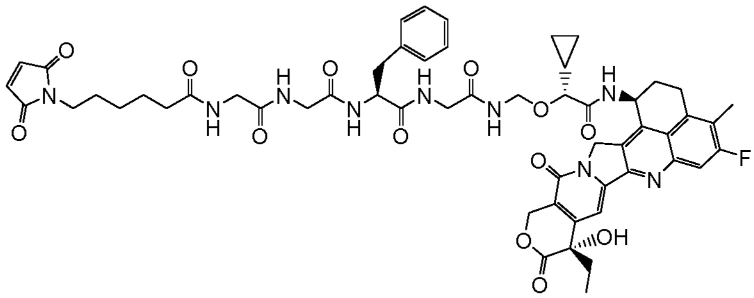

.

12. A method for preparing an antibody drug conjugate, wherein the antibody drug conjugate has a structure as shown in the following formula:

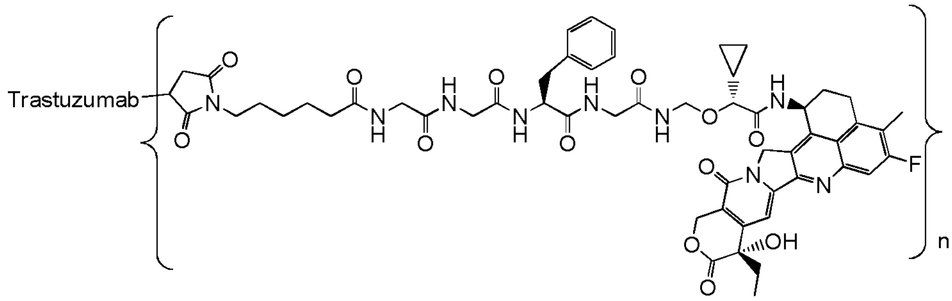

;

wherein n is a decimal or an integer from 4 to 8;
the preparation method comprises the following steps:

step (a): reacting trastuzumab with TCEP at a reaction temperature of about 25 °C and a pH of about 5.6, the reaction being performed in an EDTA-containing histidine-hydrochloric acid buffer;
step (b): reacting the product of the step (a) with a compound of the following formula;

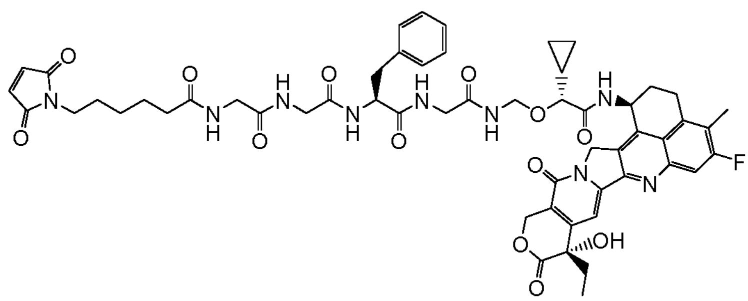

;

and
step (c): subjecting the product of the step (b) to purification through a cation chromatography column.

13. An antibody drug conjugate or a pharmaceutically acceptable salt thereof, wherein the antibody drug conjugate has a structure as shown in general formula (Pc-$L_a$-Y-D):

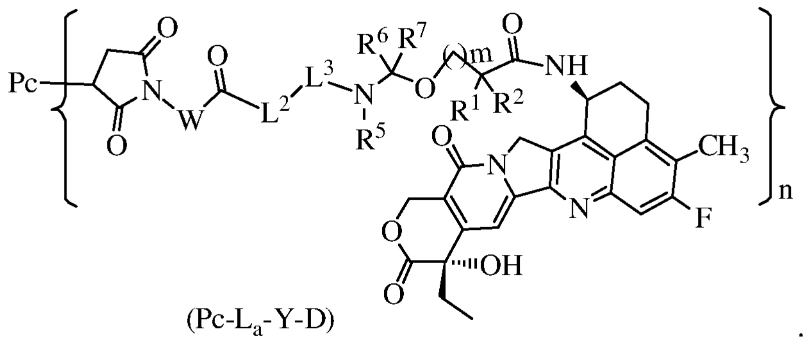

(Pc-$L_a$-Y-D) ;

wherein:

W is selected from the group consisting of $C_{1-8}$ alkyl, $C_{1-8}$ alkyl-$C_{3-7}$ cycloalkyl and linear heteroalkyl of 1 to 8 atoms, the linear heteroalkyl comprising 1 to 3 heteroatoms selected from the group consisting of N, O and S, wherein the $C_{1-8}$ alkyl, the $C_{3-7}$ cycloalkyl and the linear heteroalkyl are each independently optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-7}$ cycloalkyl;
$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$ and a chemical bond, wherein $p^1$ is an integer from 1 to 20;
$L^3$ is a peptide residue consisting of 2 to 7 amino acid residues, wherein the amino acid residues are selected from the group consisting of amino acid residues formed from amino acids from phenylalanine, glycine, valine, lysine, citrulline, serine, glutamic acid and aspartic acid, and are optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-7}$ cycloalkyl;
$R^1$ is $C_{1-6}$ haloalkyl or $C_{3-7}$ cycloalkyl;
$R^2$ is selected from the group consisting of hydrogen, $C_{1-6}$ haloalkyl and $C_{3-7}$ cycloalkyl; or, $R^1$ and $R^2$, together with carbon atoms linked thereto, form $C_{3-7}$ cycloalkyl;
$R^5$ is selected from the group consisting of hydrogen, $C_{1-6}$ alkyl, $C_{1-6}$ haloalkyl, deuterated $C_{1-6}$ alkyl and hydroxy $C_{1-6}$ alkyl;
$R^6$ and $R^7$ are identical or different and are each independently selected from the group consisting of hydrogen, halogen, $C_{1-6}$ alkyl, $C_{1-6}$ haloalkyl, deuterated $C_{1-6}$ alkyl and hydroxy $C_{1-6}$ alkyl;
m is 0 or 1;
n is a decimal or an integer from 4 to 8;
Pc is an antibody or an antigen-binding fragment thereof;
and, drug loading distribution of the antibody drug conjugate is as follows: in a population of antibody heavy chains, a proportion of antibody heavy chains binding to 4 drugs is 4% or less, and a proportion of antibody heavy chains not binding to drugs is 5% or less;
preferably, drug loading distribution of the antibody drug conjugate is as follows: in a population of antibody heavy chains, a proportion of antibody heavy chains binding to four drugs is 4% or less, and a proportion of antibody heavy chains not binding to drugs is 5% or less; and in a population of antibody light chains, a proportion of antibody light chains binding to one drug is 65% or more.

**14.** An antibody drug conjugate or a pharmaceutically acceptable salt thereof, wherein the antibody drug conjugate is prepared by the method for preparing the antibody drug conjugate according to any one of claims 1 to 9; and drug loading distribution of the antibody drug conjugate is as follows: in a population of antibody heavy chains, a proportion of antibody heavy chains binding to 4 drugs is 4% or less, and a proportion of antibody heavy chains not binding to drugs is 5% or less;
preferably, drug loading distribution of the antibody drug conjugate is as follows: in a population of antibody heavy chains, a proportion of antibody heavy chains binding to four drugs is 4% or less, and a proportion of antibody heavy chains not binding to drugs is 5% or less; and in a population of antibody light chains, a proportion of antibody light chains binding to one drug is 65% or more.

**15.** An antibody drug conjugate or a pharmaceutically acceptable salt thereof, wherein the antibody drug conjugate has a structure as shown in the following formula:

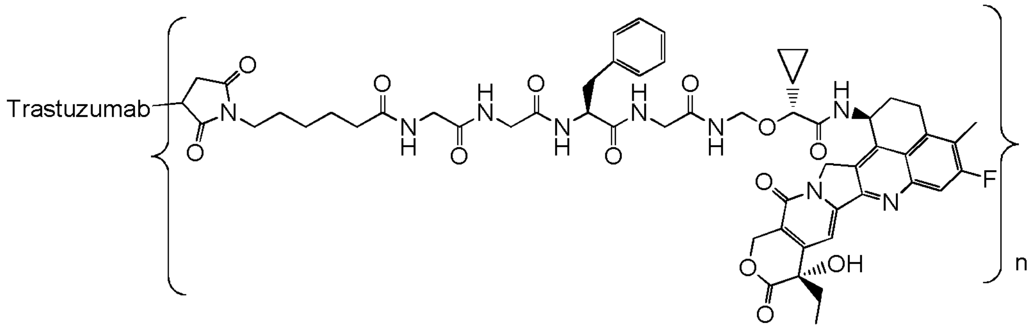

;

wherein n is a decimal or an integer from 4 to 8;

the antibody drug conjugate is prepared by the method for preparing the antibody drug conjugate according to claim 11 or 12; and drug loading distribution of the antibody drug conjugate is as follows: in a population of antibody heavy chains, a proportion of antibody heavy chains binding to 4 drugs is 4% or less, and a proportion of antibody heavy chains not binding to drugs is 5% or less;

preferably, drug loading distribution of the antibody drug conjugate is as follows: in a population of antibody heavy chains, a proportion of antibody heavy chains binding to four drugs is 4% or less, and a proportion of antibody heavy chains not binding to drugs is 5% or less; and in a population of antibody light chains, a proportion of antibody light chains binding to one drug is 65% or more.

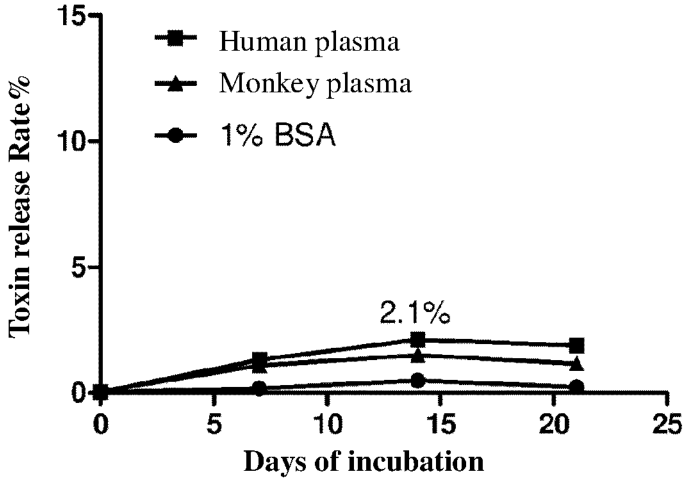
Human plasma
Monkey plasma
1% BSA
2.1%
Toxin release Rate%
Days of incubation
15
10
5
0
0
5
10
15
20
25

FIG. 1A

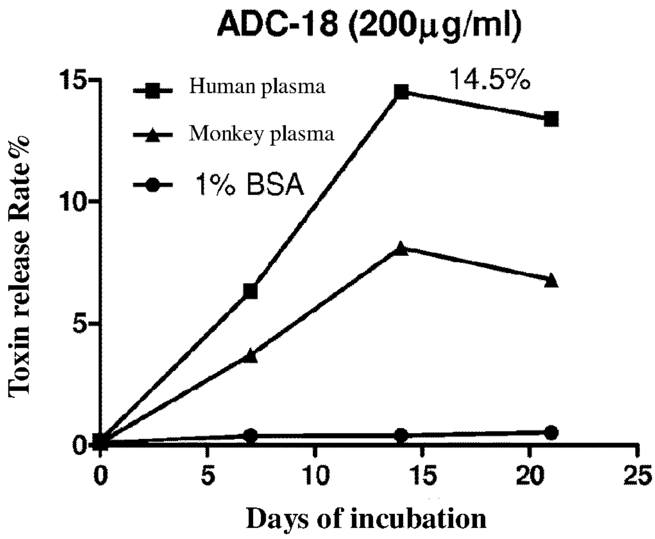
ADC-18 (200μg/ml)
Human plasma
Monkey plasma
1% BSA
14.5%
Toxin release Rate%
Days of incubation
15
10
5
0
0
5
10
15
20
25

FIG. 1B

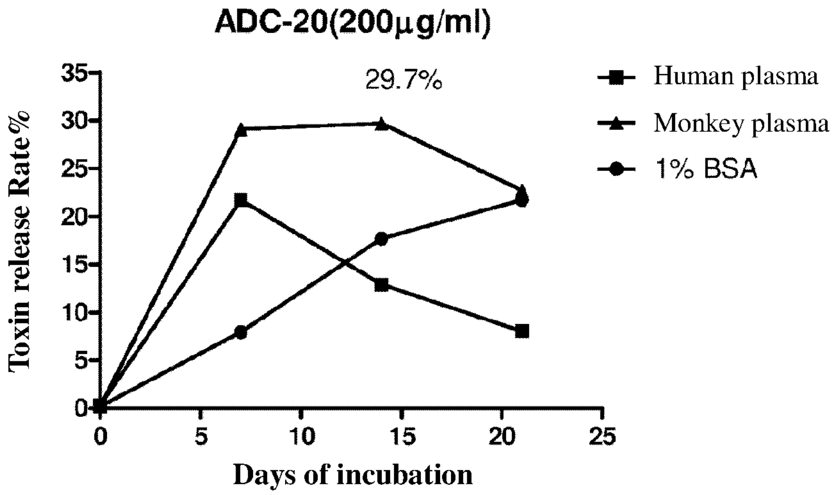
ADC-20(200μg/ml)
29.7%
Human plasma
Monkey plasma
1% BSA
Toxin release Rate%
Days of incubation
0
5
10
15
20
25
30
35

FIG. 1C

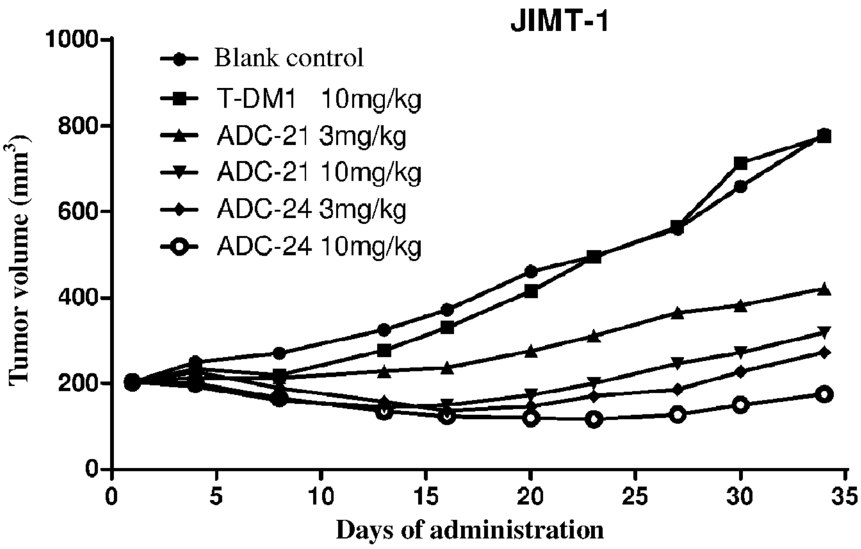
JIMT-1
Blank control
T-DM1 10mg/kg
ADC-21 3mg/kg
ADC-21 10mg/kg
ADC-24 3mg/kg
ADC-24 10mg/kg
Tumor volume (mm3)
Days of administration
0
200
400
600
800
1000
5
10
15
20
25
30
35

FIG. 2

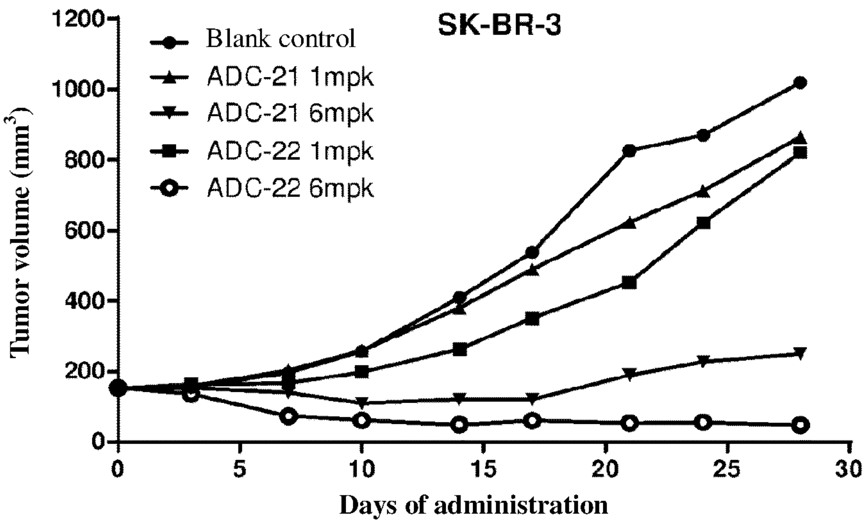
SK-BR-3
Blank control
ADC-21 1mpk
ADC-21 6mpk
ADC-22 1mpk
ADC-22 6mpk
Tumor volume (mm3)
1200
1000
800
600
400
200
0
0
5
10
15
20
25
30
Days of administration

FIG. 3

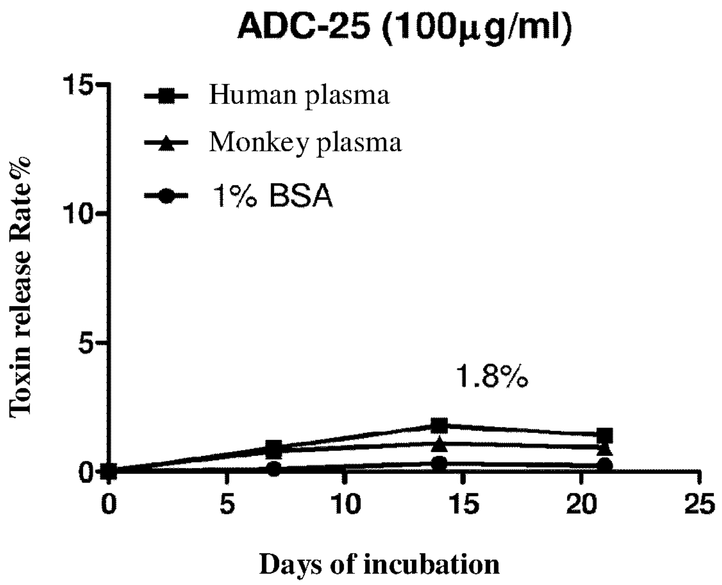
ADC-25 (100μg/ml)
Human plasma
Monkey plasma
1% BSA
Toxin release Rate%
15
10
5
0
1.8%
0
5
10
15
20
25
Days of incubation

FIG. 4

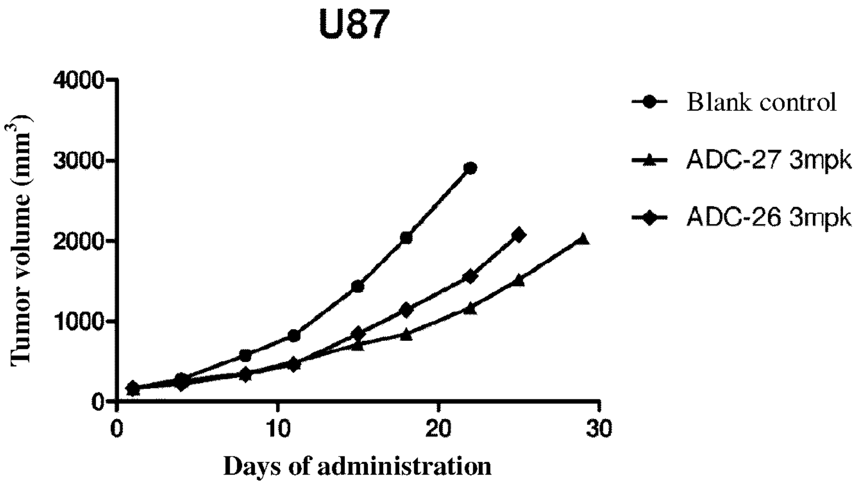
U87
4000
3000
2000
1000
0
Tumor volume (mm3)
0
10
20
30
Days of administration
Blank control
ADC-27 3mpk
ADC-26 3mpk

FIG. 5

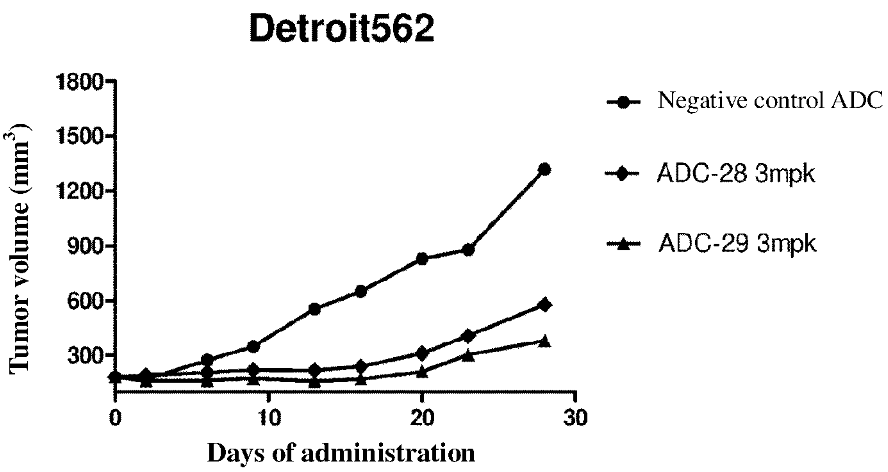
Detroit562
1800
1500
1200
900
600
300
Tumor volume (mm3)
0
10
20
30
Days of administration
Negative control ADC
ADC-28 3mpk
ADC-29 3mpk

FIG. 6

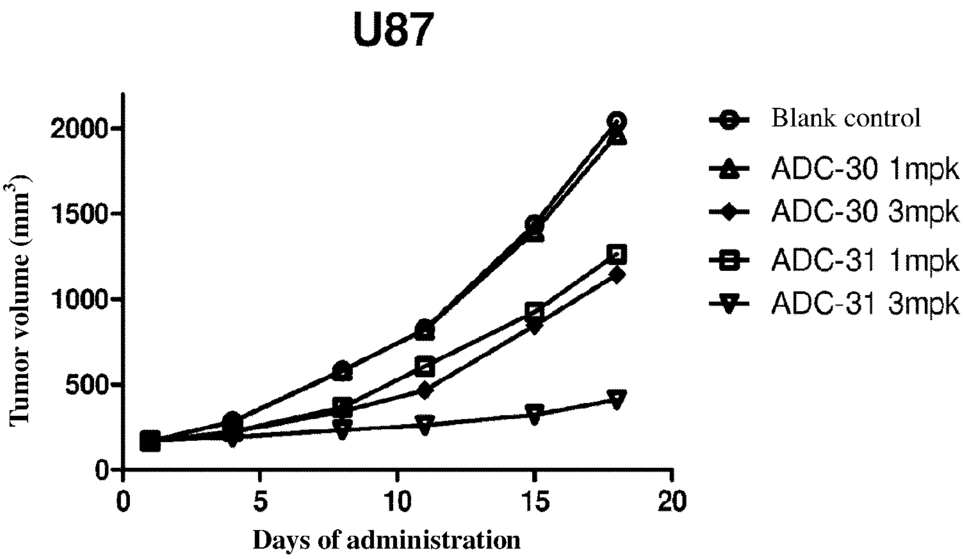
U87
Tumor volume (mm3)
2000
1500
1000
500
0
0
5
10
15
20
Days of administration
Blank control
ADC-30 1mpk
ADC-30 3mpk
ADC-31 1mpk
ADC-31 3mpk

FIG. 7

## INTERNATIONAL SEARCH REPORT

International application No.
**PCT/CN2021/083014**

**A. CLASSIFICATION OF SUBJECT MATTER**

C07K 16/30(2006.01)i; A61K 39/395(2006.01)i; A61P 35/00(2006.01)i; A61K 31/4745(2006.01)i; A61K 31/48(2006.01)i

According to International Patent Classification (IPC) or to both national classification and IPC

**B. FIELDS SEARCHED**

Minimum documentation searched (classification system followed by classification symbols)

C07K, A61K, A61P

Documentation searched other than minimum documentation to the extent that such documents are included in the fields searched

Electronic data base consulted during the international search (name of data base and, where practicable, search terms used)

CNABS, CNMED, MOABS, HKABS, TWABS, TWMED, DWPI, SIPOABS, CPEA, AUABS, SGABS, ILABS, FRABS, LEXIS, PLABS, JPABS, KRABS, DEABS, RUABS, WOTXT, EPTXT, USTXT, CNTXT, PUBMED, CNKI, 恒瑞医药, 抗体药物偶联物, 依沙替康, 药物载量, 缓冲剂, 层析, ADC, exatecan, trastuzumab, drug load, buffer, chromatography

**C. DOCUMENTS CONSIDERED TO BE RELEVANT**

| Category* | Citation of document, with indication, where appropriate, of the relevant passages | Relevant to claim No. |
|---|---|---|
| X | CN 104755494 A (DAIICHI SANKYO COMPANY, LIMITED) 01 July 2015 (2015-07-01) see embodiment 46, description paragraphs 1236, 1247 | 1-15 |
| A | CN 109195631 A (SORRENTO THERAPEUTICS INC.) 11 January 2019 (2019-01-11) see entire document | 1-15 |
| A | CN 110577600 A (SHANGHAI INSTITUTE OF MATERIA MEDICA, CHINESE ACADEMY OF SCIENCES et al.) 17 December 2019 (2019-12-17) see entire document | 1-15 |
| A | CN 107029242 A (INDUSTRIAL TECHNOLOGY RESEARCH INSTITUTE) 11 August 2017 (2017-08-11) see entire document | 1-15 |
| A | SG 11201502887 B (DAUC DAIICHI SANKYO CO LTD) 04 January 2019 (2019-01-04) see entire document | 1-15 |
| A | CN 109106951 A (SICHUAN BAILI PHARMACEUTICAL CO., LTD.) 01 January 2019 (2019-01-01) see entire document | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 June 2021** | **29 June 2021** |

Name and mailing address of the ISA/CN

**China National Intellectual Property Administration (ISA/CN)**
**No. 6, Xitucheng Road, Jimenqiao, Haidian District, Beijing 100088**
**China**

Facsimile No. **(86-10)62019451**

Authorized officer

Telephone No.

Form PCT/ISA/210 (second sheet) (January 2015)

**INTERNATIONAL SEARCH REPORT**

International application No. **PCT/CN2021/083014**

**C. DOCUMENTS CONSIDERED TO BE RELEVANT**

| Category* | Citation of document, with indication, where appropriate, of the relevant passages | Relevant to claim No. |
|---|---|---|
| A | CA 3054939 A1 (SEATTLE GENETICS INC.) 07 September 2018 (2018-09-07) see entire document | 1-15 |
| A | CN 106188293 A (JIANGSU HENGRUI MEDICINE CO., LTD. et al.) 07 December 2016 (2016-12-07) see entire document | 1-15 |

Form PCT/ISA/210 (second sheet) (January 2015)

**INTERNATIONAL SEARCH REPORT**
**Information on patent family members**

International application No.
**PCT/CN2021/083014**

| Patent document cited in search report | | | Publication date (day/month/year) | Patent family member(s) | | | Publication date (day/month/year) |
|---|---|---|---|---|---|---|---|
| CN | 104755494 | A | 01 July 2015 | TW | 202033499 | A | 16 September 2020 |
| | | | | HR | P20200353 | T1 | 12 June 2020 |
| | | | | LT | 2907824 | T | 11 June 2018 |
| | | | | KR | 20200026323 | A | 10 March 2020 |
| | | | | SG | 10201804788 X | A | 30 July 2018 |
| | | | | KR | 101841818 | B1 | 26 March 2018 |
| | | | | AU | 2013328111 | A1 | 12 March 2015 |
| | | | | TW | 201811746 | A | 01 April 2018 |
| | | | | TW | 201420117 | A | 01 June 2014 |
| | | | | HU | E039000 | T2 | 28 December 2018 |
| | | | | PH | 12015500667 | B1 | 18 May 2015 |
| | | | | JP | 6030267 | B1 | 24 November 2016 |
| | | | | JP | 2020180124 | A | 05 November 2020 |
| | | | | EP | 3342785 | A1 | 04 July 2018 |
| | | | | JP | 2018188455 | A | 29 November 2018 |
| | | | | CA | 2885800 | A1 | 17 April 2014 |
| | | | | HR | P20180870 | T1 | 13 July 2018 |
| | | | | MX | 2019004502 | A | 21 August 2019 |
| | | | | JP | 6715888 | B2 | 01 July 2020 |
| | | | | PL | 3342785 | T3 | 07 September 2020 |
| | | | | KR | 102087017 | B1 | 10 March 2020 |
| | | | | ES | 2773710 | T3 | 14 July 2020 |
| | | | | LT | 3342785 | T | 10 February 2020 |
| | | | | JP | 2016196484 | A | 24 November 2016 |
| | | | | HU | E048748 | T2 | 28 August 2020 |
| | | | | MX | 2015003903 | A | 17 July 2015 |
| | | | | NZ | 705394 | A | 26 October 2018 |
| | | | | CY | 1120363 | T1 | 10 July 2019 |
| | | | | TW | 201920098 | A | 01 June 2019 |
| | | | | PL | 2907824 | T3 | 31 August 2018 |
| | | | | JP | 5953378 | B2 | 20 July 2016 |
| | | | | MX | 364484 | B | 29 April 2019 |
| | | | | AU | 2013328111 | B2 | 02 November 2017 |
| | | | | CN | 104755494 | B | 07 September 2018 |
| | | | | TW | I615152 | B | 21 February 2018 |
| | | | | DK | 2907824 | T3 | 23 July 2018 |
| | | | | EP | 2907824 | B1 | 11 April 2018 |
| | | | | KR | 20190135559 | A | 06 December 2019 |
| | | | | IL | 271760 | D0 | 27 February 2020 |
| | | | | JP | 6371452 | B2 | 08 August 2018 |
| | | | | WO | 2014057687 | A1 | 17 April 2014 |
| | | | | CA | 3021435 | A1 | 17 April 2014 |
| | | | | US | 2016279259 | A1 | 29 September 2016 |
| | | | | RU | 2018128384 | A | 02 October 2018 |
| | | | | JP | 2018008982 | A | 18 January 2018 |
| | | | | JP | WO2014057687 | A1 | 05 September 2016 |
| | | | | US | 2019008981 | A1 | 10 January 2019 |
| | | | | NZ | 746440 | A | 29 November 2019 |
| | | | | US | 10195288 | B2 | 05 February 2019 |
| CN | 109195631 | A | 11 January 2019 | EP | 3471771 | A1 | 24 April 2019 |

Form PCT/ISA/210 (patent family annex) (January 2015)

**INTERNATIONAL SEARCH REPORT**
**Information on patent family members**

International application No.
**PCT/CN2021/083014**

| Patent document cited in search report | | | Publication date (day/month/year) | Patent family member(s) | | | Publication date (day/month/year) |
|---|---|---|---|---|---|---|---|
| | | | | JP | 2019523761 | A | 29 August 2019 |
| | | | | WO | 2017210288 | A1 | 07 December 2017 |
| | | | | US | 2017340750 | A1 | 30 November 2017 |
| | | | | EP | 3471771 | A4 | 15 April 2020 |
| | | | | CA | 3025931 | A1 | 07 December 2017 |
| CN | 110577600 | A | 17 December 2019 | | None | | |
| CN | 107029242 | A | 11 August 2017 | US | 10683327 | B2 | 16 June 2020 |
| | | | | TW | I660741 | B | 01 June 2019 |
| | | | | US | 2017119902 | A1 | 04 May 2017 |
| | | | | JP | 6412906 | B2 | 24 October 2018 |
| | | | | US | 10233212 | B2 | 19 March 2019 |
| | | | | JP | 2017114848 | A | 29 June 2017 |
| | | | | EP | 3165532 | B1 | 19 December 2018 |
| | | | | EP | 3165237 | B1 | 19 December 2018 |
| | | | | US | 10618935 | B2 | 14 April 2020 |
| | | | | JP | 6486316 | B2 | 20 March 2019 |
| | | | | CN | 107043406 | A | 15 August 2017 |
| | | | | JP | 2017114847 | A | 29 June 2017 |
| | | | | TW | 201716088 | A | 16 May 2017 |
| | | | | EP | 3165532 | A2 | 10 May 2017 |
| | | | | TW | 201716380 | A | 16 May 2017 |
| | | | | EP | 3165532 | A3 | 14 June 2017 |
| | | | | EP | 3165237 | A1 | 10 May 2017 |
| | | | | US | 2018016300 | A1 | 18 January 2018 |
| | | | | TW | I618697 | B | 21 March 2018 |
| | | | | US | 2019106459 | A1 | 11 April 2019 |
| SG | 11201502887 | B | 04 January 2019 | | None | | |
| CN | 109106951 | A | 01 January 2019 | WO | 2019034176 | A1 | 21 February 2019 |
| CA | 3054939 | A1 | 07 September 2018 | SG | 11201907889 Y | A | 27 September 2019 |
| | | | | WO | 2018160909 | A1 | 07 September 2018 |
| | | | | BR | 112019018043 | A2 | 07 April 2020 |
| | | | | EP | 3589319 | A1 | 08 January 2020 |
| | | | | KR | 20190125398 | A | 06 November 2019 |
| | | | | AU | 2018226824 | A1 | 19 September 2019 |
| | | | | JP | 2020510671 | A | 09 April 2020 |
| | | | | US | 2020000932 | A1 | 02 January 2020 |
| | | | | MX | 2019010202 | A | 02 October 2019 |
| | | | | IL | 268966 | D0 | 31 October 2019 |
| | | | | CN | 110913903 | A | 24 March 2020 |
| CN | 106188293 | A | 07 December 2016 | EP | 3284751 | A4 | 05 December 2018 |
| | | | | CN | 111196853 | A | 26 May 2020 |
| | | | | RU | 2017135257 | A | 17 May 2019 |
| | | | | US | 2018110875 | A1 | 26 April 2018 |
| | | | | CN | 106687480 | A | 17 May 2017 |
| | | | | KR | 20170138451 | A | 15 December 2017 |
| | | | | CN | 106687480 | B | 19 April 2019 |
| | | | | MX | 2017012965 | A | 06 June 2018 |
| | | | | US | 10543284 | B2 | 28 January 2020 |
| | | | | BR | 112017021245 | A2 | 26 June 2018 |

Form PCT/ISA/210 (patent family annex) (January 2015)

**INTERNATIONAL SEARCH REPORT**
**Information on patent family members**

International application No.
**PCT/CN2021/083014**

| Patent document cited in search report | Publication date (day/month/year) | Patent family member(s) | | | Publication date (day/month/year) |
|---|---|---|---|---|---|
| | | AU | 2016248357 | A1 | 26 October 2017 |
| | | JP | 2018516539 | A | 28 June 2018 |
| | | CA | 2982777 | A1 | 20 October 2016 |
| | | EP | 3284751 | A1 | 21 February 2018 |
| | | RU | 2017135257 | A3 | 24 September 2019 |
| | | TW | 201638108 | A | 01 November 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010219311
- CN 202110297397
- US 4970198 A
- US 5079233 A
- US 5585089 A
- US 5606040 A
- US 5693762 A
- US 5739116 A
- US 5767285 A
- US 5773001 A
- WO 2004010957 A
- WO 2005001038 A
- US 7090843 B
- US 7659241 B
- WO 2008025020 A
- WO 2005037992 A
- US 8088387 B
- WO 2014057687 A
- CN 2019107873 W
- US 20050238649 A1
- US 5208020 A
- EP 0737686 A1
- WO 2013106717 A
- WO 201396771 A
- US 200520645 B
- CN 105829346 A
- EP 2907824 A
- EP 2907824 A1
- EP 2862856 A1
- CN 108853514 A
- CN 104755494 A
- US 20050169933 A


### Non-patent literature cited in the description


- **MULLARD A.** *Nature Reviews Drug Discovery,* 2013, vol. 12, 329-332
- **DIJOSEPH JF ; ARMELLINO DC.** *Blood,* 2004, vol. 103, 1807-1814
- *Drugs of the Future,* 2000, vol. 25 (7), 686
- *Nat. Biotechnol,* 2003, vol. 21 (7), 778-784
- *Clinical Cancer Research,* 2016, vol. 22 (20), 5097-5108
- *Cancer Sci,* 2016, vol. 107, 1039-1046
- *J. Biol. Chem,* 1968, vol. 243, 3558
- **KABAT, E. A. et al.** Sequences of Proteins of Immunological Interest. 1991
- **WATSON et al.** Molecular Biology of the Gene. The Benjamin/Cummings Pub. Co, 1987, 224
- **CHARI et al.** *Cancer Research,* 1992, vol. 52, 127-131
- *Tetrahedron Letters,* 1984, vol. 25 (12), 1269-72
- *Journal of the American Chemical Society,* 2014, vol. 136 (22), 8138-8142
- *Journal of Medicinal Chemistry,* 2013, vol. 56 (13), 5541-5552